(19) Europäisches Patentamt
European Patent Office
Office européen des brevets

(11) EP 3 359 967 B1

(12) **EUROPEAN PATENT SPECIFICATION**

(45) Date of publication and mention of the grant of the patent:
**18.08.2021 Bulletin 2021/33**

(21) Application number: **16787748.9**

(22) Date of filing: **06.10.2016**

(51) Int Cl.:
***G01N 33/68*** *(2006.01)*

(86) International application number:
**PCT/EP2016/073880**

(87) International publication number:
**WO 2017/060353 (13.04.2017 Gazette 2017/15)**

(54) **METHODS FOR TREATING INFLAMMATORY AND OTHER DISEASES AND THE USE OF BIOMARKERS AS PREDICTORS OF CLINICAL SENSITIVITY TO TREATMENT WITH APREMILAST**

VERFAHREN ZUR BEHANDLUNG ENTZÜNDLICHER UND ANDERER KRANKHEITEN UND VERWENDUNG VON BIOMARKERN ALS PRÄDIKATOREN VON KLINISCHER EMPFÄNGLICHKEIT ZUR BEHANDLUNG MIT APREMILAST

PROCÉDÉS DE TRAITEMENT DES MALADIES INFLAMMATOIRES ET D’AUTRES MALADIES, ET UTILISATION DE BIOMARQUEURS COMME FACTEURS PRÉDICTIFS DE LA SENSIBILITÉ CLINIQUE AU TRAITEMENT PAR APRÉMILAST

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2015 US 201562237910 P**

(43) Date of publication of application:
**15.08.2018 Bulletin 2018/33**

(73) Proprietor: **Amgen (Europe) GmbH**
**6343 Risch-Rotkreuz (CH)**

(72) Inventors:
- **SCHAFER, Peter H.**
  **Belle Mead, New Jersey 08502 (US)**
- **TROTTER, Matthew William Burnell**
  **41807 Sevilla (ES)**

(74) Representative: **Carpmaels & Ransford LLP**
**One Southampton Row**
**London WC1B 5HA (GB)**

(56) References cited:
**WO-A1-2010/077722** **WO-A2-2011/117366**
**US-A1- 2013 040 835**

- **PETER H. SCHAFER ET AL: "The Pharmacodynamic Impact of Apremilast, an Oral Phosphodiesterase 4 Inhibitor, on Circulating Levels of Inflammatory Biomarkers in Patients with Psoriatic Arthritis: Substudy Results from a Phase III, Randomized, Placebo-Controlled Trial (PALACE 1)", JOURNAL OF IMMUNOLOGY RESEARCH, vol. 91, no. 1, 20 April 2015 (2015-04-20) , pages 118-10, XP055319978, ISSN: 2314-8861, DOI: 10.3899/jrheum.121162**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 359 967 B1

## Description

### 1. BACKGROUND

Inflammatory diseases, such as arthritis or related arthritic conditions (*e.g.,* ankylosing spondylitis, osteoarthritis and rheumatoid arthritis), Behcet's disease, inflammatory bowel disease (*e.g.,* Crohn's disease and ulcerative colitis), psoriasis, atopic dermatitis and contact dermatitis are prevalent and problematic ailments. TNF-$\alpha$ plays a central role in the inflammatory response and the administration of their antagonists block chronic and acute responses in animal models of inflammatory disease. Enhanced or unregulated TNF-$\alpha$ production has been implicated in a number of diseases, for example ankylosing spondylitis, Behcet's disease, rheumatoid arthritis, Crohn's disease, and ulcerative colitis.

Phosphodiesterase type 4 (PDE4) belongs to a family of isoenzymes referred to as cyclic nucleotide phosphodiesterases (PDE). It is believed that the primary cellular mechanism for the inactivation of cAMP is the breakdown of adenosine 3',5'-cyclic monophosphate (cAMP) by PDE. Inhibition of PDE4 has been shown to be particularly effective in the inhibition of inflammatory mediator releases.

No treatment for a diagnosed disease is 100% effective in all patient populations having the disease. For example, in the largest study trial, etanercept produced a response rate of 57% compared with 22% for placebo after 24 weeks (response was determined via the validated ASAS 20 response criteria developed by the Assessments in Ankylosing Spondylitis [ASAS] Working Group). *See* McCormack et al., BioDrugs: clinical immunotherapeutics, biopharmaceuticals and gene therapy 18 (3): 199-205 (2003). Accordingly, there is still a significant need for accurate and sensitive methods of identifying patients with various inflammatory diseases or disorders that are likely to be responsive to a treatment. Serum markers predicting clinical response to anti-TNF antibodies in patients with ankylosing spondylitis are described in WO 2010/077722 A1.

### 2. SUMMARY

The invention is set out in the appended set of claims. Provided herein are methods of identifying a subject having a disease or disorder, *e.g*., ankylosing spondylitis, who is likely to be responsive to a treatment with apremilast, or predicting the responsiveness of a subject having or suspected of having a disease or disorder, e.g., ankylosing spondylitis, to a treatment with apremilast. Also provided herein is apremilast for use in methods for treating a patient who is likely to be responsive to apremilast.

In one aspect, provided herein are methods of identifying a subject having ankylosing spondylitis who is likely to be responsive to a treatment with apremilast, or predicting the responsiveness of a subject having or suspected of having ankylosing spondylitis to a treatment with apremilast, comprising:

(a) measuring the level of monocyte chemotactic protein 1 (MCP1) in a sample from the subject; and
(b) diagnosing the subject as being likely to be responsive to apremilast if the level of MCP1 in the sample is lower than a threshold level of MCP1.

In some aspects of the disclosure, the methods provided herein further comprises administering a therapeutically effective amount of apremilast to the subject diagnosed as being likely to be responsive to apremilast.

In another aspect, provided herein is apremilast for use in methods of treating a subject having ankylosing spondylitis, comprising:

(a) identifying the subject having ankylosing spondylitis that may be responsive to a treatment with apremilast, comprising:

  (i) measuring the level of MCP1 in a sample from the subject; and
  (ii) diagnosing the subject as being likely to be responsive to apremilast if the level of MCP1 in the sample is lower than a threshold level of MCP1, and

(b) administering to the subject a therapeutically effective amount of apremilast if the subject is identified as being likely to be responsive to treatment with apremilast.

In some embodiments, the threshold level of MCP1 is determined based on the levels of MCP1 in a population. In some embodiments, the threshold level of MCP1 is between 200 pg/ml to 50 pg/ml. In some embodiments, the threshold level of MCP1 is between 105 pg/ml to 70 pg/ml. In some embodiments, the threshold level of MCP1 is lower than 200 pg/ml, 150 pg/ml, 120 pg/ml, 105 pg/ml, 100 pg/ml, 95 pg/ml, 90 pg/ml, 85 pg/ml, 80 pg/ml, 75 pg/ml, or 70 pg/ml. In some embodiments, the threshold level of MCP1 is about 105 pg/ml. In some embodiments, the threshold level

of MCP1 is about 95 pg/ml. In some embodiments, the threshold level of MCP1 is about 75 pg/ml.

In some embodiments, the subject is diagnosed as having more than a 50% to 90% likelihood of being responsive to apremilast if the level of MCP1 is lower than the threshold level of MCP1. In other embodiments, the subject is diagnosed as having more than a 50% likelihood of being responsive to apremilast if the level of MCP1 is lower than the threshold level of MCP1. In yet other embodiments, the subject is diagnosed as having more than a 60% likelihood of being responsive to apremilast if the level of MCP1 is lower than the threshold level of MCP1. In yet other embodiments, the subject is diagnosed as having more than a 70% likelihood of being responsive to apremilast if the level of MCP1 is lower than the threshold level of MCP1. In yet other embodiments, the subject is diagnosed as having more than a 80% likelihood of being responsive to apremilast if the level of MCP1 is lower than the threshold level of MCP1. In yet other embodiments, the subject is diagnosed as having more than a 90% likelihood of being responsive to apremilast if the level of MCP1 is lower than the threshold level of MCP1.

## 3. BRIEF DESCRIPTION OF THE FIGURES

**FIGS. 1A-1D** show that a patient group (cluster 4 or MCP1 low group) identified based on biomarker expression profile is associated with responsiveness to a treatment with apremilast. **FIG. 1A** shows unsupervised clustering results with biomarker profiles and identification of a group of patients (cluster 4) with higher response rate to apremilast treatment. **FIG. 1B** shows cluster 4 is significantly associated with responsiveness to apremilast. **FIG. 1C** shows the response rate to apremilast treatment is significantly higher in the MCP1 low patient group in the ASAS20 study. **FIG. 1D** shows the response rate to apremilast treatment is significantly higher in the MCP1 low patient group in the ASAS40 study.

**FIGS. 2A-2F** shows using MCP1 level alone to classify a patient for the purpose of predicting the patient's response to a treatment with apremilast. **FIG. 2A** shows that the patients in the cluster 4 patient group have significantly lower expression level of MCP1 as compared with the rest patients. **FIG. 2B** shows the level of MCP1 was used to differentiate a patient subgroup (14% of total population) highly responsive to apremilast (in apremilast arm with either 20 mg or 30 mg apremilast) with a response rate of about 62.5%. **FIG. 2C** shows MCP1 level differentiated a patient subgroup (20% of the apremilast 30 mg arm) with a response rate to 30 mg apremilast treatment of about 66.6%. **FIG. 2D** shows MCP1 distribution using a threshold level (103.97 pg/ml) calculated based on the median value of MCP1 of the total population. **FIG. 2E** shows MCP1 distribution using the threshold calculated as the mid-point between MCP1 low median and non-MCPl low median across all patients. **FIG. 2F** shows MCP1 distribution using the threshold level calculated as the mean of the thresholds obtained from 10-fold cross validation.

**FIGS. 3A-3E** show using expression levels of multiple biomarkers for classifying a patient for the purpose of predicting the patient's response to a treatment with apremilast. **FIG. 3A** shows sensitivity and AUC of two classifiers-Naive Bayes and Weighted RSVM. **FIG. 3B** shows a patient distribution plot based on the expression levels of the top 2 biomarkers (MCP1 and Interleukin-15) identified in the present study. **FIG.3C** shows a patient distribution plot based on the expression levels of the top 5 biomarkers (MCP1, Interleukin-15, C - reactive protein, Matrix Metalloproteinase-3, and Interleukin-1 receptor antagonist) identified in the present study. **FIG. 3D** shows a patient distribution plot based on the expression levels of the top 10 biomarkers (MCP1, Interleukin-15, C-Reactive Protein, Matrix Metalloproteinase-3, Interleukin-1 receptor antagonist, von Willebrand Factor, Eotaxin-1, Alpha-1-Antitrypsin, Macrophage Inflammatory Protein-1 beta, and Vascular Cell Adhesion Molecule-1) identified in the present study. **FIG. 3E** shows a patient distribution plot based on the expression levels of all of the 49 biomarkers provided in the present study.

## 4. DETAILED DESCRIPTION

### 4.1 Definitions

As used herein, the term "apremilast" refers to (+)-2-[1-(3-ethoxy-4-methoxyphenyl)-2-methylsulfonylethyl]-4-acetylaminoisoindoline-1,3-dione (also known as N-[2-[(1S)-1-(3-ethoxy-4-methoxylphenyl)-2-(methylsulfonyl)ethyl]-2,3-dihydro-1,3-dioxo-1H-isoindol-4-yl]acetamide), or a pharmaceutically acceptable salt, solvate, hydrate, co-crystal, clathrate, a polymorph, stereoisomer, or isotopologue thereof. The active enantiomer of apremilast is believed to be the (+) enantiomer. The structure of (+)-2-[1-(3-ethoxy-4-methoxyphenyl)-2-methylsulfonylethyl]-4-acetylaminoisoindoline-1,3-dione, also known as N-[2-[(1S)-1-(3-ethoxy-4-methoxylphenyl)-2-(methylsulfonyl)ethyl]-2,3-dihydro-1,3-dioxo-1H-isoindol-4-yl]acetamide is:

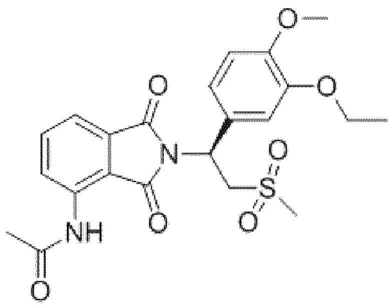

,

which may be prepared according to methods disclosed in U.S. Patent Nos. 6,962,940; 7,208,516; 7,427,638; or 7,893,101.

As used herein, the terms "compound" and "treatment compound" are used interchangeably, and include apremilast.

As used herein and unless otherwise indicated, the term "pharmaceutically acceptable salt" includes, but is not limited to, salts prepared from pharmaceutically acceptable non-toxic acids or bases including inorganic acids and bases and organic acids and bases. Suitable pharmaceutically acceptable base addition salts provided herein include metallic salts made from aluminum, calcium, lithium, magnesium, potassium, sodium and zinc or organic salts made from lysine, N,N'-dibenzylethylenediamine, chloroprocaine, choline, diethanolamine, ethyl enediamine, meglumine (N-methylglucamine) and procaine. Suitable non-toxic acids include, but are not limited to, inorganic and organic acids, such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, and methanesulfonic acids. Examples of specific salts thus include hydrochloride and mesylate salts.

As used herein and unless otherwise indicated, the term "hydrate" means a compound provided herein or a salt thereof that further includes a stoichiometric or non-stoichiometric amount of water bound by non-covalent intermolecular forces.

As used herein and unless otherwise indicated, the term "solvate" means a solvate formed from the association of one or more solvent molecules to a compound provided herein. The term "solvate" includes hydrates (*e.g.,* monohydrate, dihydrate, trihydrate, or tetrahydrate).

As used herein and unless otherwise indicated, the term "polymorph" means solid crystalline forms of a compound provided herein or complex thereof. Different polymorphs of the same compound can exhibit different physical, chemical and /or spectroscopic properties.

As used herein and unless otherwise specified, the term "prodrug" means a derivative of a compound that can hydrolyze, oxidize, or otherwise react under biological conditions (*in vitro* or *in vivo*) to provide the compound. Examples of prodrugs include, but are not limited to, derivatives and metabolites of apremilast that include biohydrolyzable moieties, such as biohydrolyzable amides, biohydrolyzable esters, biohydrolyzable carbamates, biohydrolyzable carbonates, biohydrolyzable ureides, and biohydrolyzable phosphate analogues.

As used herein, and unless otherwise specified, the term "enantiomer," "isomer" or "stereoisomer" encompasses all enantiomerically/stereomerically pure and enantiomerically/stereomerically enriched compounds provided herein.

As used herein, and unless otherwise indicated, the term "stereomerically pure" or "enantiomerically pure" means that a compound comprises one stereoisomer and is substantially free of its counter stereoisomer or enantiomer. For example, a compound is stereomerically or enantiomerically pure, when the compound contains greater than or equal to 80%, 90%, 95%, 96%, 97%, 98% or 99% of one stereoisomer, and 20%, 10%, 5%, 4%, 3%, 2%, 1% or less of the counter stereoisomer. "Substantially free of its (R) enantiomer" is encompassed by the term stereomerically pure or enantiomerically pure.

It should also be noted compounds can contain unnatural proportions of atomic isotopes at one or more of the atoms. For example, the compounds may be radiolabeled with radioactive isotopes, such as for example tritium ($^3H$), iodine-125 ($^{125}I$), sulfur-35 ($^{35}S$), or carbon-14 ($^{14}C$), or may be isotopically enriched, such as with deuterium ($^2H$), carbon-13 ($^{13}C$), or nitrogen-15 ($^{15}N$). As used herein, an "isotopologue" is an isotopically enriched compound. The term "isotopically enriched" refers to an atom having an isotopic composition other than the natural isotopic composition of that atom. "Isotopically enriched" may also refer to a compound containing at least one atom having an isotopic composition other than the natural isotopic composition of that atom. The term "isotopic composition" refers to the amount of each isotope present for a given atom. Radiolabeled and isotopically encriched compounds are useful as therapeutic agents, e.g., cancer and inflammation therapeutic agents, research reagents, e.g., binding assay reagents, and diagnostic

agents, e.g., in vivo imaging agents. All isotopic variations of the compounds as described herein, whether radioactive or not, are intended to be encompassed within the scope of the embodiments provided herein. In some embodiments, there are provided isotopologues of the compounds, for example, the isotopologues are deuterium, carbon-13, or nitrogen-15 enriched compounds.

As used herein, and unless otherwise specified, the terms "treat," "treating" and "treatment" contemplate an action that occurs while a patient is suffering from the specified disease or disorder, which reduces the severity or symptoms of the disease or disorder, or retards or slows the progression or symptoms of the disease or disorder.

As used herein, unless otherwise specified, the terms "prevent," "preventing" and "prevention" contemplate an action that occurs before a patient begins to suffer from the specified disease or disorder, which inhibits or reduces the severity or symptoms of the disease or disorder.

As used herein, and unless otherwise indicated, the terms "manage," "managing," and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

As used herein, and unless otherwise specified, the term "therapeutically effective amount" of a compound is an amount sufficient to provide a therapeutic benefit in the treatment or management of a disease or disorder, or to delay or minimize one or more symptoms associated with the presence of the disease or disorder. A therapeutically effective amount of a compound means an amount of therapeutic agent, alone or in combination with other therapies, which provides a therapeutic benefit in the treatment or management of the disease or disorder. The term "therapeutically effective amount" can encompass an amount that improves overall therapy, reduces or avoids symptoms or causes of a disease or disorder, or enhances the therapeutic efficacy of another therapeutic agent. The term also refers to the amount of a compound that is sufficient to elicit the biological or medical response of a biological molecule *(e.g.,* a protein, enzyme, RNA, or DNA), cell, tissue, system, animal, or human, which is being sought by a researcher, veterinarian, medical doctor, or clinician.

The term "responsiveness" or "responsive" when used in reference to a treatment refer to the degree of effectiveness of the treatment in lessening or decreasing the symptoms of a disease being treated. For example, the term "increased responsiveness" when used in reference to a treatment of a cell or a subject refers to an increase in the effectiveness in lessening or decreasing the symptoms of the disease when measured using any methods known in the art. In certain embodiments, the increase in the effectiveness is at least about 5%, at least about 10%, at least about 20%, at least about 30%, at least about 40%, or at least about 50%. An improvement in the disease or disorder can be characterized as a complete or partial response. "Complete response" refers to an absence of clinically detectable disease. "Partial response" refers to at least about a 10%, 20%, 30%, 40%, 50%, 60%, 70%, 80%, or 90% decrease in any symptoms of the disease. The term "treatment" contemplates both a complete and a partial response.

The term "likelihood" or "likely" generally refers to an increase in the probability of an event.

The term "predict" generally means to determine or tell in advance. When used to "predict" the effectiveness of a treatment, for example, the term "predict" can mean that the likelihood of the outcome of the treatment can be determined at the outset, before the treatment has begun, or before the treatment period has progressed substantially.

As used herein the terms "polypeptide" and "protein" as used interchangeably herein, refer to a polymer of amino acids of three or more amino acids in a serial array, linked through peptide bonds. The term "polypeptide" includes proteins, protein fragments, protein analogues, and oligopeptides. The term polypeptide as used herein can also refer to a peptide. The amino acids making up the polypeptide may be naturally derived, or may be synthetic. The polypeptide can be purified from a biological sample. The polypeptide, protein, or peptide also encompasses modified polypeptides, proteins, and peptides, *e.g.,* a glycopolypeptide, glycoprotein, or glycopeptide; or a lipopolypeptide, lipoprotein, or lipopeptide.

The term "antibody" is used herein in the broadest sense and covers fully assembled antibodies, antibody fragments which retain the ability to specifically bind to the antigen (*e.g.,* Fab, $F(ab')_2$, Fv, and other fragments), single chain antibodies, diabodies, antibody chimeras, hybrid antibodies, bispecific antibodies, and humanized antibodies. The term "antibody" covers both polyclonal and monoclonal antibodies. The term "antibody" and "immunoglobulin" or "Ig" may be used interchangeably herein. Antibodies provided herein include, but are not limited to, synthetic antibodies, monoclonal antibodies, recombinantly produced antibodies, multispecific antibodies (including bi-specific antibodies), human antibodies, humanized antibodies, chimeric antibodies, intrabodies, single-chain Fvs (scFv) (*e.g.,* including monospecific, bispecific, etc.), camelized antibodies, Fab fragments, F(ab") fragments, disulfide-linked Fvs (sdFv), anti-idiotypic (anti-Id) antibodies, and epitope-binding fragments of any of the above. In particular, antibodies provided herein include immunoglobulin molecules and immunologically active portions of immunoglobulin molecules, *i.e.,* antigen binding domains or molecules that contain an antigen-binding site that immunospecifically binds to a biomarker provided herein. The antibodies provided herein can be of any type (*e.g.,* IgG, IgE, IgM, IgD, IgA and IgY), any class (*e.g.,* IgG1, IgG2, IgG3, IgG4, IgA1 and IgA2), or any subclass (*e.g.,* IgG2a and IgG2b) of immunoglobulin molecule. In certain embodiments,

antibodies provided herein are IgG antibodies, or a class (*e.g.,* human IgG1 or IgG4) or subclass thereof.

The term "expressed" or "expression" as used herein refers to the transcription from a gene to give a ribonucleic acid molecule at least complementary in part to a region of one of the two nucleic acid strands of the gene. The term "expressed" or "expression" as used herein also refers to the translation from the RNA molecule to give a protein, a polypeptide or a portion thereof.

As used herein, the term "level" refers to the amount, accumulation, or rate of a biomarker molecule. A level can be represented, for example, by the amount or the rate of synthesis of a messenger RNA (mRNA) encoded by a gene, the amount or the rate of synthesis of a polypeptide or protein encoded by a gene, or the amount or the rate of synthesis of a biological molecule accumulated in a cell or biological fluid. The term "level" refers to an absolute amount of a molecule in a sample or to a relative amount of the molecule, determined under steady-state or non-steady-state conditions. An mRNA expressed at a higher level can be, for example, present at a level of about 5%, 10%, 20%, 30%, 40%, 50%, 60%, 70%, 90%, 100%, 200%, 300%, 500%, 1,000%, 5,000% or more of the comparative control mRNA level. An mRNA expressed at a lower level can be, for example, present at a level of about 99%, 95%, 90%, 80%, 70%, 60%, 50%, 40%, 30%, 20%, 10%, 1% or less of the comparative control mRNA level. Similarly, a polypeptide or protein biomarker at a higher level can be, for example, present at a level of about 5%, 10%, 20%, 30%, 40%, 50%, 60%, 70%, 90%, 100%, 200%, 300%, 500%, 1,000%, 5,000% or more of the comparative control protein level. A polypeptide or protein biomarker expressed at a lower level can be, for example, present at a level of about 99%, 95%, 90%, 80%, 70%, 60%, 50%, 40%, 30%, 20%, 10%, 1% or less of the comparative control protein level.

The terms "determining", "measuring", "evaluating", "assessing" and "assaying" as used herein generally refer to any form of measurement, and include determining if an element is present or not. These terms include both quantitative and/or qualitative determinations. Assessing may be relative or absolute. "Assessing the presence of' can include determining the amount of something present, as well as determining whether it is present or absent.

The terms "nucleic acid" and "polynucleotide" are used interchangeably herein to describe a polymer of any length composed of nucleotides, e.g., deoxyribonucleotides or ribonucleotides, or compounds produced synthetically, which can hybridize with naturally occurring nucleic acids in a sequence specific manner analogous to that of two naturally occurring nucleic acids, *e.g.,* can participate in Watson-Crick base pairing interactions. As used herein in the context of a polynucleotide sequence, the term "bases" (or "base") is synonymous with "nucleotides" (or "nucleotide"), *i.e.,* the monomer subunit of a polynucleotide. The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, *e.g.,* wherein one or more of the hydroxyl groups are replaced with halogen atoms or aliphatic groups, or are functionalized as ethers, amines, or the like. "Analogues" refer to molecules having structural features that are recognized in the literature as being mimetics, derivatives, having analogous structures, or other like terms, and include, for example, polynucleotides incorporating non-natural nucleotides, nucleotide mimetics, such as 2'-modified nucleosides, peptide nucleic acids, oligomeric nucleoside phosphonates, and any polynucleotide that has added substituent groups, such as protecting groups or linking moieties.

As used herein, the term "complementary" refers to specific binding between polynucleotides based on the sequences of the polynucleotides. As used herein, a first polynucleotide and a second polynucleotide are complementary if they bind to each other in a hybridization assay under stringent conditions, *e.g.* if they produce a given or detectable level of signal in a hybridization assay. Portions of polynucleotides are complementary to each other if they follow conventional base-pairing rules, *e.g.* A pairs with T (or U) and G pairs with C, although small regions (*e.g.* less than about 3 bases) of mismatch, insertion, or deleted sequence may be present.

As used herein, the term "sequence identity" or "identity" in the context of two nucleic acid sequences refers to the residues in the two sequences which are the same when aligned for maximum correspondence over a specified comparison window, and can take into consideration additions, deletions and substitutions.

As used herein, the term "substantial identity" or "homologous" in their various grammatical forms in the context of polynucleotide generally means that a polynucleotide comprises a sequence that has a desired identity, for example, at least 60% identity, at least 70% sequence identity, at least 80%, at least 90% or at least 95%, compared to a reference sequence. Another indication that nucleotide sequences are substantially identical is if two molecules hybridize to each other under stringent conditions.

As used herein, the terms "isolated" and "purified" refer to isolation of a substance (such as mRNA, antibody or protein) such that the substance comprises a substantial portion of the sample in which it resides, *i.e.* greater than the substance is typically found in its natural or un-isolated state. Typically, a substantial portion of the sample comprises, *e.g.,* greater than 1%, greater than 2%, greater than 5%, greater than 10%, greater than 20%, greater than 50%, or more, usually up to about 90%-100% of the sample. For example, a sample of isolated mRNA can typically comprise at least about 1% total mRNA. Techniques for purifying polynucleotides are well known in the art and include, for example,

gel electrophoresis, ion-exchange chromatography, affinity chromatography, flow sorting, and sedimentation according to density.

As used herein, the term "capture agent," refers to an agent that binds an mRNA or protein through an interaction that is sufficient to permit the agent to bind and concentrate the mRNA or protein from a homogeneous mixture.

As used herein, the term "probe" refers to a capture agent that is directed to a specific target mRNA biomarker sequence. Accordingly, each probe of a probe set has a respective target mRNA biomarker. A probe/target mRNA duplex is a structure formed by hybridizing a probe to its target mRNA biomarker.

As used herein, the term "nucleic acid probe" or "oligonucleotide probe" refers to a nucleic acid capable of binding to a target nucleic acid of complementary sequence, such as the mRNA biomarkers provided herein, through one or more types of chemical bonds, usually through complementary base pairing, usually through hydrogen bond formation. As used herein, a probe may include natural (*e.g.,* A, G, C, or T) or modified bases (7-deazaguanosine, inosine, etc.). In addition, the bases in a probe may be joined by a linkage other than a phosphodiester bond, so long as it does not interfere with hybridization. It will be understood by one of skill in the art that probes may bind target sequences lacking complete complementarity with the probe sequence depending upon the stringency of the hybridization conditions. The probes are can be directly labeled with isotopes, for example, chromophores, lumiphores, chromogens, or indirectly labeled with biotin to which a streptavidin complex may later bind. By assaying for the presence or absence of the probe, one can detect the presence or absence of a target mRNA biomarker of interest.

As used herein, the term "stringent assay conditions" refers to conditions that are compatible to produce binding pairs of nucleic acids, *e.g.,* probes and target mRNAs, of sufficient complementarity to provide for the desired level of specificity in the assay while being generally incompatible to the formation of binding pairs between binding members of insufficient complementarity to provide for the desired specificity. The term stringent assay conditions generally refer to the combination of hybridization and wash conditions.

As used herein, a "label" or a "detectable moiety" in reference to a nucleic acid, refers to a composition that, when linked with a nucleic acid, renders the nucleic acid detectable, for example, by spectroscopic, photochemical, biochemical, immunochemical, or chemical means. Exemplary labels include, but are not limited to, radioactive isotopes, magnetic beads, metallic beads, colloidal particles, fluorescent dyes, enzymes, biotin, digoxigenin, and haptens. A "labeled nucleic acid or oligonucleotide probe" is generally one that is bound, either covalently, through a linker or a chemical bond, or noncovalently, through ionic bonds, van der Waals forces, electrostatic attractions, hydrophobic interactions, or hydrogen bonds, to a label such that the presence of the nucleic acid or probe can be detected by detecting the presence of the label bound to the nucleic acid or probe.

As used herein, the term "sample" relates to a material or mixture of materials, typically, although not necessarily, in fluid form, containing one or more components of interest. "Biological sample" as used herein refers to a sample obtained from a biological subject, including sample of biological tissue or fluid origin, obtained, reached, or collected *in vivo* or *in situ.* A biological sample also includes samples from a region of a biological subject containing precancerous or cancer cells or tissues. Such samples can be, but are not limited to, organs, tissues, fractions and cells isolated from a mammal. Exemplary biological samples include but are not limited to cell lysate, a cell culture, a cell line, a tissue, oral tissue, gastrointestinal tissue, an organ, an organelle, a biological fluid, a blood sample, a urine sample, and a skin sample.

As used herein, a "biological marker" or "biomarker" is a substance whose amount or detection indicates a particular biological state. In another embodiment, biomarkers can either be determined individually, or several biomarkers can be measured simultaneously. Exemplary biomarkers of the present application are those described in Section 4.2 below.

As used herein, the term "about" or "approximately" means an acceptable error for a particular value as determined by one of ordinary skill in the art, which depends in part on how the value is measured or determined. In certain embodiments, the term "about" or "approximately" means within 1, 2, 3, or 4 standard deviations. In certain embodiments, the term "about" or "approximately" means within 50%, 20%, 15%, 10%, 9%, 8%, 7%, 6%, 5%, 4%, 3%, 2%, 1%, 0.5%, or 0.05% of a given value or range.

As used herein, the term "classifier" refers to a mathematical function that separates two or more groups, bodies or distributions of data according to the values with which each example of data is described, or an output of a function applied to those values in a manner that may be used to assign new data examples to one or more of the groups, bodies or distributions separated.

As used herein, the terms "subject" and "patient" refer to an animal including human being.

### 4.2 Methods of Selecting Patients and Predicting Responsiveness

The present disclosure is based, in part, on the finding that a group of patients selected based on expression level(s) of one or more biomarker(s) have a higher likelihood to be responsive to a treatment with a PDE4 inhibitor, *e.g.,* apremilast. Thus, provided herein are certain biomarkers (*e.g.,* a cytokine such as MCP1) for selecting patients for a treatment with apremilast or for predicting a patient's response for a treatment with apremilast. Also provided herein are

methods of identifying a subject having a disease or disorder, predicting the responsiveness of a subject having or suspected of having a disease or disorder, or correlating the level(s) of one or more biomarker(s) in a sample with a likelihood of the subject being responsive to a treatment with a PDE4 inhibitor.

In certain embodiments, the disease or disorder is selected from the group consisting of psoriasis, psoriatic arthritis, rheumatoid arthritis, rheumatoid spondylitis, osteoarthritis, other arthritic conditions, non-radiographic axial spondyloarthropathy, Crohn's disease, inflammatory bowel disease, ulcerative colitis, celiac disease, atopic dermatitis, and multiple sclerosis. In some embodiments, the disease or disorder is a chronic inflammatory disease. In some embodiments, the inflammatory disease is ankylosing spondylitis.

In one aspect, the level of a single biomarker is used for identifying a subject having a disease or disorder who is likely to be responsive to a treatment with apremilast, or predicting the responsiveness of a subject having or suspected of having a disease or disorder to a treatment with apremilast. In another aspect, the levels of two or more biomarkers are used for identifying a subject having a disease or disorder who is likely to be responsive to a treatment with apremilast, or predicting the responsiveness of a subject having or suspected of having a disease or disorder to a treatment with apremilast.

According to the present disclosure, the level(s) of one or more biomarker(s) can be correlated with a likelihood of a subject being responsive to a treatment with a PDE4 inhibitor. *e.g.,* apremilast. In certain embodiments, correlating involves associating the level(s) of one or more biomarker(s) measured in a subject sample with a likelihood that a patient responds to PDE4 inhibitor treatment, otherwise known as a response rate. For example, a threshold score (*e.g.,* a threshold level for one biomarker, or a threshold composite score for multiple biomarkers) using one or more biomarkers in a population having the disease or disorder is measured or calculated. Based on the threshold score, a sub-group of the patient population is identified (*e.g.,* the sub-group contains only those patients with a threshold score *e.g.,* a threshold level of X). The likelihood that a patient from this sub-group is responsive to PDE4 treatment is calculated (*i.e.* if the sub-group contains 20 patients, and 15 of those patients responded to treatment, then the sub-group has a 75% response rate). The same one or more biomarkers used to calculate the threshold score (*e.g.,* the threshold level) are measured or calculated in a sample from a patient that is going to be diagnosed or treated. The patient's biomarker level(s) or the calculation result based on which is then compared to the threshold score (*e.g.,* the threshold level of *X* used in the above example) and correlated with the calculated response rate of the sub-group depending on if the patient's biomarker level(s) or the calculation result based on which falls within or outside of *X.*

In some embodiments, when a single biomarker is used, the threshold score is a threshold level of the biomarker. More detailed description of the threshold level of a single biomarker is described in Section 4.2.1 below.

In other embodiments, when two or more biomarkers are used, the threshold score is a threshold composite score based on the levels of the two or more biomarkers. More detailed description of the threshold composite score based on the levels of two or more biomarkers is described in Section 4.2.2 below.

### 4.2.1 Using a Single Biomarker for Identifying a Subject Who is Likely to be Responsive to a PDE4 Inhibitor, or Predicting the Responsiveness of a Subject to a Treatment with a PDE4 Inhibitor

As described in Section 5.1 and 5.2 below, a single biomarker, *e.g.,* MCP1, can sufficiently differentiate a group of patients with higher response rate to PDE4 inhibitor treatment. Thus, in one aspec*t*, *p*rovided herein are methods for using a single biomarker identified in the present disclosure, *e.g.,* those listed in **Table 3**, for predicting a patient's responsiveness to a treatment with a PDE4 inhibitor, *e.g.,* apremilast.

In certain aspects of the disclosure, provided herein is a method of identifying a subject having a disease or disorder who is likely to be responsive to a treatment with apremilast, or predicting the responsiveness of a subject having or suspected of having a disease or disorder to a treatment with apremilast, comprising:

(a) obtaining a sample from the subject;
(b) measuring the level of a biomarker in the sample; and
(c) diagnosing the subject as being likely to be responsive to apremilast if the level of the biomarker in the sample is lower or higher as compared with a threshold level of the biomarker, or correlating the level of the biomarker in the sample with a likelihood of the subject being responsive to treatment with apremilast.

In some aspects of the disclosure, the biomarker is selected from the group consisting of Alpha-1-Antitrypsin, Alpha-2-Macroglobulin, Beta-2-Microglobulin, Brain-Derived Neurotrophic Factor, C-Reactive Protein, Complement C3, Eotaxin-1, Factor VII, Ferritin, Fibrinogen, Granulocyte-Macrophage Colony-Stimulating Factor, Haptoglobin, Intercellular Adhesion Molecule 1, Interferon gamma, Interleukin-1 alpha, Interleukin-1 beta, Interleukin-1 receptor antagonist, Interleukin-10, Interleukin-12 Subunit p40, Interleukin-12 Subunit p70, Interleukin-15, Interleukin-17, Interleukin-2, Interleukin-23, Interleukin-3, Interleukin-4, Interleukin-5, Interleukin-6, Interleukin-7, Interleukin-8, Macrophage Inflammatory Protein-1 alpha, Macrophage Inflammatory Protein-1 beta, Matrix Metalloproteinase-3, Matrix Metalloproteinase-9,

MCP1, Stem Cell Factor, T-Cell-Specific Protein RANTES, Tissue Inhibitor of Metalloproteinases, Tumor Necrosis Factor alpha, Tumor Necrosis Factor beta, Tumor necrosis factor receptor 2, Vascular Cell Adhesion Molecule-1, Vascular Endothelial Growth Factor, Vitamin D-Binding Protein, von Willebrand Factor, Osteoprotegerin, Interleukin-18, Receptor Activator of Nuclear Factor Kappa B Ligand (RANKL), and Sclerostin.

In other aspects of the disclosure, the biomarker is selected from the group consisting of MCP1, Interleukin-15, C-Reactive Protein, Matrix Metalloproteinase-3, Interleukin-1 receptor antagonist, von Willebrand Factor, Eotaxin-1, Alpha-1-Antitrypsin, Macrophage Inflammatory Protein-1 beta, Vascular Cell Adhesion Molecule-1, Tissue Inhibitor of Metalloproteinases 1, Osteoprotegerin, Vascular Endothelial Growth Factor, Alpha-2-Macroglobulin, Brain-Derived Neurotrophic Factor, Intercellular Adhesion Molecule 1, Interleukin-18, Matrix Metalloproteinase-9, and Vitamin D-Binding Protein.

In one embodiment, the biomarker is MCP1. In one aspect of the disclosure, the biomarker is Interleukin-15. In one aspect of the disclosure, the biomarker is C-Reactive Protein. In one aspect of the disclosure, the biomarker is Matrix Metalloproteinase-3. In one aspect of the disclosure, the biomarker is Interleukin-1 receptor antagonist. In one aspect of the disclosure, the biomarker is von Willebrand Factor. In one aspect of the disclosure, the biomarker is Eotaxin-1. In one aspect of the disclosure, the biomarker is Alpha-1-Antitrypsin. In one aspect of the disclosure, the biomarker is Macrophage Inflammatory Protein-1 beta. In one aspect of the disclosure, the biomarker is Vascular Cell Adhesion Molecule-1. In one aspect of the disclosure, the biomarker is Tissue Inhibitor of Metalloproteinases 1. In one aspect of the disclosure, the biomarker is Osteoprotegerin. In one aspect of the disclosure, the biomarker is Vascular Endothelial Growth Factor. In one aspect of the disclosure, the biomarker is Alpha-2-Macroglobulin. In one aspect of the disclosure, the biomarker is Brain-Derived Neurotrophic Factor. In one aspect of the disclosure, the biomarker is Intercellular Adhesion Molecule 1. In one aspect of the disclosure, the biomarker is Interleukin-18. In one aspect of the disclosure, the biomarker is Matrix Metalloproteinase-9. In one aspect of the disclosure, the biomarker is Vitamin D-Binding Protein.

As shown in Section 5.2 below, MCP1 is identified as the biomarker that associates with the group of ankylosing spondylitis patients which have a higher response rate to apremilast than the remaining ankylosing spondylitis patients treated with apremilast. Thus, in some specific aspects of the disclosure, provided herein is a method of identifying a subject having ankylosing spondylitis who is likely to be responsive to a treatment with apremilast, or predicting the responsiveness of a subject having or suspected of having ankylosing spondylitis to a treatment with apremilast, comprising:

(a) obtaining a sample from the subject;
(b) measuring the level of MCP1 in the sample; and
(c) diagnosing the subject as being likely to be responsive to apremilast if the level of MCP1 in the sample is lower than a threshold level of MCP1, or correlating the level of MCP1 in the sample with a likelihood of the subject being responsive to treatment with apremilast.

As shown in the Section 5.1 and **Table 1,** certain biomarkers are expressed at a lower level in the group of patients likely to be responsive to the apremilast treatment, such as MCP1, Interleukin-8, Macrophage Inflammatory Protein-1 beta, Eotaxin-1, Vascular Cell Adhesion Molecule-1, Interleukin-18, Sclerostin, Tissue Inhibitor of Metalloproteinases, and Osteoprotegerin. Other biomarkers are expressed at a higher levels in the group of patients likely to be responsive to the apremilast treatment, such as von Willebrand Factor, C-Reactive Protein, Alpha-1-Antitrypsin, Interleukin-1 receptor antagonist, Interleukin-15, Fibrinogen, Matrix Metalloproteinase-3, Complement C3, and Haptoglobin.

Thus, in some aspects of the disclosure, the subject is determined as being likely to be responsive to apremilast if the level of the biomarker in the sample is lower than a threshold level of the biomarker, and the biomarker is MCP1, Interleukin-8, Macrophage Inflammatory Protein-1 beta, Eotaxin-1, Vascular Cell Adhesion Molecule-1, Interleukin-18, Sclerostin, Tissue Inhibitor of Metalloproteinases, or Osteoprotegerin. In a specific embodiment, the biomarker is MCP1, and the subject is diagnosed as being likely to be responsive to apremilast if the level of MCP1 in the sample is lower than a threshold level of MCP1.

In some specific embodiments, the methods comprise determining the subject as being likely to be responsive to treatment with apremilast if the level of MCP1 of the patient is lower than a threshold level of MCP1, and the threshold level of the MCP1 is between about 150 pg/ml to about 70 pg/ml. In another embodiment, the threshold level of MCP1 is between about 110 pg/ml to about 75 pg/ml. In other embodiments, the threshold level of MCP1 is between about 95 pg/ml to about 75 pg/ml. In some embodiments, the threshold level of MCP1 is about 105 pg/ml, 95 pg/ml, or 75 pg/ml. In other embodiments, the threshold level of MCP1 is lower than 70 pg/ml, such as 65 pg/ml, 60 pg/ml, 55 pg/ml, 50 pg/ml, 45 pg/ml, 40 pg/ml, 30 pg/ml, 25 pg/ml, 20 pg/ml, and 10 pg/ml. In a specific embodiment, the threshold level of MCP1 is about 105 pg/ml. In another specific embodiment, the threshold level of MCP1 is about 95 pg/mll. In another specific embodiment, the threshold level of MCP1 is about 75 pg/ml.

In other aspects of the disclosure, the subject is determined as being likely to be responsive to apremilast if the level of the biomarker in the sample is higher than a threshold level of the biomarker, and the biomarker is Willebrand

Factor, C-Reactive Protein, Alpha-1-Antitrypsin, Interleukin-1 receptor antagonist, Interleukin-15, Fibrinogen, Matrix Metalloproteinase-3, Complement C3, or Haptoglobin.

It should be noted that the value of the threshold level of a single biomarker may affect the likelihood of a patient's responsiveness to the treatment with apremilast. In some embodiments, a lower threshold level of a single biomarker is correlated with a higher response rate. In other embodiments, a higher threshold level of a single biomarker is correlated with a higher response.

In certain embodiments as illustrated in Section 5.2.1 below, the threshold level of a single biomarker can be determined based on the levels of the biomarker in a population of subjects. In some embodiments, the threshold level of the biomarker is calculated as the average or median of the biomarker levels in the population. In other embodiments, the threshold level of the biomarker is determined based on the levels of the biomarker of the subjects that are responsive or are likely to be responsive to apremilast and the levels of the biomarker of those subjects that are not responsive or are not likely to be responsive to apremilast. In a more specific embodiment, the threshold level of the biomarker is determined as the mid-point between the median level of the biomarker of the patients that are likely to be responsive to apremilast and the median level of the biomarker of the patients that are not likely to be responsive to apremilast. In yet other embodiments, the threshold level of the biomarker is calculated as mean of thresholds obtained from cross validation (e.g., 3-fold, 5-fold, or 10-fold cross validation). In other embodiments, the threshold level of the biomarker is calculated as mean of thresholds obtained from 3-fold, 4-fold, 5-fold, 6-fold, 10-fold, 20-fold, or 50-fold cross validation. In one embodiment, the threshold level of the biomarker is calculated as mean of thresholds obtained from 3-fold cross validation. In other embodiments, the threshold level of the biomarker is calculated as mean of thresholds obtained from 4-fold cross validation. In yet other embodiments, the threshold level of the biomarker is calculated as mean of thresholds obtained from 5-fold cross validation. In yet other embodiments, the threshold level of the biomarker is calculated as mean of thresholds obtained from 6-fold cross validation. In yet other embodiments, the threshold level of the biomarker is calculated as mean of thresholds obtained from 10-fold cross validation. In yet other embodiments, the threshold level of the biomarker is calculated as mean of thresholds obtained from 20-fold cross validation. In yet other embodiments, the threshold level of the biomarker is calculated as mean of thresholds obtained from 50-fold cross validation. The fold number can be more than 50, and can be various depending on the size of the population.

In certain aspects of the disclosure, the subject is diagnosed as having more than 50% to 90% likelihood to be responsive to a treatment with apremilast, if the level of the biomarker is lower or higher as compared with the threshold level of the biomarker. In one aspect of the disclosure, the subject is diagnosed as having more than 50% likelihood to be responsive to a treatment with apremilast, if the level of the biomarker is lower or higher as compared with the threshold level of the biomarker. In another aspect of the disclosure, the subject is diagnosed as having more than 60% likelihood to be responsive to a treatment with apremilast, if the level of the biomarker is lower or higher as compared with the threshold level of the biomarker. In another aspect of the disclosure, the subject is diagnosed as having more than 70% likelihood to be responsive to a treatment with apremilast, if the level of the biomarker is lower or higher as compared with the threshold level of the biomarker. In yet another aspect of the disclosure, the subject is diagnosed as having more than 80% likelihood to be responsive to a treatment with apremilast, if the level of the biomarker is lower or higher as compared with the threshold level of the biomarker. In yet another aspect of the disclosure, the subject is diagnosed as having more than 90% likelihood to be responsive to a treatment with apremilast, if the level of the biomarker is lower or higher as compared with the threshold level of the biomarker.

In some more specific embodiments, the biomarker is MCP1, and the subject is diagnosed as having more than 50% likelihood to be responsive to apremilast if the level of MCP1 is lower than the threshold level of MCP1. In some more specific embodiments, the biomarker is MCP1, and the subject is diagnosed as having more than 55% likelihood to be responsive to apremilast if the level of MCP1 is lower than the threshold level of MCP1. In some more specific embodiments, the biomarker is MCP1, and the subject is diagnosed as having more than 60% likelihood to be responsive to apremilast if the level of MCP1 is lower than the threshold level of MCP1. In some more specific embodiments, the biomarker is MCP1, and the subject is diagnosed as having more than 65% likelihood to be responsive to apremilast if the level of MCP1 is lower than the threshold level of MCP1. In some more specific embodiments, the biomarker is MCP1, and the subject is diagnosed as having more than 70% likelihood to be responsive to apremilast if the level of MCP1 is lower than the threshold level of MCP1. In some more specific embodiments, the biomarker is MCP1, and the subject is diagnosed as having more than 80% likelihood to be responsive to apremilast if the level of MCP1 is lower than the threshold level of MCP1. In some more specific embodiments, the biomarker is MCP1, and the subject is diagnosed as having more than 90% likelihood to be responsive to apremilast if the level of MCP1 is lower than the threshold level of MCP1.

### 4.2.2 Using Multiple Biomarkers for Identifying a Subject Who is Likely to be Responsive to a PDE4 Inhibitor, or Predicting the Responsiveness of a Subject to a Treatment with a PDE4 Inhibitor

As described in Sections 5.1 and 5.3 below, an expression profile of 49 biomarkers or a subset thereof (multiple

biomarkers) can be used to differentiate a group of patients having higher response rate to apremilast. For example, the combination of the top 2 biomarkers identified in the present study, the combination of the top 5 biomarkers, the combination of the top 10 biomarkers, or the combination of all the 49 biomarkers can be used to group a patient for the purpose of predicting a patient's response, as demonstrated in Section 5.3 and **FIGS. 3B-3E.**

Thus, also disclosed herein are methods of using multiple biomarkers (two or more biomarkers) for identifying a subject who is likely to be responsive to apremilast, or predicting the responsiveness of a subject to a treatment with apremilast. In certain aspects of the disclosure, provided herein is a method of identifying a subject having a disease or disorder who is likely to be responsive to a treatment with apremilast, or predicting the responsiveness of a subject having or suspected of having a disease or disorder to a treatment with apremilast, comprising:

(a) obtaining a sample from the subject;
(b) measuring the levels of two or more biomarkers in the sample; and
(c) correlating the levels of two or more biomarkers with a likelihood of the subject being responsive to treatment with apremilast.

In some aspects of the disclosure, 2 to 49 biomarkers are used to predict a patient's response. Any range therein is also contemplated. In one aspect of the disclosure, 2 biomarkers are used to predict a patient's response. In one aspect of the disclosure, 3 biomarkers are used to predict a patient's response. In one aspect of the disclosure, 4 biomarkers are used to predict a patient's response. In one aspect of the disclosure, 5 biomarkers are used to predict a patient's response. In another aspect of the disclosure, 6 biomarkers are used to predict a patient's response. In another aspect of the disclosure, 7 biomarkers are used to predict a patient's response. In yet another aspect of the disclosure, 8 biomarkers are used to predict a patient's response. In yet another aspect of the disclosure, 9 biomarkers are used to predict a patient's response. In yet another aspect of the disclosure, 10 biomarkers are used to predict a patient's response. In yet another aspect of the disclosure, 49 biomarkers are used to predict a patient's response.

In some aspects of the disclosure, the two or more biomarkers are independently selected from the group consisting of Alpha-1-Antitrypsin, Alpha-2-Macroglobulin, Beta-2-Microglobulin, Brain-Derived Neurotrophic Factor, C-Reactive Protein, Complement C3, Eotaxin-1, Factor VII, Ferritin, Fibrinogen, Granulocyte-Macrophage Colony-Stimulating Factor, Haptoglobin, Intercellular Adhesion Molecule 1, Interferon gamma, Interleukin-1 alpha, Interleukin-1 beta, Interleukin-1 receptor antagonist, Interleukin-10, Interleukin-12 Subunit p40, Interleukin-12 Subunit p70, Interleukin-15, Interleukin-17, Interleukin-2, Interleukin-23, Interleukin-3, Interleukin-4, Interleukin-5, Interleukin-6, Interleukin-7, Interleukin-8, Macrophage Inflammatory Protein-1 alpha, Macrophage Inflammatory Protein-1 beta, Matrix Metalloproteinase-3, Matrix Metalloproteinase-9, MCP1, Stem Cell Factor, T-Cell-Specific Protein RANTES, Tissue Inhibitor of Metalloproteinases, Tumor Necrosis Factor alpha, Tumor Necrosis Factor beta, Tumor necrosis factor receptor 2, Vascular Cell Adhesion Molecule-1, Vascular Endothelial Growth Factor, Vitamin D-Binding Protein, von Willebrand Factor, Osteoprotegerin, Interleukin-18, RANKL, and Sclerostin.

In one aspect of the disclosure, the two or more biomarkers are independently selected from the group consisting of MCP1, Interleukin-15, C-Reactive Protein, Matrix Metalloproteinase-3, Interleukin-1 receptor antagonist, von Willebrand Factor, Eotaxin-1, Alpha-1-Antitrypsin, Macrophage Inflammatory Protein-1 beta, Vascular Cell Adhesion Molecule-1, Tissue Inhibitor of Metalloproteinases 1, Osteoprotegerin, Vascular Endothelial Growth Factor, Alpha-2-Macroglobulin, Brain-Derived Neurotrophic Factor, Intercellular Adhesion Molecule 1, Interleukin-18, Matrix Metalloproteinase-9, and Vitamin D-Binding Protein.

In the present invention, one of the two or more biomarkers is MCP1, thus in another aspect of the disclosure, provided herein is a method of identifying a subject having a disease or disorder who is likely to be responsive to apremilast, or predicting the responsiveness of a subject having or suspected of having a disease or disorder to apremilast, comprising:

(a) obtaining a sample from the subject;
(b) determining the levels of two or more biomarkers in the sample, wherein one of the two or more biomarkers is MCP1;
(c) correlating the levels of the two or more biomarkers with a likelihood of the subject being responsive to treatment with apremilast.

In some embodiments, the two or more cytokines include MCP1 and Interleukin-15. Thus, in some specific aspects of the disclosure, provided herein is a method of identifying a subject having a disease or disorder who is likely to be responsive to apremilast, or predicting the responsiveness of a subject having or suspected of having a disease or disorder to apremilast, comprising:

(a) obtaining a sample from the subject;

(b) determining the levels of MCP1 and Interleukin-15;
(c) correlating the levels of MCP1 and Interleukin-15 with a likelihood of the subject being responsive to treatment with apremilast.

In some embodiments, the two or more cytokines include MCP1, Interleukin-15, and C-Reactive Protein. Thus, in some specific aspects of the disclosure, provided herein is a method of identifying a subject having a disease or disorder who is likely to be responsive to apremilast, or predicting the responsiveness of a subject having or suspected of having a disease or disorder to apremilast, comprising:

(a) obtaining a sample from the subject;
(b) determining the levels of MCP1, Interleukin-15, and C-Reactive Protein;
(c) correlating the levels of MCP1, Interleukin-15, and C-Reactive Protein with a likelihood of the subject being responsive to treatment with apremilast.

In other embodiments, the two or more cytokines are MCP1, Interleukin-15, C-Reactive Protein, and Matrix Metalloproteinase-3. Thus, in some specific aspects of the disclosure, provided herein is a method of identifying a subject having a disease or disorder who is likely to be responsive to apremilast, or predicting the responsiveness of a subject having or suspected of having a disease or disorder to apremilast, comprising:

(a) obtaining a sample from the subject;
(b) determining the levels of MCP1, Interleukin-15, C-Reactive Protein, and Matrix Metalloproteinase-3;
(c) correlating the levels of MCP1, Interleukin-15, C-Reactive Protein, and Matrix Metalloproteinase-3 with a likelihood of the subject being responsive to treatment with apremilast.

In other embodiments, the two or more cytokines are MCP1, Interleukin-15, C-Reactive Protein, Matrix Metalloproteinase-3, and Interleukin-1 receptor antagonist. Thus, in some specific aspects of the disclosure, provided herein is a method of identifying a subject having a disease or disorder who is likely to be responsive to apremilast, or predicting the responsiveness of a subject having or suspected of having a disease or disorder to apremilast, comprising:

(a) obtaining a sample from the subject;
(b) determining the levels of MCP1, Interleukin-15, C-Reactive Protein, Matrix Metalloproteinase-3, and Interleukin-1 receptor antagonist;
(c) correlating the levels of MCP1, Interleukin-15, C-Reactive Protein, Matrix Metalloproteinase-3, and Interleukin-1 receptor antagonist with a likelihood of the subject being responsive to treatment with apremilast.

In yet other embodiments, the two or more cytokines are MCP1, Interleukin-15, C-Reactive Protein, Matrix Metalloproteinase-3, Interleukin-1 receptor antagonist, and von Willebrand Factor. Thus, in some specific aspects of the disclosure, provided herein is a method of identifying a subject having a disease or disorder who is likely to be responsive to apremilast, or predicting the responsiveness of a subject having or suspected of having a disease or disorder to apremilast, comprising:

(a) obtaining a sample from the subject;
(b) determining the levels of MCP1, Interleukin-15, C-Reactive Protein, Matrix Metalloproteinase-3, Interleukin-1 receptor antagonist, and von Willebrand Factor;
(c) correlating the levels of MCP1, Interleukin-15, C-Reactive Protein, Matrix Metalloproteinase-3, Interleukin-1 receptor antagonist, and von Willebrand Factor with a likelihood of the subject being responsive to treatment with apremilast.

In yet other embodiments, the two or more cytokines are MCP1, Interleukin-15, C-Reactive Protein, Matrix Metalloproteinase-3, Interleukin-1 receptor antagonist, von Willebrand Factor, and Eotaxin-1. Thus, in some specific aspects of the disclosure, provided herein is a method of identifying a subject having a disease or disorder who is likely to be responsive to apremilast, or predicting the responsiveness of a subject having or suspected of having a disease or disorder to apremilast, comprising:

(a) obtaining a sample from the subject;
(b) determining the levels of MCP1, Interleukin-15, C-Reactive Protein, Matrix Metalloproteinase-3, Interleukin-1 receptor antagonist, von Willebrand Factor, and Eotaxin-1;
(c) correlating the levels of MCP1, Interleukin-15, C-Reactive Protein, Matrix Metalloproteinase-3, Interleukin-1

receptor antagonist, von Willebrand Factor, and Eotaxin-1 with a likelihood of the subject being responsive to treatment with apremilast.

In yet other embodiments, the two or more cytokines include MCP1, Interleukin-15, C-Reactive Protein, Matrix Metalloproteinase-3, Interleukin-1 receptor antagonist, von Willebrand Factor, Eotaxin-1, and Alpha-1-Antitrypsin. Thus, in some specific aspects of the disclosure, provided herein is a method of identifying a subject having a disease or disorder who is likely to be responsive to apremilast, or predicting the responsiveness of a subject having or suspected of having a disease or disorder to apremilast, comprising:

(a) obtaining a sample from the subject;
(b) determining the levels of MCP1, Interleukin-15, C-Reactive Protein, Matrix Metalloproteinase-3, Interleukin-1 receptor antagonist, von Willebrand Factor, Eotaxin-1, and Alpha-1-Antitrypsin;
(c) correlating the levels of MCP1, Interleukin-15, C-Reactive Protein, Matrix Metalloproteinase-3, Interleukin-1 receptor antagonist, von Willebrand Factor, Eotaxin-1, and Alpha-1-Antitrypsin with a likelihood of the subject being responsive to treatment with apremilast.

In yet other embodiments, the two or more cytokines include MCP1, Interleukin-15, C-Reactive Protein, Matrix Metalloproteinase-3, Interleukin-1 receptor antagonist, von Willebrand Factor, Eotaxin-1, Alpha-1-Antitrypsin, and Macrophage Inflammatory Protein-1 beta. Thus, in some specific aspects of the disclosure, provided herein is a method of identifying a subject having a disease or disorder who is likely to be responsive to apremilast, or predicting the responsiveness of a subject having or suspected of having a disease or disorder to apremilast, comprising:

(a) obtaining a sample from the subject;
(b) determining the levels of MCP1, Interleukin-15, C-Reactive Protein, Matrix Metalloproteinase-3, Interleukin-1 receptor antagonist, von Willebrand Factor, Eotaxin-1, Alpha-1-Antitrypsin, and Macrophage Inflammatory Protein-1 beta;
(c) correlating the levels of MCP1, Interleukin-15, C-Reactive Protein, Matrix Metalloproteinase-3, Interleukin-1 receptor antagonist, von Willebrand Factor, Eotaxin-1, Alpha-1-Antitrypsin, and Macrophage Inflammatory Protein-1 beta with a likelihood of the subject being responsive to treatment with apremilast.

In yet other embodiments, the two or more cytokines include MCP1, Interleukin-15, C-Reactive Protein, Matrix Metalloproteinase-3, Interleukin-1 receptor antagonist, von Willebrand Factor, Eotaxin-1, Alpha-1-Antitrypsin, Macrophage Inflammatory Protein-1 beta, and Vascular Cell Adhesion Molecule-1. Thus, in some specific aspects of the disclosure, provided herein is a method of identifying a subject having a disease or disorder who is likely to be responsive to apremilast, or predicting the responsiveness of a subject having or suspected of having a disease or disorder to apremilast, comprising:

(a) obtaining a sample from the subject;
(b) determining the levels of MCP1, Interleukin-15, C-Reactive Protein, Matrix Metalloproteinase-3, Interleukin-1 receptor antagonist, von Willebrand Factor, Eotaxin-1, Alpha-1-Antitrypsin, Macrophage Inflammatory Protein-1 beta, and Vascular Cell Adhesion Molecule-1;
(c) correlating the levels of MCP1, Interleukin-15, C-Reactive Protein, Matrix Metalloproteinase-3, Interleukin-1 receptor antagonist, von Willebrand Factor, Eotaxin-1, Alpha-1-Antitrypsin, Macrophage Inflammatory Protein-1 beta, and Vascular Cell Adhesion Molecule-1 with a likelihood of the subject being responsive to treatment with apremilast.

In certain aspects of the disclosure the step of correlating the levels of the two or more biomarkers with a likelihood of the subject being responsive to treatment with apremilast comprises (a) generating a threshold composite score based upon the levels of two or more biomarker levels measured in a population having the disease or disorder; and (b) determining the likelihood of a subject being responsive to apremilast by comparing the threshold composite score to the composite score of the subject.

In more specific aspects of the disclosure, the step of correlating the levels of the two or more biomarkers with a likelihood of the subject being responsive to treatment with apremilast comprises (a) determining the expression profile of the biomarkers provided herein or a subset thereof in a biological sample from a population of subjects that previously have been administered with apremilast, wherein each subset of the biomarkers corresponds to biomarkers differentially expressed by the subjects; (b) generating a composite score for the biomarkers or the subset thereof for each subject of the population; (c) differentiating the subjects that are responsive to or are likely to be responsive to apremilast from those subjects that are not responsive to or are not likely to be responsive to apremilast; and (d) determining responsiveness to apremilast based on the composite scores for the subjects that are responsive to or are likely to be responsive to apremilast and those subjects that are not responsive to or are not likely to be responsive to apremilast.

In another more specific embodiments, the step of correlating the levels of the two or more biomarkers with a likelihood of the subject being responsive to treatment with apremilast comprises (a) determining the expression profile of the biomarkers provided herein or a subset thereof in a biological sample from a population of subjects that previously have been administered with apremilast, wherein each subset of the biomarkers corresponds to biomarkers differentially expressed by the subjects; (b) generating a composite score for the biomarkers or the subset thereof for each subject of the population; (c) differentiating the subjects that are responsive to or are likely to be responsive to apremilast from those subjects that are not responsive to or are not likely to be responsive to apremilast; (d) generating a threshold composite score that is predictive of the responsiveness of a subject to apremilast using a model based on the composite scores for the subjects that are responsive to or are likely to be responsive to apremilast and those subjects that are not responsive to are not likely to be responsive to apremilast, and (e) determining the likelihood of a subject being responsive to apremilast by comparing the threshold composite score to the composite score of the subject.

In some embodiments, a classifier can be used to correlate the levels of the two or more biomarkers with a likelihood of the subject being responsive to treatment with apremilast. Any classifier that classifies based on two or more features (*i.e.,* assigning a sample into a group based on two or more features of the sample) can be used in the present disclosure. In some embodiments, the classifier is a supervised classification algorithm. Unlimited examples of such classifiers include standard SVM, random forest, decision trees, rule based classifiers and discriminate analysis. Other classifiers, known to those skilled in the art, that can analyze the expression profile of multiple biomarkers from a population in order to "learn" a decision boundary between the patients likely to be responsive to a treatment compound and those patients not likely to be responsive to the treatment compound, are also included in the present disclosure.

In a specific embodiment, the classifier is Naive Bayes, and Naive Bayes is used to classify a patient based on two or more biomarker levels. In another specific embodiment, the classifier is Weighted RSVM, and Weighted RSVM is used to classify a patient based on two or more biomarker levels.

As explained in Section 5.3, Naive Bayes learns a probabilistic model between features and cluster membership that maximizes the posterior probability of the observed subgroups given the features (assuming independence of all features). The formula for Naive Bayes is:

$$\hat{y} = \underset{k\in\{1,\ldots,K\}}{\operatorname{argmax}} \; p(C_k)\prod_{i=1}^{n} p(x_i|C_k),$$

where p(Ck) represents overall class (MCP1 low cluster membership) probability, K is number of classes (here two), n is the number of features (number of biomarkers used (2 for Top 2, etc)), x are the features themselves and p(xi|Ck) is the posterior probability of observing $x_i$ given class $C_k$.

SVM (support vector machine) is a classifier that draws a boundary between MCP1 low and non-MCPIlow clusters. Weighted RSVM is weighted reduced SVM. Reduced version of SVM uses a subset of the data to improve efficiency and generalization of the model. Weighted version balances classes of different sizes. The formula of SVM is

$$f(\vec{x}) = \operatorname{sign}(\sum \alpha_i y_i K(\vec{x}_i, \vec{x}) + b)$$

where $x_i$ is a sample represented by a vector of features and x is all the samples, K is a kernel matrix which encodes the distance between all samples, $y_i$ is the cluster membership for sample i, alpha is an indicator variable that describes which samples are closest to the classification boundary and b is an offset.

In some embodiments, the levels of the two or more biomarkers are correlated with a greater than 50%, 60%, 70%, 80% or 90% likelihood of the subject being responsive to apremilast. In some embodiments, the levels of the two or more biomarkers are correlated with a greater than 50% likelihood of the subject being responsive to apremilast. In one embodiment, the levels of the two or more biomarkers are correlated with a greater than 60% likelihood of the subject being responsive to apremilast. In another embodiment, the levels of the two or more biomarkers are correlated with a greater than 70% likelihood of the subject being responsive to apremilast. In some embodiments, the levels of the two or more biomarkers are correlated with a greater than 80% likelihood of the subject being responsive to apremilast. In one embodiment, the levels of the two or more biomarkers are correlated with a greater than 90% likelihood of the subject being responsive to apremilast.

**4.3 Methods of Treatment, Management or Prevention**

In another aspect of the disclosure, provided herein are methods of treating, managing and/or preventing an inflammatory disease or related disorders, which comprise administering to a patient in need of such treatment, management or prevention a therapeutically or prophylactically effective amount of a PDE4 inhibitor, e.g., apremilast. In one aspect of the disclosure, the method is a method of treating an inflammatory disease or a related disorder. In one aspect of the disclosure, the method is a method of managing an inflammatory disease or a related disorder. In one aspect of the disclosure, the method is a method of preventing an inflammatory disease or a related disorder. In the present invention, the inflammatory disease is ankylosing spondylitis.

In some embodiments, provided herein is apremilast for use in methods for treating a subject determined to be likely to be responsive to apremilast with apremilast. In one embodiment of apremilast for use in the various methods provided herein, the methods comprise administering apremilast to the subject determined to be likely to be responsive to apremilast using the methods provided herein, *e.g.,* those described in Section 4.2. Thus, in some aspects of the disclosure, provided herein is a method of treating a subject, comprising:

(a) identifying the subject as having a disease or disorder that may be responsive to a treatment with apremilast, comprising:

(i) obtaining a sample from the subject;
(ii) measuring the level(s) of one or more biomarker(s) in the sample; and
(iii) correlating the level(s) of the one or more biomarker(s) with a likelihood of the subject being responsive to treatment with apremilast; and

(b) administering the subject a therapeutically effective amount of apremilast if the subject is identified as being likely to be responsive to treatment with apremilast.

In some embodiments, a single biomarker is used for predicting a patient's response. In one aspect of the disclosure, the biomarker is selected from the group consisting of Alpha-1-Antitrypsin, Alpha-2-Macroglobulin, Beta-2-Microglobulin, Brain-Derived Neurotrophic Factor, C-Reactive Protein, Complement C3, Eotaxin-1, Factor VII, Ferritin, Fibrinogen, Granulocyte-Macrophage Colony-Stimulating Factor, Haptoglobin, Intercellular Adhesion Molecule 1, Interferon gamma, Interleukin-1 alpha, Interleukin-1 beta, Interleukin-1 receptor antagonist, Interleukin-10, Interleukin-12 Subunit p40, Interleukin-12 Subunit p70, Interleukin-15, Interleukin-17, Interleukin-2, Interleukin-23, Interleukin-3, Interleukin-4, Interleukin-5, Interleukin-6, Interleukin-7, Interleukin-8, Macrophage Inflammatory Protein-1 alpha, Macrophage Inflammatory Protein-1 beta, Matrix Metalloproteinase-3, Matrix Metalloproteinase-9, MCP1, Stem Cell Factor, T-Cell-Specific Protein RANTES, Tissue Inhibitor of Metalloproteinases, Tumor Necrosis Factor alpha, Tumor Necrosis Factor beta, Tumor necrosis factor receptor 2, Vascular Cell Adhesion Molecule-1, Vascular Endothelial Growth Factor, Vitamin D-Binding Protein, von Willebrand Factor, Osteoprotegerin, Interleukin-18, RANKL, and Sclerostin.

In another aspect of the disclosure, the biomarker is selected from the group consisting of MCP1, Interleukin-15, C-Reactive Protein, Matrix Metalloproteinase-3, Interleukin-1 receptor antagonist, von Willebrand Factor, Eotaxin-1, Alpha-1-Antitrypsin, Macrophage Inflammatory Protein-1 beta, Vascular Cell Adhesion Molecule-1, Tissue Inhibitor of Metalloproteinases 1, Osteoprotegerin, Vascular Endothelial Growth Factor, Alpha-2-Macroglobulin, Brain-Derived Neurotrophic Factor, Intercellular Adhesion Molecule 1, Interleukin-18, Matrix Metalloproteinase-9, and Vitamin D-Binding Protein.

In the present invention, the biomarker is MCP1. In one aspect of the disclosure, the biomarker is Interleukin-15. In another aspect of the disclosure, the biomarker is C-Reactive Protein. In a specific aspect of the disclosure, the biomarker is Matrix Metalloproteinase-3. In one aspect of the disclosure, the biomarker is Interleukin-1 receptor antagonist. In a specific aspect of the disclosure, the biomarker is von Willebrand Factor. In a specific aspect of the disclosure, the biomarker is Eotaxin-1. In one aspect of the disclosure, the biomarker is Alpha-1-Antitrypsin. In another aspect of the disclosure, the biomarker is Macrophage Inflammatory Protein-1 beta. In a specific aspect of the disclosure, the biomarker is Vascular Cell Adhesion Molecule-1. In one aspect of the disclosure, the biomarker is Tissue Inhibitor of Metalloproteinases 1. In a specific aspect of the disclosure, the biomarker is Osteoprotegerin. In another aspect of the disclosure, the biomarker is Vascular Endothelial Growth Factor. In one aspect of the disclosure, the biomarker is Alpha-2-Macroglobulin. In a specific aspect of the disclosure, the biomarker is Brain-Derived Neurotrophic Factor. In a specific aspect of the disclosure, the biomarker is Intercellular Adhesion Molecule 1. In one aspect of the disclosure, the biomarker is Interleukin-18. In another aspect of the disclosure, the biomarker is Matrix Metalloproteinase-9. In a specific aspect of the disclosure, the biomarker is Vitamin D-Binding Protein.

In some aspects of the disclosure, the subject is diagnosed as being likely to be responsive to apremilast if the level of the biomarker in the sample is lower than a threshold level of the biomarker, and the biomarker is MCP1,

Interleukin-8, Macrophage Inflammatory Protein-1 beta, Eotaxin-1, Vascular Cell Adhesion Molecule-1, Interleukin-18, Sclerostin, Tissue Inhibitor of Metalloproteinases, or Osteoprotegerin. In the present invention, the biomarker is MCP1, and the subject is diagnosed as being likely to be responsive to the treatment compound if the level of MCP1 in the sample is lower than a threshold level of MCP1.

In other aspects of the disclosure, the subject is diagnosed as being likely to be responsive to apremilast if the level of the biomarker in the sample is higher than a threshold level of the biomarker, and the biomarker is Willebrand Factor, C-Reactive Protein, Alpha-1-Antitrypsin, Interleukin-1 receptor antagonist, Interleukin-15, Fibrinogen, Matrix Metalloproteinase-3, Complement C3, or Haptoglobin.

In another embodiment, the threshold level of the biomarker is determined based on the levels of the biomarker in a population of subjects. In other embodiments, the threshold level of the biomarker is calculated as the average or median of the biomarker levels in the population. In other embodiments, the threshold level of the biomarker is determined based on the levels of the biomarker of the subjects that are responsive to apremilast and the levels of the biomarker of those subjects that are not responsive to apremilast. In a more specific embodiment, the threshold level of the biomarker is determined as the mid-point between the median level of the biomarker of the patients that are likely to be responsive to apremilast and the median level of the biomarker of the patients that are not likely to be responsive to apremilast. In yet other embodiments, the threshold level of the biomarker is calculated as mean of thresholds obtained from cross validation (*e.g.,* 3-fold, 5-fold, or 10-fold cross validation). Other statistic methods commonly used for classification based on one feature known to those skilled in the art are also included in the present disclosure.

In some specific embodiments, the methods comprise determining the subject as being likely to be responsive to treatment with apremilast if the level of MCP1 of the patient is lower than a threshold level of MCP1, and the threshold level of the MCP1 is between about 150 pg/ml to about 70 pg/ml. In another embodiment, the threshold level of MCP1 is between about 110 pg/ml to about 75 pg/ml. In other embodiments, the threshold level of MCP1 is between about 95 pg/ml to about 75 pg/ml. In some embodiments, the threshold level of MCP1 is about 105 pg/ml, 95 pg/ml, or 75 pg/ml. In other embodiments, the threshold level of MCP1 is lower than 70 pg/ml, such as 65 pg/ml, 60 pg/ml, 55 pg/ml, 50 pg/ml, 45 pg/ml, 40 pg/ml, 30 pg/ml, 25 pg/ml, 20 pg/ml, and 10 pg/ml. In a specific embodiment, the threshold level of MCP1 is about 105 pg/ml. In another specific embodiment, the threshold level of MCP1 is about 95 pg/ml. In another specific embodiment, the threshold level of MCP1 is about 75 pg/ml.

In certain aspects of the disclosure, the subject is diagnosed as having more than 50% to 90% likelihood to be responsive to a treatment with apremilast, if the level of the biomarker is lower or higher as compared with the threshold level of the biomarker. In one aspect of the disclosure, the subject is diagnosed as having more than 50% likelihood to be responsive to a treatment with apremilast, if the level of the biomarker is lower or higher as compared with the threshold level of the biomarker. In another aspect of the disclosure, the subject is diagnosed as having more than 60% likelihood to be responsive to a treatment with apremilast, if the level of the biomarker is lower or higher as compared with the threshold level of the biomarker. In another aspect of the disclosure, the subject is diagnosed as having more than 70% likelihood to be responsive to a treatment with apremilast, if the level of the biomarker is lower or higher as compared with the threshold level of the biomarker. In yet another aspect of the disclosure, the subject is diagnosed as having more than 80% likelihood to be responsive to a treatment with apremilast, if the level of the biomarker is lower or higher as compared with the threshold level of the biomarker. In yet another aspect of the disclosure, the subject is diagnosed as having more than 90% likelihood to be responsive to a treatment with apremilast, if the level of the biomarker is lower or higher as compared with the threshold level of the biomarker.

In some more specific embodiments, the biomarker is MCP1, and the subject is diagnosed as having more than 50% likelihood to be responsive to apremilast if the level of MCP1 is lower than the threshold level of MCP1. In some more specific embodiments, the biomarker is MCP1, and the subject is diagnosed as having more than 55% likelihood to be responsive to apremilast if the level of MCP1 is lower than the threshold level of MCP1. In some more specific embodiments, the biomarker is MCP1, and the subject is diagnosed as having more than 60% likelihood to be responsive to apremilast if the level of MCP1 is lower than the threshold level of MCP1. In some more specific embodiments, the biomarker is MCP1, and the subject is diagnosed as having more than 65% likelihood to be responsive to apremilast if the level of MCP1 is lower than the threshold level of MCP1. In some more specific embodiments, the biomarker is MCP1, and the subject is diagnosed as having more than 70% likelihood to be responsive to apremilast if the level of MCP1 is lower than the threshold level of MCP1. In some more specific embodiments, the biomarker is MCP1, and the subject is diagnosed as having more than 80% likelihood to be responsive to apremilast if the level of MCP1 is lower than the threshold level of MCP1. In some more specific embodiments, the biomarker is MCP1, and the subject is diagnosed as having more than 90% likelihood to be responsive to apremilast if the level of MCP1 is lower than the threshold level of MCP1.

It should be noted that the value of the threshold level of a single biomarker may affect the likelihood of a patient's responsiveness to the treatment with apremilast. In some embodiments, a lower threshold level of a single biomarker is correlated with a higher response rate. In other embodiments, a higher threshold level of a single biomarker correlated with a higher response.

In aspects of the disclosure, two or more biomarkers are used to predict a patient's response. In another aspect of the disclosure, 2 to 49 biomarkers are used to predict a patient's response. In one aspect of the disclosure, 2 biomarkers are used to predict a patient's response. In some aspects of the disclosure, 3 biomarkers are used to predict a patient's response. In one aspect of the disclosure, 4 biomarkers are used to predict a patient's response. In other aspects of the disclosure, 5 biomarkers are used to predict a patient's response. In other aspects of the disclosure, 6 biomarkers are used to predict a patient's response. In other aspects of the disclosure, 7 biomarkers are used to predict a patient's response. In yet other aspects of the disclosure, 8 biomarkers are used to predict a patient's response. In yet other aspects of the disclosure, 9 biomarkers are used to predict a patient's response. In yet other aspects of the disclosure, 10 biomarkers are used to predict a patient's response. In yet other aspects of the disclosure, 49 biomarkers are used to predict a patient's response.

In some aspects of the disclosure, the two or more biomarkers are independently selected from the group consisting of Alpha-1-Antitrypsin, Alpha-2-Macroglobulin, Beta-2-Microglobulin, Brain-Derived Neurotrophic Factor, C-Reactive Protein, Complement C3, Eotaxin-1, Factor VII, Ferritin, Fibrinogen, Granulocyte-Macrophage Colony-Stimulating Factor, Haptoglobin, Intercellular Adhesion Molecule 1, Interferon gamma, Interleukin-1 alpha, Interleukin-1 beta, Interleukin-1 receptor antagonist, Interleukin-10, Interleukin-12 Subunit p40, Interleukin-12 Subunit p70, Interleukin-15, Interleukin-17, Interleukin-2, Interleukin-23, Interleukin-3, Interleukin-4, Interleukin-5, Interleukin-6, Interleukin-7, Interleukin-8, Macrophage Inflammatory Protein-1 alpha, Macrophage Inflammatory Protein-1 beta, Matrix Metalloproteinase-3, Matrix Metalloproteinase-9, MCP1, Stem Cell Factor, T-Cell-Specific Protein RANTES, Tissue Inhibitor of Metalloproteinases, Tumor Necrosis Factor alpha, Tumor Necrosis Factor beta, Tumor necrosis factor receptor 2, Vascular Cell Adhesion Molecule-1, Vascular Endothelial Growth Factor, Vitamin D-Binding Protein, von Willebrand Factor, Osteoprotegerin, Interleukin-18, RANKL, and Sclerostin.

In another aspect of the disclosure, the two or more biomarkers are independently selected from the group consisting of MCP1, Interleukin-15, C-Reactive Protein, Matrix Metalloproteinase-3, Interleukin-1 receptor antagonist, von Willebrand Factor, Eotaxin-1, Alpha-1-Antitrypsin, Macrophage Inflammatory Protein-1 beta, Vascular Cell Adhesion Molecule-1, Tissue Inhibitor of Metalloproteinases 1, Osteoprotegerin, Vascular Endothelial Growth Factor, Alpha-2-Macroglobulin, Brain-Derived Neurotrophic Factor, Intercellular Adhesion Molecule 1, Interleukin-18, Matrix Metalloproteinase-9, and Vitamin D-Binding Protein.

In the present invention, one of the two or more biomarkers is MCP1. In one embodiment, the two or more biomarkers include MCP1 and Interleukin-15. In some embodiments, the two or more biomarkers include MCP1, Interleukin-15, and C-Reactive Protein. In other embodiments, the two or more biomarkers are MCP1, Interleukin-15, C-Reactive Protein, and Matrix Metalloproteinase-3. In other embodiments, the two or more biomarkers are MCP1, Interleukin-15, C-Reactive Protein, Matrix Metalloproteinase-3, and Interleukin-1 receptor antagonist. In yet other embodiments, the two or more biomarkers are MCP1, Interleukin-15, C-Reactive Protein, Matrix Metalloproteinase-3, Interleukin-1 receptor antagonist, and von Willebrand Factor. In yet other embodiments, the two or more biomarkers are MCP1, Interleukin-15, C-Reactive Protein, Matrix Metalloproteinase-3, Interleukin-1 receptor antagonist, von Willebrand Factor, and Eotaxin-1. In yet other embodiments, the two or more biomarkers include MCP1, Interleukin-15, C-Reactive Protein, Matrix Metalloproteinase-3, Interleukin-1 receptor antagonist, von Willebrand Factor, Eotaxin-1, and Alpha-1-Antitrypsin. In yet other embodiments, the two or more biomarkers include MCP1, Interleukin-15, C-Reactive Protein, Matrix Metalloproteinase-3, Interleukin-1 receptor antagonist, von Willebrand Factor, Eotaxin-1, Alpha-1-Antitrypsin, and Macrophage Inflammatory Protein-1 beta. In yet other embodiments, the two or more biomarkers include MCP1, Interleukin-15, C-Reactive Protein, Matrix Metalloproteinase-3, Interleukin-1 receptor antagonist, von Willebrand Factor, Eotaxin-1, Alpha-1-Antitrypsin, Macrophage Inflammatory Protein-1 beta, and Vascular Cell Adhesion Molecule-1.

In certain embodiments, when two or more biomarkers are used, the levels of the two or more biomarkers can be correlated with a likelihood of the subject being responsive to treatment with apremilast by (a) generating a threshold composite score based upon the levels of two or more biomarker levels measured in a population having the disease or disorder; and (b) determining the likelihood of a subject being responsive to apremilast by comparing the threshold composite score to the composite score of the subject.

In more specific embodiments, the levels of the two or more biomarkers can be correlated with a likelihood of the subject being responsive to treatment with apremilast by (a) determining the expression profile of the biomarkers provided herein or a subset thereof in a biological sample from a population of subjects that previously have been administered with apremilast, wherein each subset of the biomarkers corresponds to biomarkers differentially expressed by the subjects; (b) generating a composite score for the biomarkers or the subset thereof for each subject of the population; (c) differentiating the subjects that are responsive to or are likely to be responsive to apremilast from those subjects that are not responsive to or are not likely to be responsive to apremilast; and (d) determining responsiveness to apremilast based on the composite scores for the subjects that are responsive to or are likely to be responsive to apremilast and those subjects that are not responsive to or are not likely to be responsive to apremilast.

In another more specific embodiments, the levels of the two or more biomarkers can be correlated with a likelihood of the subject being responsive to treatment with apremilast by (a) determining the expression profile of the

biomarkers provided herein or a subset thereof in a biological sample from a population of subjects that previously have been administered with apremilast, wherein each subset of the biomarkers corresponds to biomarkers differentially expressed by the subjects; (b) generating a composite score for the biomarkers or the subset thereof for each subject of the population; (c) differentiating the subjects that are responsive to or are likely to be responsive to apremilast from those subjects that are not responsive to or are not likely to be responsive to apremilast; (d) generating a threshold composite score that is predictive of the responsiveness of a subject to apremilast using a model based on the composite scores for the subjects that are responsive to or are likely to be responsive to apremilast and those subjects that are not responsive to are not likely to be responsive to apremilast, and (e) determining the likelihood of a subject being responsive to apremilast by comparing the threshold composite score to the composite score of the subject.

In some embodiments, a classifier can be used to correlate the levels of the two or more biomarkers with a likelihood of the subject being responsive to treatment with apremilast. Any classifier that classifies based on two or more features (*i.e.,* assigning a sample into a group based on two or more features of the sample) can be used in the present disclosure. In some embodiments, the classifier is a supervised classification algorithm. Unlimited examples of such classifiers include standard SVM, random forest, decision trees, rule based classifiers and discriminate analysis. Other classifiers, known to those skilled in the art, that can analyze the expression profile of multiple biomarkers from a population in order to "learn" a decision boundary between the patients likely to be responsive to a treatment compound and those patients not likely to be responsive to the treatment compound, are also included in the present disclosure.

In a specific embodiment, the classifier is Naive Bayes, and Naive Bayes is used to classify a patient based on two or more biomarker levels. The formula for Naive Bayes is:

$$\hat{y} = \underset{k \in \{1,\ldots,K\}}{\operatorname{argmax}} \; p(C_k) \prod_{i=1}^{n} p(x_i | C_k) ,$$

where p(Ck) represents overall class (MCP1 low cluster membership) probability, K is number of classes (here two), n is the number of features (number of biomarkers used (2 for Top 2, etc)), x are the features themselves and p(xi|Ck) is the posterior probability of observing $x_i$ given class $C_k$.

In another specific embodiment, the classifier is Weighted RSVM, and Weighted RSVM is used to classify a patient based on two or more biomarker levels. Weighted RSVM is weighted reduced SVM. Reduced version of SVM uses a subset of the data to improve efficiency and generalization of the model. Weighted version balances classes of different sizes. The formula of SVM is

$$f(\vec{x}) = \operatorname{sign}(\textstyle\sum \alpha_i y_i K(\vec{x}_i, \vec{x}) + b)$$

where $x_i$ is a sample represented by a vector of features and x is all the samples, K is a kernel matrix which encodes the distance between all samples, yi is the cluster membership for sample i, alpha is an indicator variable that describes which samples are closest to the classification boundary and b is an offset.

In some embodiments, the levels of the two or more biomarkers are correlated with a greater than 50%, 60%, 70%, 80% or 90% likelihood of the subject being responsive to apremilast. In some embodiments, the levels of the two or more biomarkers are correlated with a greater than 50% likelihood of the subject being responsive to apremilast. In one embodiment, the levels of the two or more biomarkers are correlated with a greater than 60% likelihood of the subject being responsive to apremilast. In another embodiment, the levels of the two or more biomarkers are correlated with a greater than 70% likelihood of the subject being responsive to apremilast. In some embodiments, the levels of the two or more biomarkers are correlated with a greater than 80% likelihood of the subject being responsive to apremilast. In one embodiment, the levels of the two or more biomarkers are correlated with a greater than 90% likelihood of the subject being responsive to apremilast.

In certain aspects of the disclosure, the disease or disorder is selected from the group consisting of psoriasis, psoriatic arthritis, rheumatoid arthritis, rheumatoid spondylitis, osteoarthritis, other arthritic conditions, non-radiographic axial spondyloarthropathy, Crohn's disease, inflammatory bowel disease, ulcerative colitis, celiac disease, atopic dermatitis, and multiple sclerosis. In some aspects of the disclosure, the disease or disorder is a chronic inflammatory disease. In the present invention, the inflammatory disease is ankylosing spondylitis.

In some embodiments, the patient is an adult. In one embodiment, the patient is a newborn. In some embodiments, the patient is an infant. In another embodiment, the patient is a child. In one embodiment, the patient is an adolescent.

In certain embodiments, about 5 mg to about 100 mg of apremilast is administered to the subject per day. In one embodiment, about 10 mg to about 60 mg of apremilast is administered to the subject per day. In some embodiments, apremilast is administered to the subject once or twice daily. In some embodiments, about 5 mg to about 50 mg of apremilast is administered to the subject for 6 or more days. In one embodiment, about 10 mg to about 100 mg of apremilast is administered to the subject 6 or more days. It should be understood that the specific amount and administration frequency and duration will depend on the age of the subject being treated, the severity and stage of disease and the amount(s) of any optional additional second active agents concurrently administered to the patient.

### 4.4 Methods of Measuring Biomarker Levels

Biomarker levels (*e.g.,* levels of cytokines) can be determined by measuring mRNA or protein levels of the biomarkers.

Several methods of detecting or quantitating mRNA levels are known in the art. Exemplary methods include, but are not limited to, northern blots, ribonuclease protection assays, and PCR-based methods. The mRNA sequence of a biomarker can be used to prepare a probe that is at least partially complementary to the mRNA sequence. The probe can then be used to detect the mRNA in a sample, using any suitable assay, such as PCR-based methods, northern blotting, or a dipstick assay.

In other embodiments, a nucleic acid assay can be prepared. An assay typically contains a solid support and at least one nucleic acid contacting the support, where the nucleic acid corresponds to at least a portion of an mRNA of a biomarker. The assay can also have a means for detecting the altered expression of the mRNA in the sample.

The assay method can be varied depending on the type of mRNA information desired. Exemplary methods include but are not limited to Northern blots and PCR-based methods (*e.g.,* qRT-PCR). Methods, such as qRT-PCR, can also accurately quantitate the amount of the mRNA in a sample.

Any suitable assay platform can be used to determine the presence and amount of mRNA in a sample. For example, an assay may be in the form of a dipstick, a membrane, a chip, a disk, a test strip, a filter, a microsphere, a slide, a multi-well plate, or an optical fiber. An assay system may have a solid support on which a nucleic acid corresponding to the mRNA is attached. The solid support may comprise, for example, a plastic, silicon, a metal, a resin, glass, a membrane, a particle, a precipitate, a gel, a polymer, a sheet, a sphere, a polysaccharide, a capillary, a film, a plate, or a slide. The assay components can be prepared and packaged together as a kit for detecting or measuring an mRNA.

The nucleic acid can be labeled, if desired, to make a population of labeled mRNAs. In general, a sample can be labeled using methods that are well known in the art (*e.g.,* using DNA ligase, terminal transferase, or by labeling the RNA backbone, *etc.*). *See, e.g.,* Ausubel et al., Short Protocols in Molecular Biology (Wiley & Sons, 3rd ed. 1995); Sambrook et al., Molecular Cloning: A Laboratory Manual (Cold Spring Harbor, N.Y., 3rd ed. 2001). In another embodiment, the sample is labeled with fluorescent label. Exemplary fluorescent dyes include, but are not limited to, xanthene dyes, fluorescein dyes (*e.g.,* fluorescein isothiocyanate (FITC), 6-carboxyfluorescein (FAM), 6 carboxy-2',4',7',4,7-hexachlorofluorescein (HEX), 6-carboxy-4',5'-dichloro-2',7'-dimethoxyfluorescein (JOE)), rhodamine dyes (*e.g.,* rhodamine 110 (R110), N,N,N',N'-tetramethyl-6-carboxyrhodamine (TAMRA), 6-carboxy-X-rhodamine (ROX), 5-carboxyrhodamine 6G (R6G5 or G5), 6-carboxyrhodamine 6G (R6G6 or G6)), cyanine dyes (*e.g.,* Cy3, Cy5 and Cy7), Alexa dyes (*e.g.,* Alexa-fluor-555), coumarin, Diethylaminocoumarin, umbelliferone, benzimide dyes (*e.g.,* Hoechst 33258) phenanthridine dyes (*e.g.,* Texas Red), ethidium dyes, acridine dyes, carbazole dyes, phenoxazine dyes, porphyrin dyes, polymethine dyes, BODIPY dyes, quinoline dyes, Pyrene, Fluorescein Chlorotriazinyl, eosin dyes, Tetramethylrhodamine, Lissamine, and Napthofluorescein.

In one aspect of the disclosure, the mRNA sequences comprise at least one mRNA of a biomarker provided herein. In some aspects of the disclosure, the biomarker is selected from the group consisting of the mRNA of Alpha-1-Antitrypsin, Alpha-2-Macroglobulin, Beta-2-Microglobulin, Brain-Derived Neurotrophic Factor, C-Reactive Protein, Complement C3, Eotaxin-1, Factor VII, Ferritin, Fibrinogen, Granulocyte-Macrophage Colony-Stimulating Factor, Haptoglobin, Intercellular Adhesion Molecule 1, Interferon gamma, Interleukin-1 alpha, Interleukin-1 beta, Interleukin-1 receptor antagonist, Interleukin-10, Interleukin-12 Subunit p40, Interleukin-12 Subunit p70, Interleukin-15, Interleukin-17, Interleukin-2, Interleukin-23, Interleukin-3, Interleukin-4, Interleukin-5, Interleukin-6, Interleukin-7, Interleukin-8, Macrophage Inflammatory Protein-1 alpha, Macrophage Inflammatory Protein-1 beta, Matrix Metalloproteinase-3, Matrix Metalloproteinase-9, MCP1, Stem Cell Factor, T-Cell-Specific Protein RANTES, Tissue Inhibitor of Metalloproteinases, Tumor Necrosis Factor alpha, Tumor Necrosis Factor beta, Tumor necrosis factor receptor 2, Vascular Cell Adhesion Molecule-1, Vascular Endothelial Growth Factor, Vitamin D-Binding Protein, von Willebrand Factor, Osteoprotegerin, Interleukin-18, RANKL, and Sclerostin.

In a specific embodiment, the biomarker is the mRNA of MCP1. In a specific aspect of the disclosure, the biomarker is the mRNA of Interleukin-15. In a specific aspect of the disclosure, the biomarker is the mRNA of C-Reactive Protein. In a specific aspect of the disclosure, the biomarker is the mRNA of Matrix Metalloproteinase-3. In a specific

aspect of the disclosure, the biomarker is the mRNA of Interleukin-1 receptor antagonist. In a specific aspect of the disclosure, the biomarker is the mRNA of von Willebrand Factor. In a specific aspect of the disclosure, the biomarker is the mRNA of Eotaxin-1. In a specific aspect of the disclosure, the biomarker is the mRNA of Alpha-1-Antitrypsin. In a specific aspect of the disclosure, the biomarker is the mRNA of Macrophage Inflammatory Protein-1 beta. In a specific aspect of the disclosure, the biomarker is the mRNA of Vascular Cell Adhesion Molecule-1. In a specific aspect of the disclosure, the biomarker is the mRNA of Tissue Inhibitor of Metalloproteinases 1. In a specific aspect of the disclosure, the biomarker is the mRNA of Osteoprotegerin. In a specific aspect of the disclosure, the biomarker is the mRNA of Vascular Endothelial Growth Factor. In a specific aspect of the disclosure, the biomarker is the mRNA of Alpha-2-Macroglobulin. In a specific aspect of the disclosure, the biomarker is the mRNA of Brain-Derived Neurotrophic Factor. In a specific aspect of the disclosure, the biomarker is the mRNA of Intercellular Adhesion Molecule 1. In a specific aspect of the disclosure, the biomarker is the mRNA of Interleukin-18. In a specific aspect of the disclosure, the biomarker is the mRNA of Matrix Metalloproteinase-9. In a specific aspect of the disclosure, the biomarker is the mRNA of Vitamin D-Binding Protein.

The nucleic acids may be present in specific, addressable locations on a solid support, each corresponding to at least a portion of mRNA sequence of a biomarker in a cell or a patient.

A typical mRNA assay method can contain the steps of 1) obtaining surface-bound subject probes; 2) hybridizing a population of mRNAs to the surface-bound probes under conditions sufficient to provide for specific binding; (3) post-hybridization washing to remove nucleic acids not specifically bound to the surface-bound probes; and (4) detecting the hybridized mRNAs. The reagents used in each of these steps and their conditions for use may vary depending on the particular application.

Hybridization can be carried out under suitable hybridization conditions, which may vary in stringency as desired. Typical conditions are sufficient to produce probe/target complexes on a solid surface between complementary binding members, *i.e.,* between surface-bound subject probes and complementary mRNAs in a sample. In certain embodiments, stringent hybridization conditions may be employed.

Hybridization is typically performed under stringent hybridization conditions. Standard hybridization techniques (*e.g.,* under conditions sufficient to provide for specific binding of target mRNAs in the sample to the probes) are described in Kallioniemi et al., Science 258:818-821 (1992) and International Patent Application Publication No. WO 93/18186. Several guides to general techniques are available, *e.g.,* Tijssen, Hybridization with Nucleic Acid Probes, Parts I and II (Elsevier, Amsterdam 1993). For descriptions of techniques suitable for *in situ* hybridizations, see Gall et al., Meth. Enzymol. 21:470-480 (1981); Angerer et al., Genetic Engineering: Principles and Methods, Vol 7, pgs 43-65 (Plenum Press, New York, Setlow and Hollaender, eds. 1985). Selection of appropriate conditions, including temperature, salt concentration, polynucleotide concentration, hybridization time, and stringency of washing conditions, will depend on experimental design, including source of sample, identity of capture agents, degree of complementarity expected, *etc.,* and may be determined as a matter of routine experimentation for those of ordinary skill in the art.

Those of ordinary skill will readily recognize that alternative but comparable hybridization and wash conditions can be utilized to provide conditions of similar stringency.

After the mRNA hybridization procedure, the surface bound polynucleotides are typically washed to remove unbound nucleic acids. Washing may be performed using any convenient washing protocol, where the washing conditions are typically stringent, as described above. The hybridization of the target mRNAs to the probes is then detected using standard techniques.

Other methods, such as PCR-based methods, can also be used to detect or measure the expression of a biomarker provided herein. Examples of PCR methods can be found in U.S. Patent No. 6,927,024. Examples of RT-PCR methods can be found in U.S. Patent No. 7,122,799. A method of fluorescent *in situ* PCR is described in U.S. Patent No. 7,186,507.

In some embodiments, quantitative Reverse Transcription-PCR (qRT-PCR) can be used for both the detection and quantification of RNA targets (Bustin et al., Clin. Sci. 109:365-379 (2005)). Quantitative results obtained by qRT-PCR are generally more informative than qualitative data. Thus, in one embodiment, qRT-PCR-based assays can be useful to measure mRNA levels during cell-based assays. The qRT-PCR method is also useful to monitor patient therapy. Examples of qRT-PCR-based methods can be found, for example, in U.S. Patent No. 7,101,663.

In contrast to regular reverse transcriptase-PCR and analysis by agarose gels, qRT-PCR gives quantitative results. An additional advantage of qRT-PCR is the relative ease and convenience of use. Instruments for qRT-PCR, such as the Applied Biosystems 7500, are available commercially, so are the reagents, such as TaqMan® Sequence Detection Chemistry. For example, TaqMan® Gene Expression Assays can be used, following the manufacturer's instructions. These kits are pre-formulated gene expression assays for rapid, reliable detection and quantification of human, mouse, and rat mRNA transcripts. An exemplary qRT-PCR program, for example, is 50 °C for 2 minutes, 95 °C for 10 minutes, 40 cycles of 95 °C for 15 seconds, then 60 °C for 1 minute.

To determine the cycle number at which the fluorescence signal associated with a particular amplicon accumulation crosses the threshold (referred to as the $C_T$), the data can be analyzed, for example, using a 7500 Real-Time

PCR System Sequence Detection software v1.3 using the comparative $C_T$ relative quantification calculation method. Using this method, the output is expressed as a fold-change of expression levels. In another embodiment, the threshold level can be selected to be automatically determined by the software. In some embodiments, the threshold level is set to be above the baseline but sufficiently low to be within the exponential growth region of an amplification curve.

Several protein detection and quantitation methods can be used to measure the level of a biomarker. Any suitable protein quantitation method can be used. In one embodiment, antibody-based methods are used. Exemplary methods that can be used include, but are not limited to, immunoblotting (Western blot), ELISA, immunohistochemistry, flow cytometry, cytometric bead array, and mass spectroscopy. Several types of ELISA are commonly used, including direct ELISA, indirect ELISA, and sandwich ELISA.

In some aspects of the disclosure, the biomarker is selected from the group consisting of the protein of Alpha-1-Antitrypsin, Alpha-2-Macroglobulin, Beta-2-Microglobulin, Brain-Derived Neurotrophic Factor, C-Reactive Protein, Complement C3, Eotaxin-1, Factor VII, Ferritin, Fibrinogen, Granulocyte-Macrophage Colony-Stimulating Factor, Haptoglobin, Intercellular Adhesion Molecule 1, Interferon gamma, Interleukin-1 alpha, Interleukin-1 beta, Interleukin-1 receptor antagonist, Interleukin-10, Interleukin-12 Subunit p40, Interleukin-12 Subunit p70, Interleukin-15, Interleukin-17, Interleukin-2, Interleukin-23, Interleukin-3, Interleukin-4, Interleukin-5, Interleukin-6, Interleukin-7, Interleukin-8, Macrophage Inflammatory Protein-1 alpha, Macrophage Inflammatory Protein-1 beta, Matrix Metalloproteinase-3, Matrix Metalloproteinase-9, MCP1, Stem Cell Factor, T-Cell-Specific Protein RANTES, Tissue Inhibitor of Metalloproteinases, Tumor Necrosis Factor alpha, Tumor Necrosis Factor beta, Tumor necrosis factor receptor 2, Vascular Cell Adhesion Molecule-1, Vascular Endothelial Growth Factor, Vitamin D-Binding Protein, von Willebrand Factor, Osteoprotegerin, Interleukin-18, RANKL, and Sclerostin.

In a specific embodiment, the biomarker is the protein of MCP1. In a specific aspect of the disclosure, the biomarker is the protein of Interleukin-15. In a specific aspect of the disclosure, the biomarker is the protein of C-Reactive Protein. In a specific aspect of the disclosure, the biomarker is the protein of Matrix Metalloproteinase-3. In a specific aspect of the disclosure, the biomarker is the protein of Interleukin-1 receptor antagonist. In a specific aspect of the disclosure, the biomarker is the protein of von Willebrand Factor. In a specific aspect of the disclosure, the biomarker is the protein of Eotaxin-1. In a specific aspect of the disclosure, the biomarker is the protein of Alpha-1-Antitrypsin. In a specific aspect of the disclosure, the biomarker is the protein of Macrophage Inflammatory Protein-1 beta. In a specific aspect of the disclosure, the biomarker is the protein of Vascular Cell Adhesion Molecule-1. In a specific aspect of the disclosure, the biomarker is the protein of Tissue Inhibitor of Metalloproteinases 1. In a specific aspect of the disclosure, the biomarker is the protein of Osteoprotegerin. In a specific aspect of the disclosure, the biomarker is the protein of Vascular Endothelial Growth Factor. In a specific aspect of the disclosure, the biomarker is the protein of Alpha-2-Macroglobulin. In a specific aspect of the disclosure, the biomarker is the protein of Brain-Derived Neurotrophic Factor. In a specific aspect of the disclosure, the biomarker is the protein of Intercellular Adhesion Molecule 1. In a specific aspect of the disclosure, the biomarker is the protein of Interleukin-18. In a specific aspect of the disclosure, the biomarker is the protein of Matrix Metalloproteinase-9. In a specific aspect of the disclosure, the biomarker is the protein of Vitamin D-Binding Protein.

### 4.5 Kits for Detecting or Measuring Biomarker Levels

In certain aspects of the disclosure, provided herein is a kit for detecting or measuring the mRNA level of one or more biomarkers. In certain aspects of the disclosure, the kit comprises one or more probes that bind specifically to the mRNAs of the one or more biomarkers. In certain aspects of the disclosure, the kit further comprises a washing solution. In certain aspects of the disclosure, the kit further comprises reagents for performing a hybridization assay, mRNA isolation or purification means, detection means, as well as positive and negative controls. In certain aspects of the disclosure, the kit further comprises an instruction for using the kit. The kit can be tailored for in-home use, clinical use, or research use.

In certain aspects of the disclosure, provided herein is a kit for detecting the protein level of one or more biomarkers. In certain aspects of the disclosure, the kits comprises a dipstick coated with an antibody that recognizes the protein biomarker, washing solutions, reagents for performing the assay, protein isolation or purification means, detection means, as well as positive and negative controls. In certain aspects of the disclosure, the kit further comprises an instruction for using the kit. The kit can be tailored for in-home use, clinical use, or research use.

Such a kit can employ, for example, a dipstick, a membrane, a chip, a disk, a test strip, a filter, a microsphere, a slide, a multi-well plate, or an optical fiber. The solid support of the kit can be, for example, a plastic, silicon, a metal, a resin, glass, a membrane, a particle, a precipitate, a gel, a polymer, a sheet, a sphere, a polysaccharide, a capillary, a film, a plate, or a slide. The biological sample can be, for example, a cell culture, a cell line, a tissue, an organ, an organelle, a biological fluid, a blood sample, a urine sample, or a skin sample.

In another aspect of the disclosure, the kit comprises a solid support, nucleic acids attached to the support, where the nucleic acids are complementary to at least 20, 50, 100, 200, 350, or more bases of mRNA, and a means for

detecting the expression of the mRNA in a biological sample.

In a specific aspect of the disclosure, the pharmaceutical or assay kit comprises, in a container, a compound or a pharmaceutical composition thereof, and further comprises, in one or more containers, components for isolating RNA. In another specific aspect of the disclosure, the pharmaceutical or assay kit comprises, in a container, a compound or a pharmaceutical composition, and further comprises, in one or more containers, components for conducting RT-PCR, qRT-PCR, deep sequencing, or microarray

In certain aspects of the disclosure, the kits provided herein employ means for detecting or measuring the expression of a biomarker by quantitative real-time PCR (qRT-PCR), microarray, flow cytometry, or immunofluorescence. In other aspects of the disclosure, the expression of the biomarker is measured by ELISA-based methodologies or other similar methods known in the art.

In another specific aspect of the disclosure, the pharmaceutical or assay kit comprises, in a container, a compound or a pharmaceutical composition thereof, and further comprises, in one or more containers, components for isolating protein. In another specific aspect of the disclosure, the pharmaceutical or assay kit comprises, in a container, a compound or a pharmaceutical composition, and further comprises, in one or more containers, components for conducting flow cytometry or ELISA.

In another aspect, provided herein are kits for measuring biomarkers that supply the materials necessary to measure the abundance of one or more gene products of the biomarkers or a subset of the biomarkers (*e.g.,* one, two, three, four, five, or more biomarkers) provided herein. Such kits may comprise materials and reagents required for measuring RNA or protein. In another aspect of the disclosure, such kits include microarrays, wherein the microarray is comprised of oligonucleotides and/or DNA and/or RNA fragments which hybridize to one or more gene products of the biomarkers or a subset of the biomarkers provided herein, or any combination thereof. In one aspect of the disclosure, such kits may include primers for PCR of either the RNA product or the cDNA copy of the RNA product of the biomarkers or a subset of the biomarkers, or both. In some aspects of the disclosure, such kits may include primers for PCR as well as probes for qPCR. In some aspects of the disclosure, such kits may include multiple primers and multiple probes, wherein some of the probes have different fluorophores so as to permit simultaneously measuring multiple gene products of the biomarkers or a subset of the biomarkers provided herein. In one aspect of the disclosure, such kits may further include materials and reagents for creating cDNA from RNA. In another aspect of the disclosure, such kits may include antibodies specific for the protein products of the biomarkers or a subset of the biomarkers provided herein. Such kits may additionally comprise materials and reagents for isolating RNA and/or proteins from a biological sample. In addition, such kits may include materials and reagents for synthesizing cDNA from RNA isolated from a biological sample. In some aspects of the disclosure, such kits may include a computer program product embedded on computer readable media for predicting whether a patient is clinically sensitive to apremilast. In one aspect of the disclosure, the kits may include a computer program product embedded on a computer readable media along with instructions.

In some aspects of the disclosure, such kits measure the expression of one or more nucleic acid products of the biomarkers or a subset of the biomarkers provided herein. In accordance with this aspect of the disclosure, the kits may comprise materials and reagents that are necessary for measuring the expression of particular nucleic acid products of the biomarkers or a subset of the biomarkers provided herein. For example, a microarray or RT-PCR kit may be produced for a specific condition and contain only those reagents and materials necessary for measuring the levels of specific RNA transcript products of the biomarkers or a subset of the biomarkers provided herein, to predict whether an inflammatory disease in a patient is clinically sensitive to apremilast. Alternatively, in another aspect of the disclosure, the kits can comprise materials and reagents necessary for measuring the expression of particular nucleic acid products of genes other than the biomarkers provided herein. For example, in certain aspects of the disclosure, the kits comprise materials and reagents necessary for measuring the expression levels of 1, 2, 3, 4, 5, 6, 7, 8, 9, 10, 15, 20, 25, 30, 35, 40, 45, 48 or more of the genes of the biomarkers provided herein, in addition to reagents and materials necessary for measuring the expression levels of at least 1, at least 2, at least 3, at least 4, at least 5, at least 6, at least 7, at least 8, at least 9, at least 10, at least 15, at least 20, at least 25, at least 30, at least 35, at least 40, at least 45, at least 48, or more genes other than the biomarkers provided herein. In other aspects of the disclosure, the kits contain reagents and materials necessary for measuring the expression levels of at least 1, at least 2, at least 3, at least 4, at least 5, at least 6, at least 7, at least 8, at least 9, at least 10, at least 15, at least 20, at least 25, at least 30, at least 35, at least 40, at least 45, at least 48, or more of the biomarkers provided herein, and 1, 2, 3, 4, 5, 10, 15, 20, 25, 30, 35, 40, 45, 50, 55, 60, 65, 70, 75, 80, 85, 90, 95, 100, 125, 150, 175, 200, 225, 250, 300, 350, 400, 450, or more genes that are not the biomarkers provided herein. In certain aspects of the disclosure, the kits contain reagents and materials necessary for measuring the expression levels of at least 1, at least 2, at least 3, at least 4, at least 5, at least 6, at least 7, at least 8, at least 9, at least 10, at least 15, at least 20, at least 25, at least 30, at least 35, at least 40, at least 45, at least 48, or more of the genes of the biomarkers provided herein, and 1-10, 1-100, 1-150, 1-200, 1-300, 1-400, 1-500, 1-1000, 25-100, 25-200, 25-300, 25-400, 25-500, 25-1000, 100-150, 100-200, 100-300, 100-400, 100-500, 100-1000 or 500-1000 genes that are not the biomarkers provided herein.

For nucleic acid microarray kits, the kits generally comprise probes attached to a solid support surface. In one

such aspect of the disclosure, probes can be either oligonucleotides or longer probes including probes ranging from 150 nucleotides to 800 nucleotides in length. The probes may be labeled with a detectable label. In a specific aspect of the disclosure, the probes are specific for one or more of the gene products of the biomarkers provided herein. The microarray kits may comprise instructions for performing the assay and methods for interpreting and analyzing the data resulting from performing the assay. In a specific aspect of the disclosure, the kits comprise instructions for predicting whether an inflammatory disease in a patient is clinically sensitive to apremilast. The kits may also comprise hybridization reagents and/or reagents necessary for detecting a signal produced when a probe hybridizes to a target nucleic acid sequence. Generally, the materials and reagents for the microarray kits are in one or more containers. Each component of the kit is generally in its own suitable container.

In certain aspects of the disclosure, a nucleic acid microarray kit comprises materials and reagents necessary for measuring the expression levels of 1, 2, 3, 4, 5, 6, 7, 8, 9, 10, 15, 20, 25, 30, 35, 40, 45, 48, or more of the genes of the biomarkers provided herein, or a combination thereof, in addition to reagents and materials necessary for measuring the expression levels of at least 1, at least 2, at least 3, at least 4, at least 5, at least 6, at least 7, at least 8, at least 9, at least 10, at least 15, at least 20, at least 25, at least 30, at least 35, at least 40, at least 45, at least 50, or more genes other than those of the biomarkers provided herein. In other aspects of the disclosure, a nucleic acid microarray kit contains reagents and materials necessary for measuring the expression levels of at least 1, at least 2, at least 3, at least 4, at least 5, at least 6, at least 7, at least 8, at least 9, at least 10, at least 15, at least 20, at least 25, at least 30, at least 35, at least 40, at least 45, at least 48, or more of the genes of the biomarkers provided herein, or any combination thereof, and 1, 2, 3, 4, 5, 10, 15, 20, 25, 30, 35, 40, 45, 50, 55, 60, 65, 70, 75, 80, 85, 90, 95, 100, 125, 150, 175, 200, 225, 250, 300, 350, 400, 450, or more genes that are not of the biomarkers provided herein. In another aspect of the disclosure, a nucleic acid microarray kit contains reagents and materials necessary for measuring the expression levels of at least 1, at least 2, at least 3, at least 4, at least 5, at least 6, at least 7, at least 8, at least 9, at least 10, at least 15, at least 20, at least 25, at least 30, at least 35, at least 40, at least 45, at least 48, or more of the genes of the biomarkers provided herein, or any combination thereof, and 1-10, 1-100, 1-150, 1-200, 1-300, 1-400, 1-500, 1-1000, 25-100, 25-200, 25-300, 25-400, 25-500, 25-1000, 100-150, 100-200, 100-300, 100-400, 100-500, 100-1000, or 500-1000 genes that are not of the biomarkers provided herein.

For quantitative PCR, the kits generally comprise pre-selected primers specific for particular nucleic acid sequences. The quantitative PCR kits may also comprise enzymes suitable for amplifying nucleic acids (e.g., polymerases, such as Taq polymerase), deoxynucleotides, and buffers needed for amplification reaction. The quantitative PCR kits may also comprise probes specific for the nucleic acid sequences associated with or indicative of a condition. The probes may or may not be labeled with a fluorophore. The probes may or may not be labeled with a quencher molecule. In one aspect of the disclosure, the quantitative PCR kits also comprise components suitable for reverse-transcribing RNA, including enzymes (e.g., reverse transcriptases, such as AMV, and MMLV) and primers for reverse transcription along with deoxynucleotides and buffers needed for reverse transcription reaction. Each component of the quantitative PCR kit is generally in its own suitable container. Thus, these kits generally comprise distinct containers suitable for each individual reagent, enzyme, primer and probe. Further, the quantitative PCR kits may comprise instructions for performing the reaction and methods for interpreting and analyzing the data resulting from performing the reaction. In a specific aspect of the disclosure, the kits contain instructions for predicting whether an inflammatory disease in a patient is clinically sensitive to apremilast.

For antibody-based kits, the kit can comprise, for example: (1) a first antibody (which may or may not be attached to a solid support) that binds to a peptide, polypeptide or protein of interest; and, optionally, (2) a second, different antibody that binds to either the first antibody or the peptide, polypeptide, or protein, and is conjugated to a detectable label (*e.g.,* a fluorescent label, radioactive isotope, or enzyme). In a specific aspect of the disclosure, the peptide, polypeptide, or protein of interest is associated with or indicative of a condition (*e.g.,* a disease). The antibody-based kits may also comprise beads for conducting immunoprecipitation. Each component of the antibody-based kits is generally in its own suitable container. Thus, these kits generally comprise distinct containers suitable for each antibody and reagent. Further, the antibody-based kits may comprise instructions for performing the assay and methods for interpreting and analyzing the data resulting from performing the assay. In a specific aspect of the disclosure, the kits contain instructions for predicting whether an inflammatory disease in a patient is clinically sensitive to apremilast.

In some aspects of the disclosure, the kits provided herein comprise materials for measuring the expression levels of one or more biomarkers selected from the group consisting of Alpha-1-Antitrypsin, Alpha-2-Macroglobulin, Beta-2-Microglobulin, Brain-Derived Neurotrophic Factor, C-Reactive Protein, Complement C3, Eotaxin-1, Factor VII, Ferritin, Fibrinogen, Granulocyte-Macrophage Colony-Stimulating Factor, Haptoglobin, Intercellular Adhesion Molecule 1, Interferon gamma, Interleukin-1 alpha, Interleukin-1 beta, Interleukin-1 receptor antagonist, Interleukin-10, Interleukin-12 Subunit p40, Interleukin-12 Subunit p70, Interleukin-15, Interleukin-17, Interleukin-2, Interleukin-23, Interleukin-3, Interleukin-4, Interleukin-5, Interleukin-6, Interleukin-7, Interleukin-8, Macrophage Inflammatory Protein-1 alpha, Macrophage Inflammatory Protein-1 beta, Matrix Metalloproteinase-3, Matrix Metalloproteinase-9, MCP1, Stem Cell Factor, T-Cell-Specific Protein RANTES, Tissue Inhibitor of Metalloproteinases, Tumor Necrosis Factor alpha, Tumor Necrosis

Factor beta, Tumor necrosis factor receptor 2, Vascular Cell Adhesion Molecule-1, Vascular Endothelial Growth Factor, Vitamin D-Binding Protein, von Willebrand Factor, Osteoprotegerin, Interleukin-18, RANKL, and Sclerostin.

In certain aspects of the disclosure of the methods and kits provided herein, solid phase supports are used for purifying proteins, labeling samples, or carrying out the solid phase assays. Examples of solid phases suitable for carrying out the methods disclosed herein include beads, particles, colloids, single surfaces, tubes, multi-well plates, microtiter plates, slides, membranes, gels, and electrodes. When the solid phase is a particulate material (*e.g.,* a bead), it is, in one aspect of the disclosure, distributed in the wells of multi-well plates to allow for parallel processing of the solid phase supports.

It is noted that any combination of the above-listed aspects of the disclosure, for example, with respect to one or more reagents, such as, without limitation, nucleic acid primers, and solid support, are also contemplated in relation to any of the various methods and/or kits provided herein.

Certain embodiments of the invention are illustrated by the following non-limiting examples.

## 5. EXAMPLES

Some embodiments are illustrated by the following non-limiting examples. The examples should not be construed as a limitation in the scope thereof.

### 5.1 Example 1-Unsupervised Clustering Based on Biomarker Expression Profiles Discovered a Patient Group More Likely to be Responsive to Apremilast

In this example, a group of ankylosing spondylitis patients was identified based on expression profile of 49 biomarkers as being more likely to be responsive to a treatment with apremilast. In particular, Myriad RBM inflammation MAP biomarker panel (having 49 biomarkers) was used to profile 280 patient samples in the study, among which 255 patient samples produced measurable biomarker levels. In these 255 patient samples, 95 patient samples were treated with 20 mg apremilast ("Apremilast 20 mg Arm Group"). Within the Apremilast 20 mg Arm Group, 36 patient samples were responsive to the treatment and 59 patient samples were not responsive to the treatment. 80 patient samples were treated with 30 mg apremilast ("Apremilast 30 mg Arm Group"). Within Apremilast 30 mg Arm Group, 31 patient samples were responsive to the treatment and 49 patient samples were not responsive to the treatment. The remaining 80 patient samples were control samples (placebo arm).

Unsupervised clustering was then used to analyze the biomarker expression profiles for these patient samples based on the 49 biomarkers (consensus clustering/distance (1-PCor)/Ward linkage method). These 49 biomarkers were Alpha-1-Antitrypsin, Alpha-2-Macroglobulin, Beta-2-Microglobulin, Brain-Derived Neurotrophic Factor, C-Reactive Protein, Complement C3, Eotaxin-1, Factor VII, Ferritin, Fibrinogen, Granulocyte-Macrophage Colony-Stimulating Factor, Haptoglobin, Intercellular Adhesion Molecule 1, Interferon gamma, Interleukin-1 alpha, Interleukin-1 beta, Interleukin-1 receptor antagonist, Interleukin-10, Interleukin-12 Subunit p40, Interleukin-12 Subunit p70, Interleukin-15, Interleukin-17, Interleukin-2, Interleukin-23, Interleukin-3, Interleukin-4, Interleukin-5, Interleukin-6, Interleukin-7, Interleukin-8, Macrophage Inflammatory Protein-1 alpha, Macrophage Inflammatory Protein-1 beta, Matrix Metalloproteinase-3, Matrix Metalloproteinase-9, MCP1, Stem Cell Factor, T-Cell-Specific Protein RANTES, Tissue Inhibitor of Metalloproteinases, Tumor Necrosis Factor alpha, Tumor Necrosis Factor beta, Tumor necrosis factor receptor 2, Vascular Cell Adhesion Molecule-1, Vascular Endothelial Growth Factor, Vitamin D-Binding Protein, von Willebrand Factor, Osteoprotegerin, Interleukin-18, RANKL, and Sclerostin.

The results are shown in the plot of **FIG. 1A.** As shown, a group of 50 patients (cluster 4) were identified as representing about 30% of the total treatment arm population. For reasons explained in Section 5.2 below, this group of patients is referred to herein as the "MCP1 low patient group." As indicated in **FIG. 1B**, this cluster of 50 ankylosing spondylitis patients is significantly associated with responsiveness to apremilast. In particular, within this group of 50 patients, the response rate was about 54%, higher than the response rate in the remaining patients which was about 31%.

This group of 50 patients was comprised of 25 patients who received 30 mg apremilast and 25 patients who received 20 mg of apremilast. Looking at the individual treatment arms, the response rate was even more pronounced in the patients receiving 30 mg apremilast. More specifically, 16 out of 25 of the patients receiving 30 mg apremilast treatment responded, which produced a response rate of 64%. This compares to the response rate of the remaining patients in the Apremilast 30 mg Arm Group, which was 27%.

### 5.2 Example 2-Using Expression Level of a Single Biomarker, such as MCP1, to Differentiate a Patient Group Having Higher Response Rate from the Remaining Patients

Univariate comparison of the expression level of each of the 49 biomarkers was performed between cluster 4 patient group and the remaining patients identified and described in Section 5.1 above. Only treatment arm populations

were used. The placebo group was excluded. The representative results are shown in **Table 1** below.

**Table 1. Comparison of Individual Biomarker between Cluster 4 and the Rest Patients**

| Biomarker | logFC | AveExpr | adj.P.Val |
|---|---|---|---|
| MCP1 | -2.26 | -0.13 | 0.00 |
| von Willebrand Factor | 0.82 | 0.07 | 0.00 |
| Interleukin-8 | -1.13 | 0.05 | 0.00 |
| C-Reactive Protein | 0.67 | -0.19 | 0.00 |
| Alpha-1-Antitrypsin | 0.66 | -0.01 | 0.00 |
| Macrophage Inflammatory Protein-1 beta | -0.76 | 0.09 | 0.00 |
| Interleukin-1 receptor antagonist | 0.53 | -0.19 | 0.00 |
| Eotaxin-1 | -0.36 | -0.09 | 0.00 |
| Interleukin-15 | 0.48 | -0.36 | 0.01 |
| Fibrinogen | 0.96 | -0.12 | 0.01 |
| Vascular Cell Adhesion Molecule-1 | -0.51 | 0.11 | 0.01 |
| Matrix Metalloproteinase-3 | 0.51 | 0.04 | 0.02 |
| Interleukin- 18 | -0.41 | 0.01 | 0.04 |
| Sclerostin | -0.43 | -0.02 | 0.04 |
| Complement C3 | 0.38 | -0.14 | 0.04 |
| Haptoglobin | 0.56 | -0.20 | 0.05 |
| Tissue Inhibitor of Metalloproteinases | -0.64 | 0.21 | 0.05 |
| Osteoprotegerin | -0.40 | 0.18 | 0.09 |

In **Table 1,** a logFC value below 0 indicates a lower expression level of the biomarker in the cluster 4 patient group as compared with the rest patients, and a logFC value above 0 indicates a higher expression level of the biomarker in the cluster 4 patient group as compared with the rest patients.

Each biomarker was then further analyzed for its association with the cluster 4 patient group. MCP1 cytokine was identified as being able to most strongly differentiate the cluster 4 group with the remaining patients. As such, cluster 4 patient group is herein referred to as the "MCP1 low group" or "MCP1 low cluster." As shown in **FIG. 2A,** the patients that represent MCP1 low group had significantly lower expression level of MCP1 as compared with the rest of the treated patients. The threshold derived from the mid-point (or average) of the median of MCP1 low cluster and the median of non MCP1 low patients was 93.5 pg/ml *(see* the dash line in the right panel of **FIG. 2A**).

As shown in **FIG. 2B,** the level of MCP1 was used to differentiate a small patient subgroup (14% of total population) highly responsive to apremilast (in apremilast arm with either 20 mg or 30 mg apremilast) with a response rate of about 62.5%. Similarly, as shown in **FIG. 2C,** the level of a single biomarker, MCP1, differentiated a patient subgroup (20% of apremilast 30 mg arm) with a response rate to 30 mg apremilast treatment of about 66.6%. As such, the level of a single biomarker, MCP1, can be used for predicting the patient’s response to apremilast treatment.

The performance of using MCP1 expression level alone as a biomarker to predict if a patient belongs to cluster 4 (MCP1 low cluster) was evaluated by cross validation. The data from the samples was split into 10 subsets (10-folds), where each fold had equal size and had a similar proportion of MCP1 low to non-MCPl low samples. One fold (10% of the data) was held out as a test set, and a classifier was learned from the remainder of the data (the other 90%, also called the training set). The learned model was then applied to the held-out test data to generate predictions of VCP1 low cluster membership for each test sample, which was then compared to the actual labels of cluster membership. This generated an unbiased estimate of how well the classifier would identify members of the MCP1 low cluster in unseen and future data. The process was performed 10 times, holding out a different test fold each time, and training on the remaining 9-folds. Final performance statistics were calculated by aggregating the predictions over all the test folds, resulting in unbiased cluster membership predictions for the entire sample set (all patients).

Furthermore, within each training set in the process above (90% of the data), another round of cross validation was performed, splitting the training set into 10 folds, holding out 1 fold (9% of the data), and training on the remaining

9-folds (81% of the data). The model was trained and tested over all the internal folds using a range of possible model parameters. The optimal parameters were selected as the ones which yielded the best classification performance (as determined by the AUC metrics) over the internal cross validation test folds. Finally, back in the outer loop of cross validation, the model was trained on the whole training set (90% of the data) using only the optimal parameters found in the internal loop. The model was applied to the outer test fold (10% of the data), which was never used in the parameter-setting or model-building process. This process is called nested cross validation. The performance of using MCP1 as a biomarker is shown in **Table 2** below.

**Table 2. Performance of MCP1 as a Biomarker**

| NB | acc | auc | mcc | sens | spec | ppv | npv |
|---|---|---|---|---|---|---|---|
| **MCP1** | 0.80 | 0.83 | 0.48 | 0.40 | 0.97 | 0.83 | 0.80 |

In **Table 2,** ACC (accuracy) indicates the proportion of predictions that the classifier gets correct. AUC indicates the area under the curve, given a classification score for each sample. AUC is the probability that a randomly picked positive sample (a sample from MCP1 low cluster) will have a higher score than a randomly picked negative sample (a sample not from MCP1 low cluster), *i.e.,* when compared to known response of the patients whose data points were withheld from model building and subsequently predicted in order to estimate classifier performance (see earlier descriptions of cross-validation). MCC (matthews correlation coefficient) is a correlation coefficient between the observed and predicted classifications. SENS indicates sensitivity or true positive rate, which means the proportion that is correctly classified in all MCP1 low samples. SPEC indicates specificity or true negative rate, which means the proportion that is correctly classified in all non-MCP1 low samples. PPV indicates positive predictive value, which means the proportion that is correctly classified out of all people predicted to be in MCP1 low group. NPV indicates negative predictive value, which means the proportion that is correctly classified out of all people predicted to be not in MCP1 low group.

### 5.2.1 Determination of a Threshold Level of MCP1

A threshold level of MCP1 (or another biomarker) can be calculated using various methods. Three methods for calculating a threshold level of MCP1 were exemplified in this study. **FIG. 2D** shows MCP1 distribution using a threshold level (103.97 pg/ml) calculated based on the median value of the total population in this study.

When the threshold was calculated as the mid-point between median MCP1 level in cluster 4 patient group and median VCP1 level in the remaining patients, the threshold level was 93.5 pg/ml. The distribution of MCP1 using this threshold level is shown in **FIG. 2E.** Using the threshold level of 93.5 pg/ml in the apremilast arms (both 20 mg and 30 mg arms), 36% of the patients that received a treatment with apremilast were grouped into MCP1 low group, and the MCP1 low group had a response rate of 49%. Looking at just the apremilast 30 mg arm and using the same threshold level of 93.5 pg/ml, 45% of the patients that received 30 mg apremilast treatment were classified into the MCP1 low group, and these patients had a response rate of 56%.

A third method used to calculate a MCP1 threshold level was by calculating the mean of thresholds obtained from 10-fold cross validation (discussed above). In particular, each fold provided an optimal threshold according to the AUC metric when classifying for cluster membership. Then, the mean value of the 10 thresholds was calculated. Using this method, the threshold of MCP1 was calculated as 75.63 pg/ml. The distribution of MCP1 based on this threshold level is shown in **FIG. 2F.** With this more restrictive learned threshold, the population selected into the MCP1 low group decreased while the response rate of the MCP1 low sub-group increased. For example, in the apremilast 30 mg arm, 22% of the patients that received 30 mg apremilast treatment were classified into the MCP1 low group, and these patients had a higher response rate of 67%.

Similar evaluation was also applied to each of the other biomarkers. Top biomarkers selected by nested cross validation are listed (in order) in **Table 3.**

**Table 3. Top Biomarkers Selected by Nested Cross Validation**

| Rank | Top Features | 2 | 3 | 4 | 5 | 6 | 7 | 8 | 9 | 10 |
|---|---|---|---|---|---|---|---|---|---|---|
| 1 | MCP1 | 8 | 10 | 10 | 10 | 10 | 10 | 10 | 10 | 10 |
| 2 | Interleukin-15 | 5 | 7 | 7 | 7 | 10 | 10 | 10 | 10 | 10 |
| 3 | C-Reactive Protein | 1 | 5 | 6 | 7 | 8 | 9 | 9 | 10 | 10 |
| 4 | Matrix Metalloproteinase-3 | 1 | 2 | 4 | 6 | 6 | 8 | 10 | 10 | 10 |

(continued)

| Rank | Top Features | 2 | 3 | 4 | 5 | 6 | 7 | 8 | 9 | 10 |
|---|---|---|---|---|---|---|---|---|---|---|
| 5 | Interleukin-1 receptor antagonist | 1 | 1 | 3 | 4 | 6 | 7 | 7 | 8 | 9 |
| 6 | von Willebrand Factor | 0 | 0 | 1 | 4 | 4 | 6 | 7 | 8 | 9 |
| 7 | Eotaxin-1 | 2 | 3 | 5 | 7 | 7 | 7 | 7 | 8 | 8 |
| 8 | Alpha-1-Antitrypsin | 0 | 0 | 1 | 2 | 3 | 3 | 3 | 4 | 8 |
| 9 | Macrophage Inflammatory Protein-1 beta | 1 | 1 | 2 | 2 | 3 | 4 | 5 | 6 | 7 |
| 10 | Vascular Cell Adhesion Molecule-1 | 0 | 0 | 0 | 0 | 1 | 3 | 6 | 7 | 7 |
| 11 | Tissue Inhibitor of Metalloproteinases | 1 | 1 | 1 | 1 | 1 | 2 | 2 | 2 | 2 |
| 12 | Osteoprotegerin | 0 | 0 | 0 | 0 | 0 | 0 | 0 | 2 | 2 |
| 13 | Vascular Endothelial Growth Factor | 0 | 0 | 0 | 0 | 0 | 0 | 2 | 2 | 2 |
| 14 | Alpha-2-Macroglobulin | 0 | 0 | 0 | 0 | 0 | 0 | 1 | 1 | 1 |
| 15 | Brain-Derived Neurotrophic Factor | 0 | 0 | 0 | 0 | 0 | 0 | 0 | 1 | 1 |
| 16 | Intercellular Adhesion Molecule 1 | 0 | 0 | 0 | 0 | 0 | 0 | 0 | 0 | 1 |
| 17 | Interleukin-18 | 0 | 0 | 0 | 0 | 1 | 1 | 1 | 1 | 1 |
| 18 | Matrix Metalloproteinase-9 | 0 | 0 | 0 | 0 | 0 | 0 | 0 | 0 | 1 |
| 19 | Vitamin D-Binding Protein | 0 | 0 | 0 | 0 | 0 | 0 | 0 | 0 | 1 |

Each of these top ranked biomarkers may be used alone to classify a patient for predicting the patient's response to a treatment with apremilast.

### 5.3 Example 3-Using Expression Levels of Multiple Biomarkers to Differentiate a Patient Group Having Higher Response Rate from the Remaining Patients

The performance of using multiple biomarkers to separate a patent group with higher response rate was studied in this example. Two classifiers-Naive Bayes and Weighted RSVM-were exemplified to classify a patient based on multiple biomarker levels.

Naive Bayes learns a probabilistic model between features and cluster membership that maximizes the posterior probability of the observed subgroups given the features (assuming independence of all features). Formula of Naive Bayes is:

$$\hat{y} = \underset{k \in \{1,\ldots,K\}}{\operatorname{argmax}} \ p(C_k) \prod_{i=1}^{n} p(x_i | C_k)$$ ,

where p(Ck) represents overall class (MCP1 low cluster membership) probability, K is number of classes (here two), n is the number of features (number of biomarkers used, *e.g.,* 2 for Top 2), x are the features themselves and p(xi|Ck) is the posterior probability of observing $x_i$ given class $C_k$.

SVM (support vector machine) is a classifier that draws a boundary between MCP1 low and non-MCPllow clusters. SVMs are supervised learning models with associated learning algorithms that analyze data and recognize patterns, used for classification and regression analysis. Given a set of training examples, each marked for belonging to one of two categories, an SVM training algorithm builds a model that assigns new examples into one category or the other, making it a non-probabilistic binary linear classifier. An SVM model is a representation of the examples as points in space, mapped so that the examples of the separate categories are divided by a clear gap that is as wide as possible. New examples are then mapped into that same space and predicted to belong to a category based on which side of the gap they fall on. Weighted RSVM is weighted reduced SVM. Reduced version of SVM uses a subset of the data to improve efficiency and generalization of the model. Weighted version balances classes of different sizes. Formula of

SVM is

$$f(\vec{x}) = \text{sign}(\sum \alpha_i y_i K(\vec{x}_i, \vec{x}) + b)$$

where $x_i$ is a sample represented by a vector of features and x is all the samples, K is a kernel matrix which encodes the distance between all samples, $y_i$ is the cluster membership for sample i, alpha is an indicator variable that describes which samples are closest to the classification boundary and b is an offset.

The performance of these two classifiers for classifying patients using top 2-10 or all 49 biomarkers are shown in **FIG. 3A,** and summarized in **Tables 4-5** below.

**Table 4. Performance of Naive Bayes for MCP1 Low Membership Prediction**

| NB | acc | auc | mcc | sens | spec | ppv | npv |
|---|---|---|---|---|---|---|---|
| top 2 | 0.82 | 0.87 | 0.52 | 0.46 | 0.96 | 0.82 | 0.82 |
| top 3 | 0.83 | 0.91 | 0.57 | 0.48 | 0.98 | 0.89 | 0.82 |
| top 4 | 0.84 | 0.90 | 0.60 | 0.62 | 0.94 | 0.79 | 0.86 |
| top 5 | 0.86 | 0.91 | 0.63 | 0.64 | 0.94 | 0.82 | 0.87 |
| top 6 | 0.84 | 0.90 | 0.61 | 0.66 | 0.92 | 0.77 | 0.87 |
| top 7 | 0.84 | 0.92 | 0.61 | 0.70 | 0.90 | 0.74 | 0.88 |
| top 8 | 0.87 | 0.93 | 0.67 | 0.74 | 0.92 | 0.79 | 0.90 |
| top 9 | 0.90 | 0.94 | 0.74 | 0.78 | 0.94 | 0.85 | 0.91 |
| top 10 | 0.91 | 0.95 | 0.77 | 0.82 | 0.94 | 0.85 | 0.93 |
| All | 0.79 | 0.89 | 0.54 | 0.78 | 0.80 | 0.61 | 0.90 |

**Table 5. Performance of Weighted RSVM for MCP1 Low Membership Prediction**

| WRSVM | acc | auc | mcc | sens | spec | ppv | npv |
|---|---|---|---|---|---|---|---|
| top1 (MCP1) | 0.82 | 0.74 | 0.53 | 0.5 | 0.95 | 0.81 | 0.83 |
| top 2 | 0.79 | 0.82 | 0.49 | 0.66 | 0.84 | 0.62 | 0.86 |
| top 3 | 0.84 | 0.89 | 0.61 | 0.70 | 0.90 | 0.74 | 0.88 |
| top 4 | 0.84 | 0.91 | 0.60 | 0.70 | 0.90 | 0.73 | 0.88 |
| top 5 | 0.86 | 0.91 | 0.65 | 0.74 | 0.90 | 0.76 | 0.90 |
| top 6 | 0.88 | 0.93 | 0.70 | 0.76 | 0.93 | 0.81 | 0.91 |
| top 7 | 0.87 | 0.94 | 0.68 | 0.80 | 0.90 | 0.75 | 0.92 |
| top 8 | 0.90 | 0.95 | 0.76 | 0.80 | 0.94 | 0.85 | 0.92 |
| top 9 | 0.89 | 0.97 | 0.74 | 0.84 | 0.91 | 0.79 | 0.93 |
| top 10 | 0.90 | 0.97 | 0.77 | 0.86 | 0.92 | 0.81 | 0.94 |
| All | 0.94 | 0.98 | 0.85 | 0.90 | 0.95 | 0.88 | 0.96 |

As discussed above, ACC indicates the proportion of predictions that the classifier gets correct. AUC indicates the probability that a randomly picked positive sample will have a higher score than a randomly picked negative sample. MCC is a correlation coefficient between the observed and predicted classifications. SENS indicates sensitivity or true positive rate, which means the proportion that is correctly classified in all MCP1 low samples. SPEC indicates specificity or true negative rate, which means the proportion that is correctly classified in all non-MCPl low samples. PPV indicates positive predictive value, which means the proportion that is correctly classified out of all people predicted to be in MCP1

low group. NPV indicates negative predictive value, which means the proportion that is correctly classified out of all people predicted to be not in MCP1 low group.

As shown in **FIG. 3A** and **Tables 4-5,** performance improved as the number of biomarkers increased. For example, the sensitivity increased with the number of biomarkers for both classifiers.

Identification of a patient group with higher response rate using top 2 biomarkers (MCP1 and Interleukin-15) is shown in **FIG. 3B.** Identification of a patient group with higher response rate using top 5 biomarkers (MCP1, Interleukin-15, C-Reactive Protein, Matrix Metalloproteinase-3, and Interleukin-1 receptor antagonist) is shown in **FIG. 3C.** Identification of a patient group with higher response rate using top 10 biomarkers (MCP1, Interleukin-15, C-Reactive Protein, Matrix Metalloproteinase-3, Interleukin-1 receptor antagonist, von Willebrand Factor, Eotaxin-1, Alpha-1-Antitrypsin, Macrophage Inflammatory Protein-1 beta, and Vascular Cell Adhesion Molecule-1) is shown in **FIG. 3D.** Identification of a patient group with higher response rate using all 49 biomarkers is shown in **FIG. 3E.** The response rate for each case is indicated in these figures.

These results indicate that the expression profile of these 49 biomarkers, or a subset thereof (*e.g.,* top 2, top 3, top 4, top 5, top 5, top 6, top 7, top 8, top 9, or top 10 biomarkers identified in the present study) can be used to classify a patient for the purpose of predicting the patient's response to apremilast treatment.

## Claims

1. A method of identifying a subject having ankylosing spondylitis who is likely to be responsive to a treatment with apremilast, or predicting the responsiveness of a subject having or suspected of having ankylosing spondylitis to a treatment with apremilast, comprising:

   (a) measuring the level of monocyte chemotactic protein 1 (MCP1) in a sample from the subject; and
   (b) diagnosing the subject as being likely to be responsive to apremilast if the level of MCP1 in the sample is lower than a threshold level of MCP1.

2. Apremilast for use in a method of treating a subject having ankylosing spondylitis, wherein the method comprises:

   (a) identifying the subject having ankylosing spondylitis that may be responsive to a treatment with apremilast according to the method of claim 1; and
   (b) administering to the subject a therapeutically effective amount of apremilast, if the subject is identified as being likely to be responsive to treatment with apremilast.

3. The method of claim 1, or apremilast for use of claim 2, wherein the threshold level of MCP1 is determined based on the levels of MCP1 in a population.

4. The method of claim 1, or apremilast for use of claim 2, wherein the threshold level of MCP1 is selected from the group consisting of between 200 pg/ml to 50 pg/ml and between 105 pg/ml to 70 pg/ml.

5. The method of claim 1, or apremilast for use of claim 2, wherein the threshold level of MCP1 is lower than 200 pg/ml, 150 pg/ml, 120 pg/ml, 105 pg/ml, 100 pg/ml, 95 pg/ml, 90 pg/ml, 85 pg/ml, 80 pg/ml, 75 pg/ml, or 70 pg/ml.

6. The method of claim 1, or apremilast for use of claim 2, wherein the threshold level of MCP1 is about 105 pg/ml.

7. The method of claim 1, or apremilast for use of claim 2, wherein the threshold level of MCP1 is about 95 pg/ml.

8. The method of claim 1, or apremilast for use of claim 2, wherein the threshold level of MCP1 is about 75 pg/ml.

9. The method of claim 1 or apremilast for use of claim 2, wherein the subject is diagnosed as having more than a 50% to 90% likelihood of being responsive to apremilast if the level of MCP1 is lower than the threshold level of MCP1.

10. The method of claim 1, or apremilast for use of claim 2, wherein the subject is diagnosed as having more than a 50%, 60%, 70%, 80% or 90% likelihood of being responsive to apremilast if the level of MCP1 is lower than the threshold level of MCP1.

11. The method of any one of claims 1 or 3 to 10, or apremilast for use of any one of claims 2 to 10, wherein the method further comprises:

(a) measuring the level of one additional biomarker Interleukin-15;
(b) measuring the level of two additional biomarkers including Interleukin-15 and C-Reactive Protein; or
(c) measuring the level of three additional biomarkers including Interleukin-15, C-Reactive Protein, and Matrix Metalloproteinase-3.

**12.** The method of any one of claims 1 or 3 to 10, or apremilast for use of any one of claims 2 to 10, wherein the method further comprises:

(a) measuring the level of four additional biomarkers including Interleukin-15, C-Reactive Protein, Matrix Merallopreoteinase-3, and Interleukin-1 receptor antagonist; or
(b) measuring the level of five additional biomarkers including Interleukin-15, C-Reactive Protein, Matrix Merallopreoteinase-3, and Interleukin-1 receptor antagonist, and von Willebrand Factor.

**13.** The method of any one of claims 1 or 3 to 10, or apremilast for use of any one of claims 2 to 10, wherein the method further comprises:

(a) measuring the level of six additional biomarkers including Interleukin-15, C-Reactive Protein, Matrix Merallopreoteinase-3, Interleukin-1 receptor antagonist, von Willebrand Factor, and Eotaxin-1; or
(b) measuring the level of seven additional biomarkers including Interleukin-15, C-Reactive Protein, Matrix Merallopreoteinase-3, Interleukin-1 receptor antagonist, von Willebrand Factor, Eotaxin-1, and Alpha-1-Antitrypsin.

**14.** The method of any one of claims 1 or 3 to 10, or apremilast for use of any one of claims 2 to 10, wherein the method further comprises:

(a) measuring the level of eight additional biomarkers including Interleukin-15, C-Reactive Protein, Matrix Merallopreoteinase-3, Interleukin-1 receptor antagonist, von Willebrand Factor, Eotaxin-1, Alpha-1-Antitrypsin, and Macrophage Inflammatory Protein-1 beta; or
(b) measuring the level of nine additional biomarkers including Interleukin-15, C-Reactive Protein, Matrix Merallopreoteinase-3, Interleukin-1 receptor antagonist, von Willebrand Factor, Eotaxin-1, Alpha-1-Antitrypsin, Macrophage Inflammatory Protein-1 beta, and Vascular Cell Adhesion Molecule-1.

**15.** The method of any one of claims 1 or 3 to 10, or apremilast for use of any one of claims 2 to 10, wherein the method further comprises measuring the level of one or more additional biomarkers selected from the group consisting of Alpha-1-Antitrypsin, Alpha-2-Macroglobulin, Beta-2-Microglobulin, Brain-Derived Neurotrophic Factor, C-Reactive Protein, Complement C3, Eotaxin-1, Factor VII, Ferritin, Fibrinogen, Granulocyte-Macrophage Colony-Stimulating Factor, Haptoglobin, Intercellular Adhesion Molecule 1, Interferon gamma, Interleukin-1 alpha, Interleukin-1 beta, Interleukin-1 receptor antagonist, Interleukin-10, Interleukin-12 Subunit p40, Interleukin-12 Subunit p70, Interleukin-15, Interleukin-17, Interleukin-2, Interleukin-23, Interleukin-3, Interleukin-4, Interleukin-5, Interleukin-6, Interleukin-7, Interleukin-8, Macrophage Inflammatory Protein-1 alpha, Macrophage Inflammatory Protein-1 beta, Matrix Metalloproteinase-3, Matrix Metalloproteinase-9, Stem Cell Factor, T-Cell-Specific Protein RANTES, Tissue Inhibitor of Metalloproteinases, Tumor Necrosis Factor alpha, Tumor Necrosis Factor beta, Tumor necrosis factor receptor 2, Vascular Cell Adhesion Molecule-1, Vascular Endothelial Growth Factor, Vitamin D-Binding Protein, von Willebrand Factor, Osteoprotegerin, Interleukin-18, RANKL, and Sclerostin.

## Patentansprüche

**1.** Verfahren zur Identifizierung eines Patienten mit Spondylitis ankylosans, der wahrscheinlich auf eine Behandlung mit Apremilast anspricht, oder zur Prognose des Ansprechens eines Patienten mit Spondylitis ankylosans oder mit Verdacht auf Spondylitis ankylosans auf eine Behandlung mit Apremilast, umfassend:

(a) Messung der Konzentration von Monozytenchemotaktischem Protein 1 (MCP1) in einer Probe des Patienten und
(b) Diagnose der Wahrscheinlichkeit, dass der Patient auf Apremilast anspricht, wenn die Konzentration von MCP1 in der Probe niedriger als ein Schwellenwert für MCP1 ist.

**2.** Apremilast zur Verwendung in einem Verfahren zur Behandlung eines Patienten mit Spondylitis ankylosans, wobei

das Verfahren umfasst:

(a) Identifizierung des Patienten mit Spondylitis ankylosans, der möglicherweise auf eine Behandlung mit Apremilast gemäß dem Verfahren nach Anspruch 1 anspricht und
(b) Verabreichung einer therapeutisch wirksamen Menge Apremilast an den Patienten, wenn der Patient als wahrscheinlich auf eine Behandlung mit Apremilast ansprechend ermittelt wurde.

**3.** Verfahren nach Anspruch 1 oder Apremilast zur Verwendung nach Anspruch 2, wobei der Schwellenwert für MCP1 auf der Grundlage der Konzentrationen von MCP1 in einer Population bestimmt wird.

**4.** Verfahren nach Anspruch 1 oder Apremilast zur Verwendung nach Anspruch 2, wobei der Schwellenwert von MCP1 ausgewählt ist aus der Gruppe bestehend aus zwischen 200 pg/ml bis 50 pg/ml und zwischen 105 pg/ml bis 70 pg/ml.

**5.** Verfahren nach Anspruch 1 oder Apremilast zur Verwendung nach Anspruch 2, wobei der Schwellenwert von MCP1 weniger als 200 pg/ml, 150 pg/ml, 120 pg/ml, 105 pg/ml, 100 pg/ml, 95 pg/ml, 90 pg/ml, 85 pg/ml, 80 pg/ml, 75 pg/ml oder 70 pg/ml beträgt.

**6.** Verfahren nach Anspruch 1 oder Apremilast zur Verwendung nach Anspruch 2, wobei der Schwellenwert von MCP1 etwa 105 pg/ml beträgt.

**7.** Verfahren nach Anspruch 1 oder Apremilast zur Verwendung nach Anspruch 2, wobei der Schwellenwert von MCP1 etwa 95 pg/ml beträgt.

**8.** Verfahren nach Anspruch 1 oder Apremilast zur Verwendung nach Anspruch 2, wobei der Schwellenwert von MCP1 etwa 75 pg/ml beträgt.

**9.** Verfahren nach Anspruch 1 oder Apremilast zur Verwendung nach Anspruch 2, wobei bei dem Patienten eine Wahrscheinlichkeit von mehr als 50 % bis 90 % diagnostiziert wird, auf Apremilast anzusprechen, wenn die Konzentration von MCP1 niedriger als der Schwellenwert für MCP1 ist.

**10.** Verfahren nach Anspruch 1 oder Apremilast zur Verwendung nach Anspruch 2, wobei bei dem Patienten eine Wahrscheinlichkeit von mehr als 50 %, 60 %, 70 %, 80 % oder 90 % diagnostiziert wird, auf Apremilast anzusprechen, wenn die Konzentration von MCP1 niedriger als der Schwellenwert für MCP1 ist.

**11.** Verfahren nach einem der Ansprüche 1 oder 3 bis 10 oder Apremilast zur Verwendung nach einem der Ansprüche 2 bis 10, wobei das Verfahren ferner umfasst:

(a) Messung der Konzentration eines zusätzlichen Biomarkers Interleukin-15;
(b) Messung der Konzentration von zwei zusätzlichen Biomarkern, einschließlich Interleukin-15 und C-reaktives Protein, oder
(c) Messung der Konzentration von drei zusätzlichen Biomarkern, einschließlich Interleukin-15, C-reaktives Protein und Matrix-Metalloproteinase-3.

**12.** Verfahren nach einem der Ansprüche 1 oder 3 bis 10 oder Apremilast zur Verwendung nach einem der Ansprüche 2 bis 10, wobei das Verfahren ferner umfasst:

(a) Messung der Konzentration von vier zusätzlichen Biomarkern, einschließlich Interleukin-15, C-reaktives Protein, Matrix-Metalloproteinase-3 und Interleukin-1-Rezeptor-Antagonist, oder
(b) Messung der Konzentration von fünf zusätzlichen Biomarkern, einschließlich Interleukin-15, C-reaktives Protein, Matrix-Metalloproteinase-3 und Interleukin-1-Rezeptor-Antagonist sowie von-Willebrand-Faktor.

**13.** Verfahren nach einem der Ansprüche 1 oder 3 bis 10 oder Apremilast zur Verwendung nach einem der Ansprüche 2 bis 10, wobei das Verfahren ferner umfasst:

(a) Messung der Konzentration von sechs zusätzlichen Biomarkern, einschließlich Interleukin-15, C-reaktives Protein, Matrix-Metalloproteinase-3, Interleukin-1-Rezeptor-Antagonist, von-Willebrand-Faktor und Eotaxin-1, oder
(b) Messung der Konzentration von sieben zusätzlichen Biomarkern, einschließlich Interleukin-15, C-reaktives

Protein, Matrix-Metalloproteinase-3, Interleukin-1-Rezeptor-Antagonist, von-Willebrand-Faktor, Eotaxin-1 und Alpha-1-Antitrypsin.

**14.** Verfahren nach einem der Ansprüche 1 oder 3 bis 10 oder Apremilast zur Verwendung nach einem der Ansprüche 2 bis 10, wobei das Verfahren ferner umfasst:

(a) Messung der Konzentration von acht zusätzlichen Biomarkern, einschließlich Interleukin-15, C-reaktives Protein, Matrix-Metalloproteinase-3, Interleukin-1-Rezeptor-Antagonist, von-Willebrand-Faktor, Eotaxin-1, Alpha-1-Antitrypsin und Makrophagen-Entzündungsprotein-1 beta, oder
(b) Messung der Konzentration von neun zusätzlichen Biomarkern, einschließlich Interleukin-15, C-reaktives Protein, Matrix-Metalloproteinase-3, Interleukin-1-Rezeptor-Antagonist, von-Willebrand-Faktor, Eotaxin-1, Alpha-1-Antitrypsin, Makrophagen-Entzündungsprotein-1 beta und vaskuläres Zelladhäsionsmolekül-1.

**15.** Verfahren nach einem der Ansprüche 1 oder 3 bis 10 oder Apremilast zur Verwendung nach einem der Ansprüche 2 bis 10, wobei das Verfahren ferner die Messung der Konzentration eines oder mehrerer zusätzlicher Biomarker umfasst, ausgewählt aus der Gruppe bestehend aus Alpha-1-Antitrypsin, Alpha-2-Makroglobulin, Beta-2-Mikroglobulin, Brain-Derived Neurotrophic Factor, C-reaktives Protein, Komplement C3, Eotaxin-1, Faktor VII, Ferritin, Fibrinogen, Granulozyten-Makrophagen-Kolonie-stimulierender Faktor, Haptoglobin, Interzelluläres Adhäsionsmolekül 1, Interferon gamma, Interleukin-1 alpha, Interleukin-1 beta, Interleukin-1-Rezeptor-Antagonist, Interleukin-10, Interleukin-12-Untereinheit p40, Interleukin-12-Untereinheit p70, Interleukin-15, Interleukin-17, Interleukin-2, Interleukin-23, Interleukin-3, Interleukin-4, Interleukin-5, Interleukin-6, Interleukin-7, Interleukin-8, Makrophagen-Entzündungsprotein-1 alpha, Makrophagen-Entzündungsprotein-1 beta, Matrix Metalloproteinase-3, Matrix Metalloproteinase-9, Stammzellfaktor, T-Zell-spezifisches Protein RANTES, Gewebeinhibitor von Metalloproteinasen, Tumor-Nekrose-Faktor alpha, Tumor-Nekrose-Faktor beta, Tumor-Nekrose-Faktor-Rezeptor 2, Vaskuläres Zelladhäsionsmolekül-1, Vaskulärer Endothelialer Wachstumsfaktor, Vitamin-D-bindendes Protein, von-Willebrand-Faktor, Osteoprotegerin, Interleukin-18, RANKL und Sclerostin.

## Revendications

**1.** Procédé d'identification d'un sujet ayant une spondylite ankylosante, qui est susceptible de répondre à un traitement avec l'aprémilast, ou de prédiction de la réponse d'un sujet ayant ou suspecté d'avoir une spondylite ankylosante à un traitement avec l'aprémilast, comprenant :

(a) la mesure du niveau de la protéine chimiotactique monocytaire 1 (MCP1) dans un échantillon provenant du sujet ; et
(b) le diagnostic du sujet comme étant susceptible de répondre à l'aprémilast si le niveau de MCP1 dans l'échantillon est inférieur à un niveau seuil de MCP1.

**2.** Aprémilast pour une utilisation dans un procédé de traitement d'un sujet ayant une spondylite ankylosante, le procédé comprenant :

(a) l'identification du sujet ayant une spondylite ankylosante qui peut répondre à un traitement avec l'aprémilast conformément au procédé selon la revendication 1 ; et
(b) l'administration au sujet d'une quantité thérapeutiquement efficace d'aprémilast si le sujet est identifié comme étant susceptible de répondre à un traitement avec l'aprémilast.

**3.** Procédé selon la revendication 1, ou aprémilast pour une utilisation selon la revendication 2, dans lequel le niveau seuil de MCP1 est déterminé sur la base des niveaux de MCP1 dans une population.

**4.** Procédé selon la revendication 1, ou aprémilast pour une utilisation selon la revendication 2, dans lequel le niveau seuil de MCP1 est choisi dans le groupe consistant en entre 200 pg/ml et 50 pg/ml et entre 105 pg/ml et 70 pg/ml.

**5.** Procédé selon la revendication 1, ou aprémilast pour une utilisation selon la revendication 2, dans lequel le niveau seuil de MCP1 est inférieur à 200 pg/ml, 150 pg/ml, 120 pg/ml, 105 pg/ml, 100 pg/ml, 95 pg/ml, 90 pg/ml, 85 pg/ml, 80 pg/ml, 75 pg/ml ou 70 pg/ml.

**6.** Procédé selon la revendication 1, ou aprémilast pour une utilisation selon la revendication 2, dans lequel le niveau

seuil de MCP1 est d'environ 105 pg/ml.

**7.** Procédé selon la revendication 1, ou aprémilast pour une utilisation selon la revendication 2, dans lequel le niveau seuil de MCP1 est d'environ 95 pg/ml.

**8.** Procédé selon la revendication 1, ou aprémilast pour une utilisation selon la revendication 2, dans lequel le niveau seuil de MCP1 est d'environ 75 pg/ml.

**9.** Procédé selon la revendication 1, ou aprémilast pour une utilisation selon la revendication 2, dans lequel le sujet est diagnostiqué comme ayant une probabilité supérieure à 50 % et allant jusqu'à 90 % de répondre à l'aprémilast si le niveau de MCP1 est inférieur au niveau seuil de MCP1.

**10.** Procédé selon la revendication 1, ou aprémilast pour une utilisation selon la revendication 2, dans lequel le sujet est diagnostiqué comme ayant une probabilité supérieure à 50 %, 60 %, 70 %, 80 % ou 90 % de répondre à l'aprémilast si le niveau de MCP1 est inférieur au niveau seuil de MCP1.

**11.** Procédé selon l'une quelconque des revendications 1 ou 3 à 10, ou aprémilast pour une utilisation selon l'une quelconque des revendications 2 à 10, le procédé comprenant en outre :

(a) la mesure du niveau d'un biomarqueur additionnel interleukine-15 ;
(b) la mesure du niveau de deux biomarqueurs additionnels incluant l'interleukine-15 et la protéine C-réactive ; ou
(c) la mesure du niveau de trois biomarqueurs additionnels incluant l'interleukine-15, la protéine C-réactive et la métalloprotéinase matricielle-3.

**12.** Procédé selon l'une quelconque des revendications 1 ou 3 à 10, ou aprémilast pour une utilisation selon l'une quelconque des revendications 2 à 10, le procédé comprenant en outre :

(a) la mesure du niveau de quatre biomarqueurs additionnels incluant l'interleukine-15, la protéine C-réactive, la métalloprotéinase matricielle-3 et l'antagoniste du récepteur de l'interleukine-1 ; ou
(b) la mesure du niveau de cinq biomarqueurs additionnels incluant l'interleukine-15, la protéine C-réactive, la métalloprotéinase matricielle-3 et l'antagoniste du récepteur de l'interleukine-1 et le facteur de von Willebrand.

**13.** Procédé selon l'une quelconque des revendications 1 ou 3 à 10, ou aprémilast pour une utilisation selon l'une quelconque des revendications 2 à 10, le procédé comprenant en outre :

(a) la mesure du niveau de six biomarqueurs additionnels incluant l'interleukine-15, la protéine C-réactive, la métalloprotéinase matricielle-3, l'antagoniste du récepteur de l'interleukine-1, le facteur de von Willebrand et l'éotaxine-1 ; ou
(b) la mesure du niveau de sept biomarqueurs additionnels incluant l'interleukine-15, la protéine C-réactive, la métalloprotéinase matricielle-3, l'antagoniste du récepteur de l'interleukine-1, le facteur de von Willebrand, l'éotaxine-1 et l'alpha-1-antitrypsine.

**14.** Procédé selon l'une quelconque des revendications 1 ou 3 à 10, ou aprémilast pour une utilisation selon l'une quelconque des revendications 2 à 10, le procédé comprenant en outre :

(a) la mesure du niveau de huit biomarqueurs additionnels incluant l'interleukine-15, la protéine C-réactive, la métalloprotéinase matricielle-3, l'antagoniste du récepteur de l'interleukine-1, le facteur de von Willebrand, l'éotaxine-1, l'alpha-1-antitrypsine et la protéine-1 bêta inflammatoire des macrophages ; ou
(b) la mesure du niveau de neuf biomarqueurs additionnels incluant l'interleukine-15, la protéine C-réactive, la métalloprotéinase matricielle-3, l'antagoniste du récepteur de l'interleukine-1, le facteur de von Willebrand, l'éotaxine-1, l'alpha-1-antitrypsine, la protéine-1 bêta inflammatoire des macrophages et la molécule-1 d'adhésion cellulaire vasculaire.

**15.** Procédé selon l'une quelconque des revendications 1 ou 3 à 10, ou aprémilast pour une utilisation selon l'une quelconque des revendications 2 à 10, le procédé comprenant en outre la mesure du niveau d'un ou plusieurs biomarqueurs additionnels choisis dans le groupe consistant en l'alpha-1-antitrypsine, l'alpha-2-macroglobuline, la bêta-2-microglobuline, le facteur neurotrophique dérivé du cerveau, la protéine C-réactive, le complément C3, l'éotaxine-1, le facteur VII, la ferritine, le fibrinogène, le facteur stimulant les colonies de granulocytes et de macrophages,

l'haptoglobine, la molécule 1 d'adhésion intercellulaire, l'interféron gamma, l'interleukine-1 alpha, l'interleukine-1 bêta, l'antagoniste du récepteur de l'interleukine-1, l'interleukine-10, la sous-unité p40 de l'interleukine-12, la sous-unité p70 de l'interleukine-12, l'interleukine-15, l'interleukine-17, l'interleukine-2, l'interleukine-23, l'interleukine-3, l'interleukine-4, l'interleukine-5, l'interleukine-6, l'interleukine-7, l'interleukine-8, la protéine-1 alpha inflammatoire des macrophages, la protéine-1 bêta inflammatoire des macrophages, la métalloprotéinase matricielle-3, la métalloprotéinase matricielle-9, le facteur des cellules-souches, la protéine spécifique du lymphocyte T RANTES, l'inhibiteur tissulaire des métalloprotéinases, le facteur alpha de nécrose tumorale, le facteur bêta de nécrose tumorale, le récepteur 2 du facteur de nécrose tumorale, la molécule-1 d'adhésion cellulaire vasculaire, le facteur de croissance endothélial vasculaire, la protéine de liaison à la vitamine D, le facteur de von Willebrand, l'ostéoprotégérine, l'interleukine-18, le RANKL et la sclérostine.

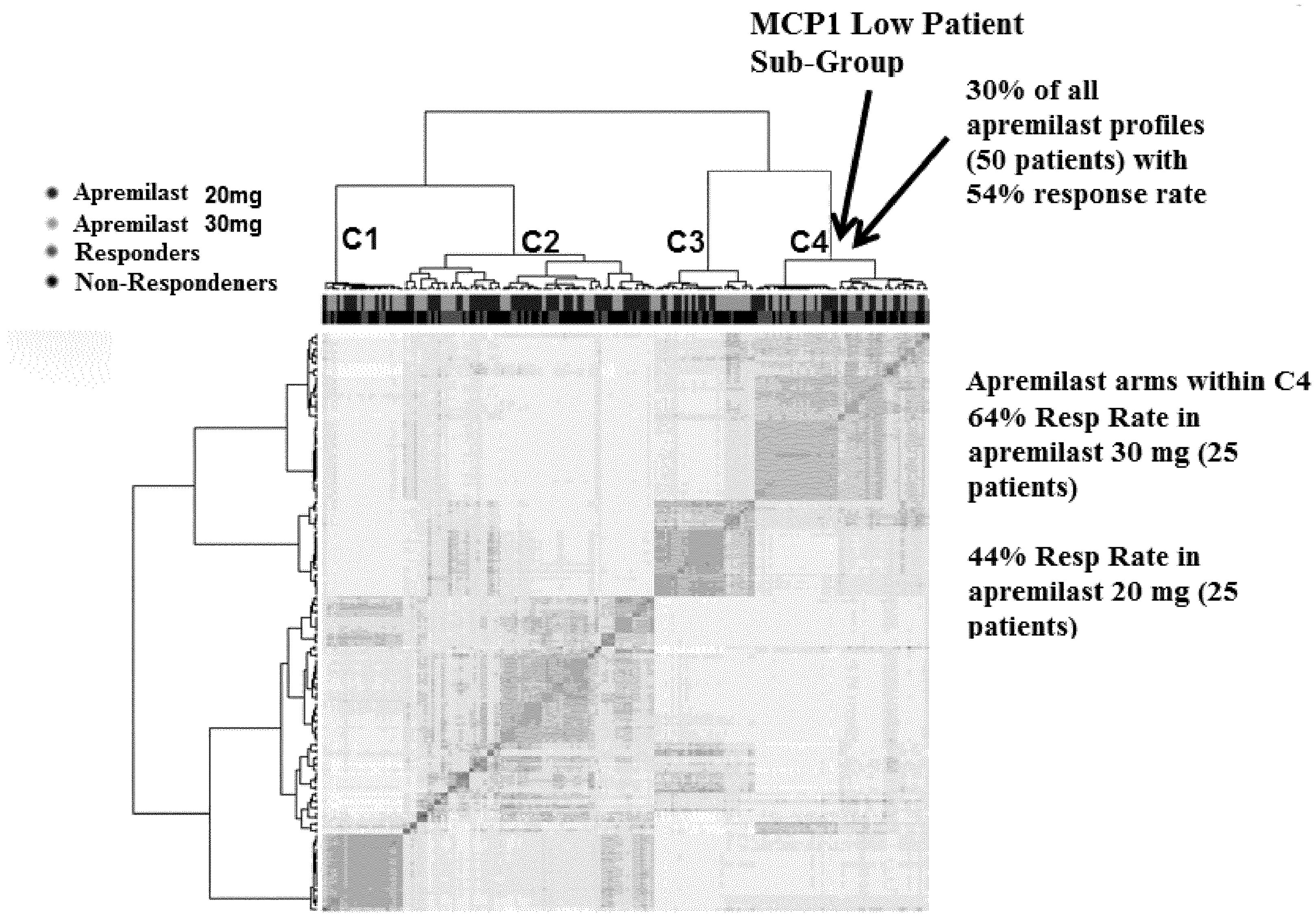

MCP1 Low Patient Sub-Group
30% of all apremilast profiles (50 patients) with 54% response rate
Apremilast 20mg
Apremilast 30mg
Responders
Non-Respondeners
C1
C2
C3
C4
Apremilast arms within C4
64% Resp Rate in apremilast 30 mg (25 patients)
44% Resp Rate in apremilast 20 mg (25 patients)


FIG. 1A

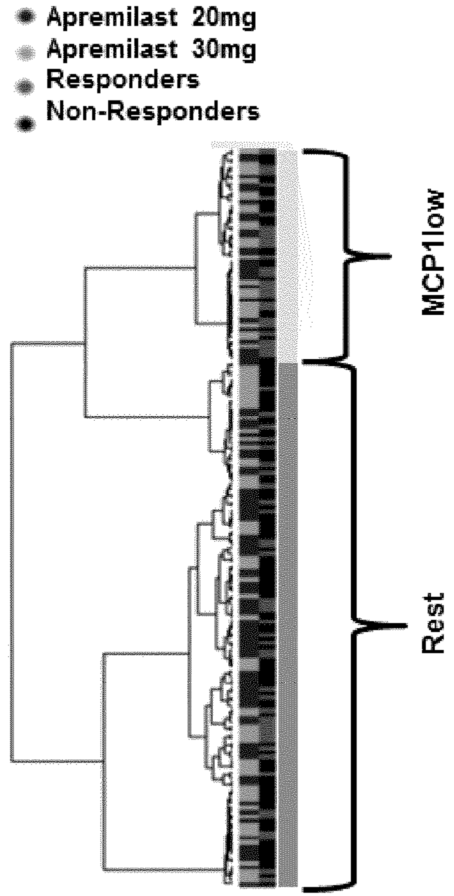


- **Significant correlation (CC) between MCP1low Membership and Response Rate (pval=0.002, CC=0.21)**
  - More significant in Apre 30mg arm (pval=**2e-04**, CC =**0.35**)
- **Significant Response Rate in MCP1low vs the Rest: 0.54 vs 0.31 (pval 0.0014)**
  - More significant in Apre 30mg: **0.64** vs **0.27** (pval **6e-04**)

FIG. 1B

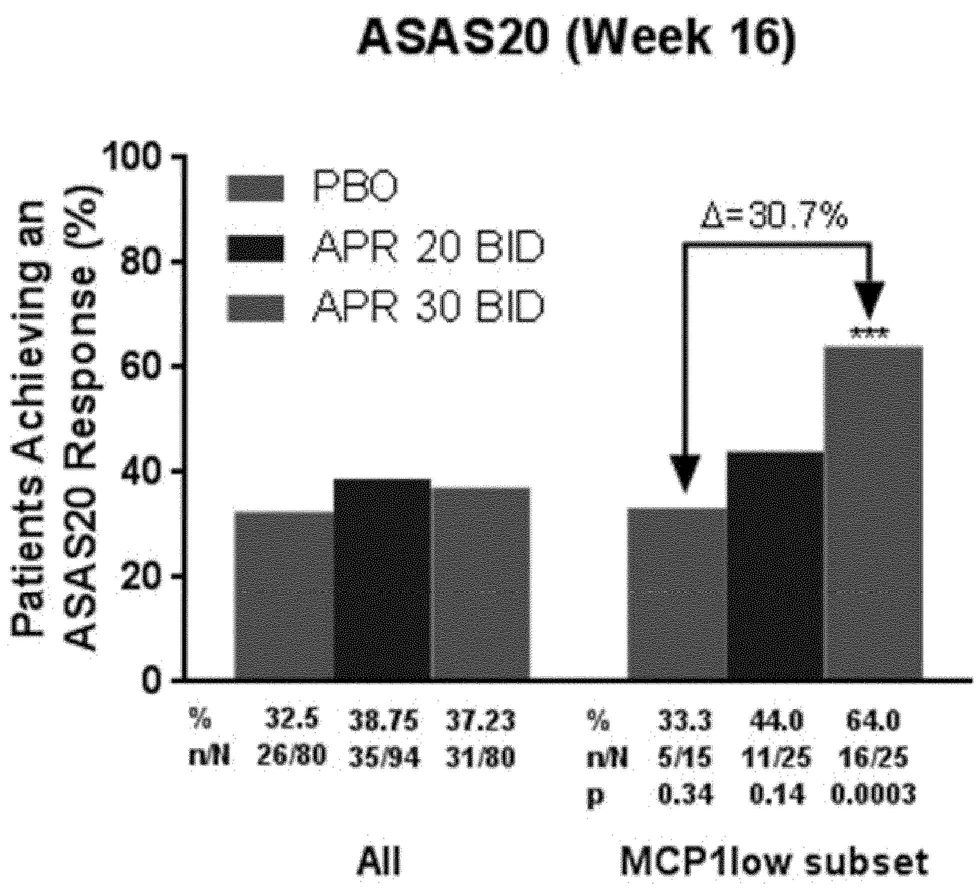


*p≤0.05, **p≤0.01, ***p≤0.001 by hypergeometric test for increase in response rate in MCP-1 low subset vs. overall biomarker subset

FIG. 1C

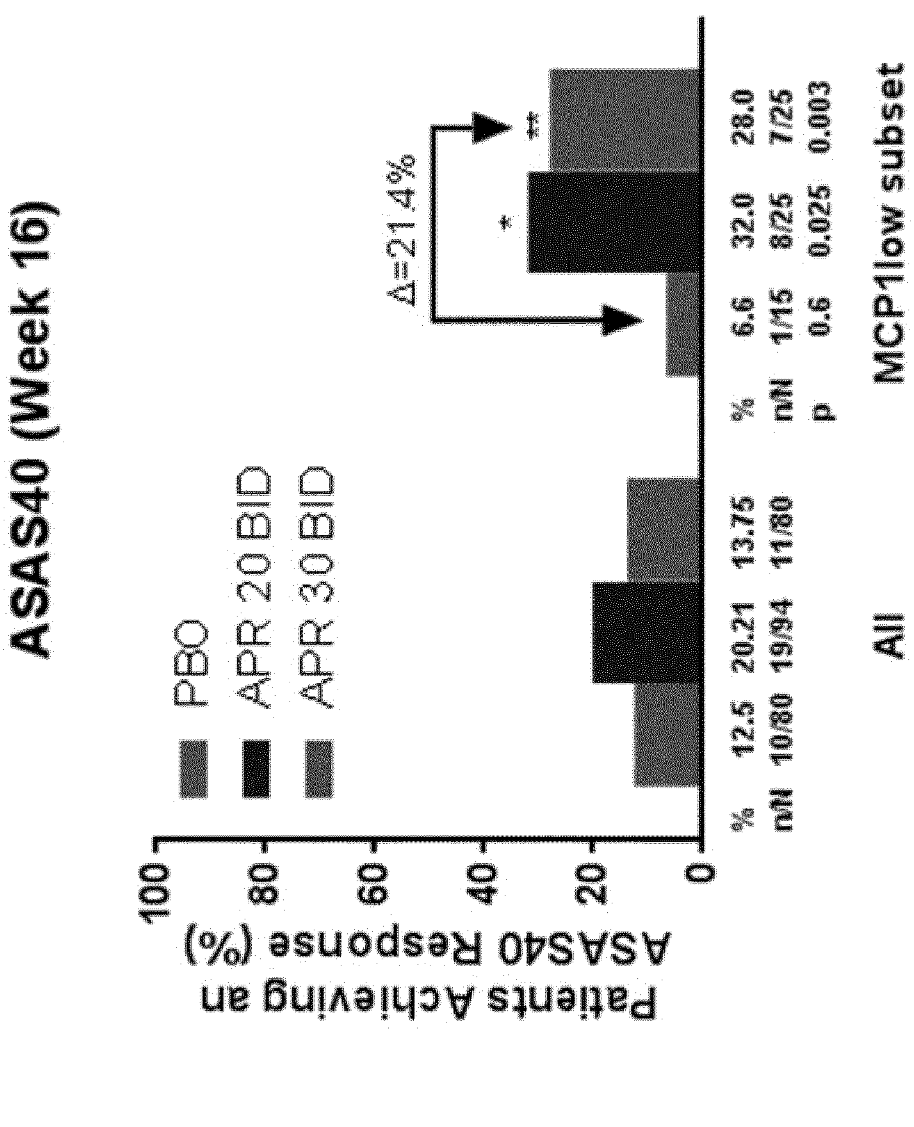


*p≤0.05, **p≤0.01, ***p≤0.001 by hypergeometric test for increase in response rate in MCP-1 low subset vs. overall biomarker subset

FIG. 1D

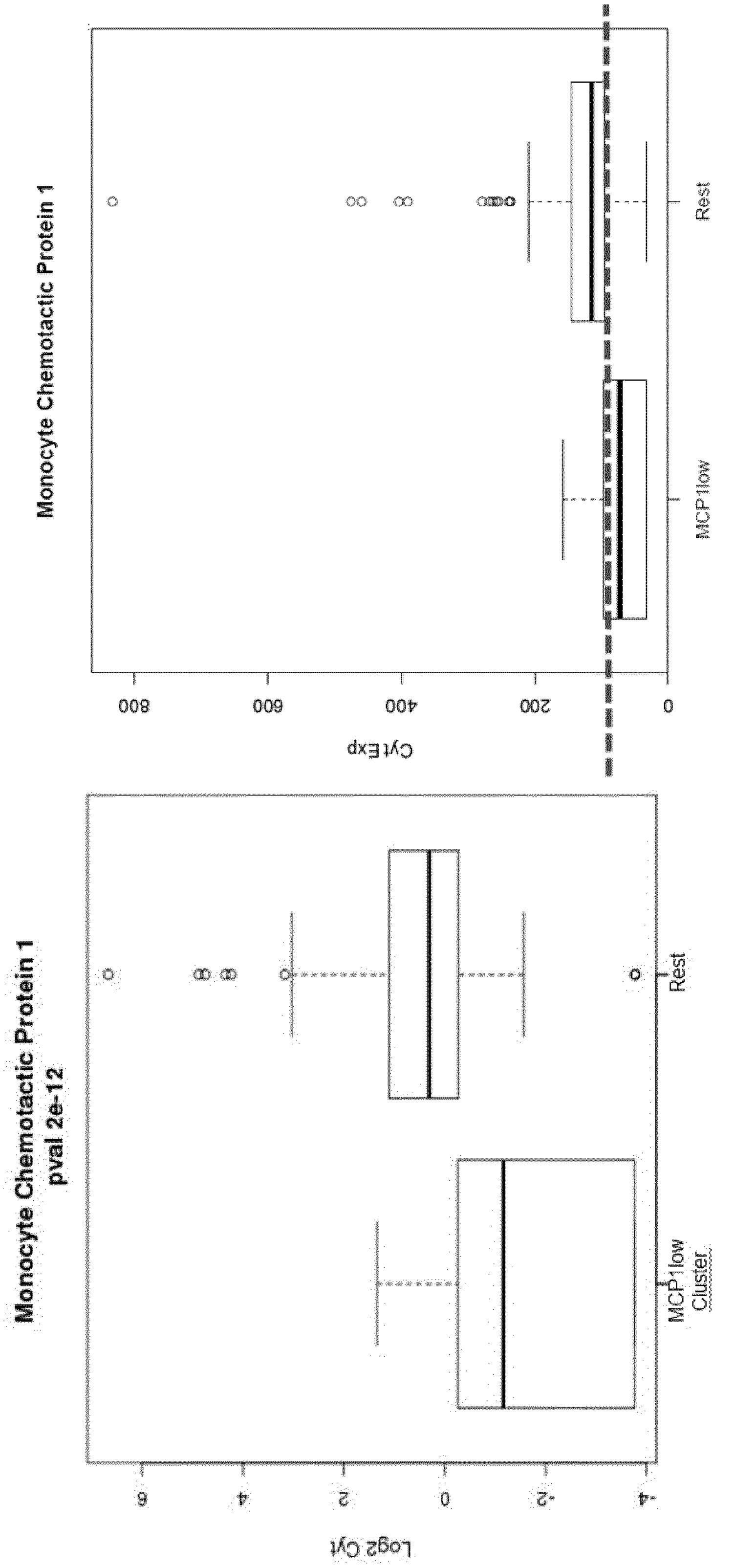
Monocyte Chemotactic Protein 1
Cyt Exp
800
600
400
200
0
MCP1low
Rest
Monocyte Chemotactic Protein 1
pval 2e-12
Log2 Cyt
6
4
2
0
-2
-4
MCP1low
Cluster
Rest

FIG. 2A

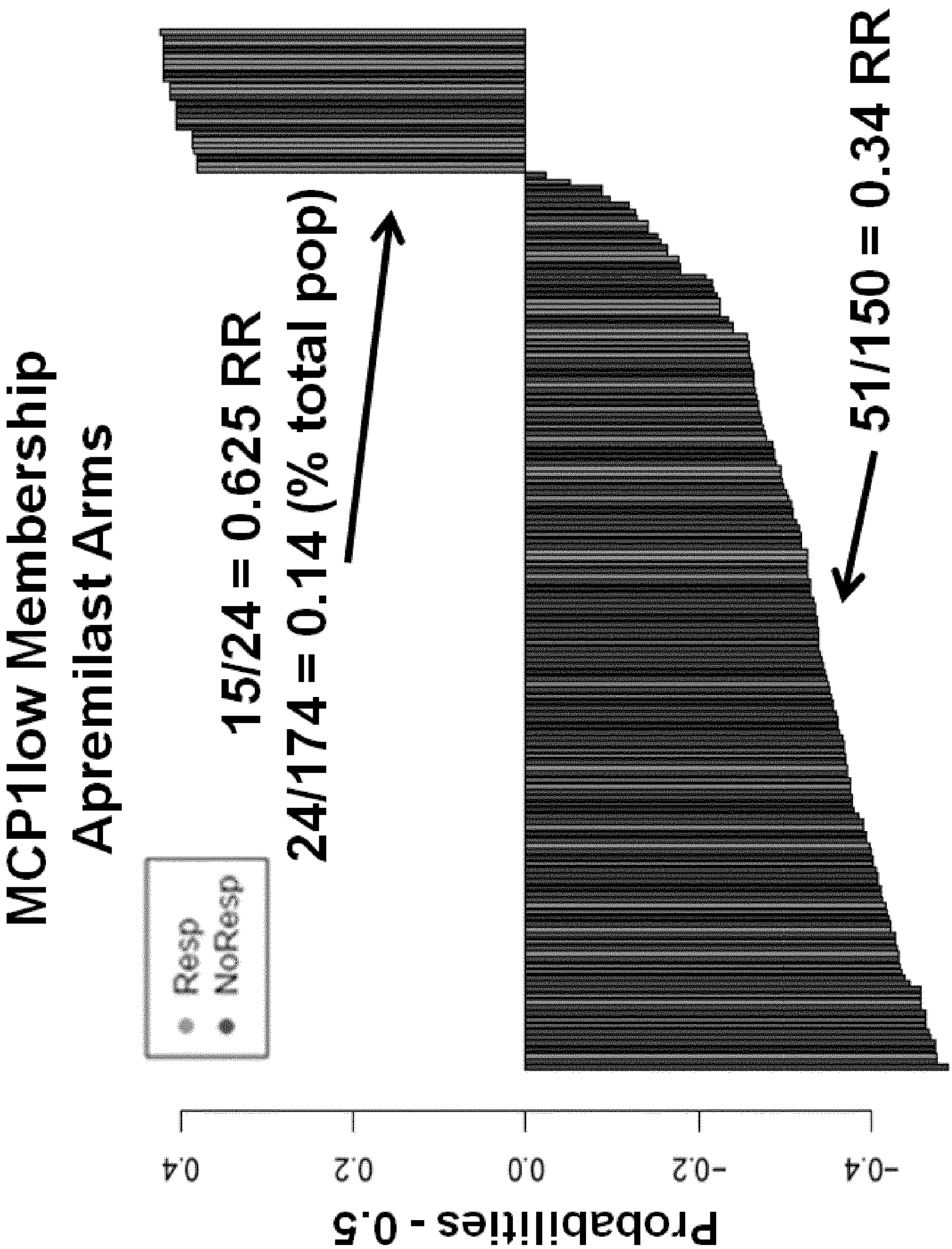
MCP1low Membership
Apremilast Arms
Resp
NoResp
15/24 = 0.625 RR
24/174 = 0.14 (% total pop)
51/150 = 0.34 RR
0.4
0.2
0.0
−0.2
−0.4
Probabilities - 0.5

FIG. 2B

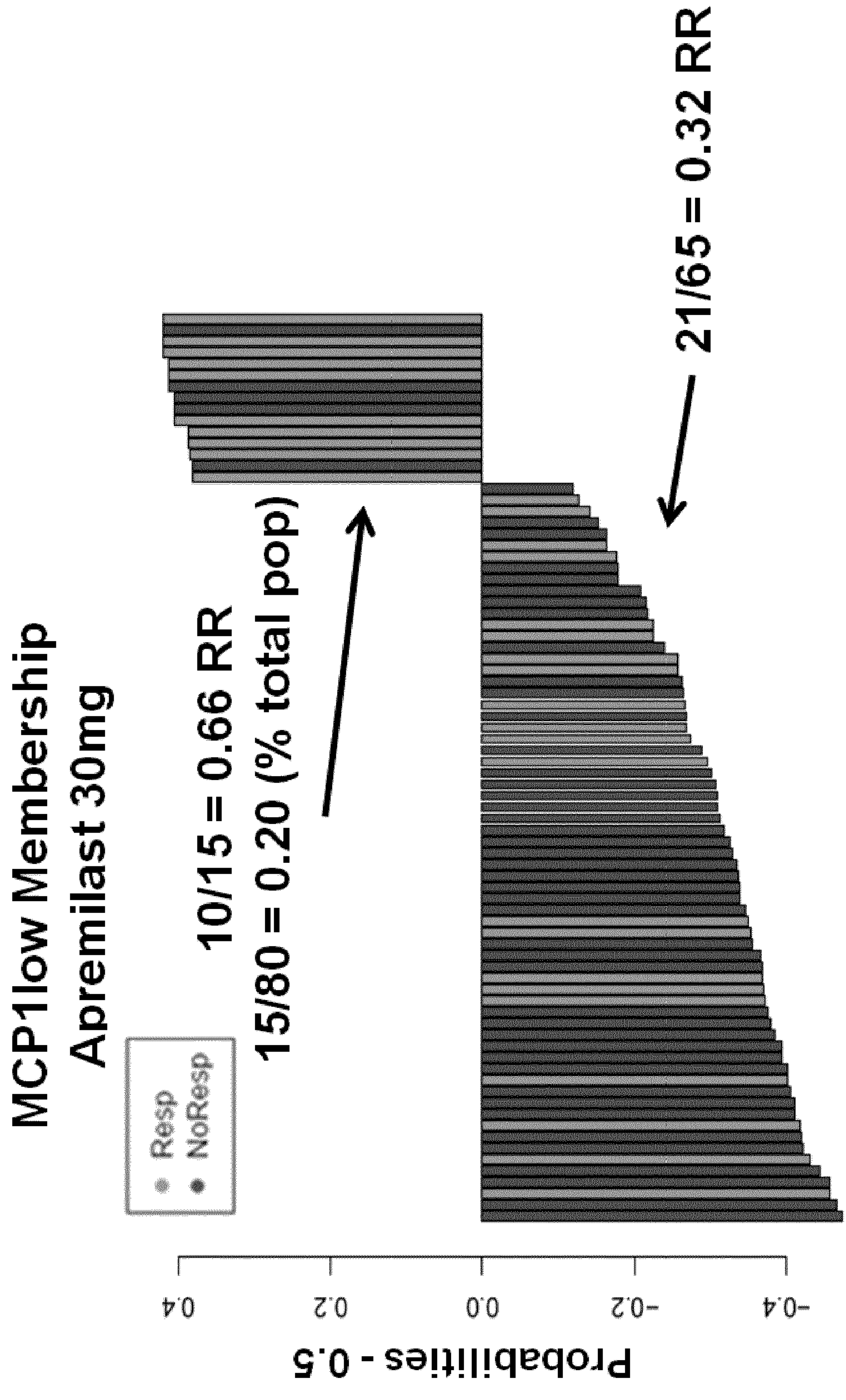
MCP1low Membership
Apremilast 30mg
Resp
NoResp
10/15 = 0.66 RR
15/80 = 0.20 (% total pop)
21/65 = 0.32 RR
Probabilities - 0.5
0.4
0.2
0.0
-0.2
-0.4

FIG. 2C

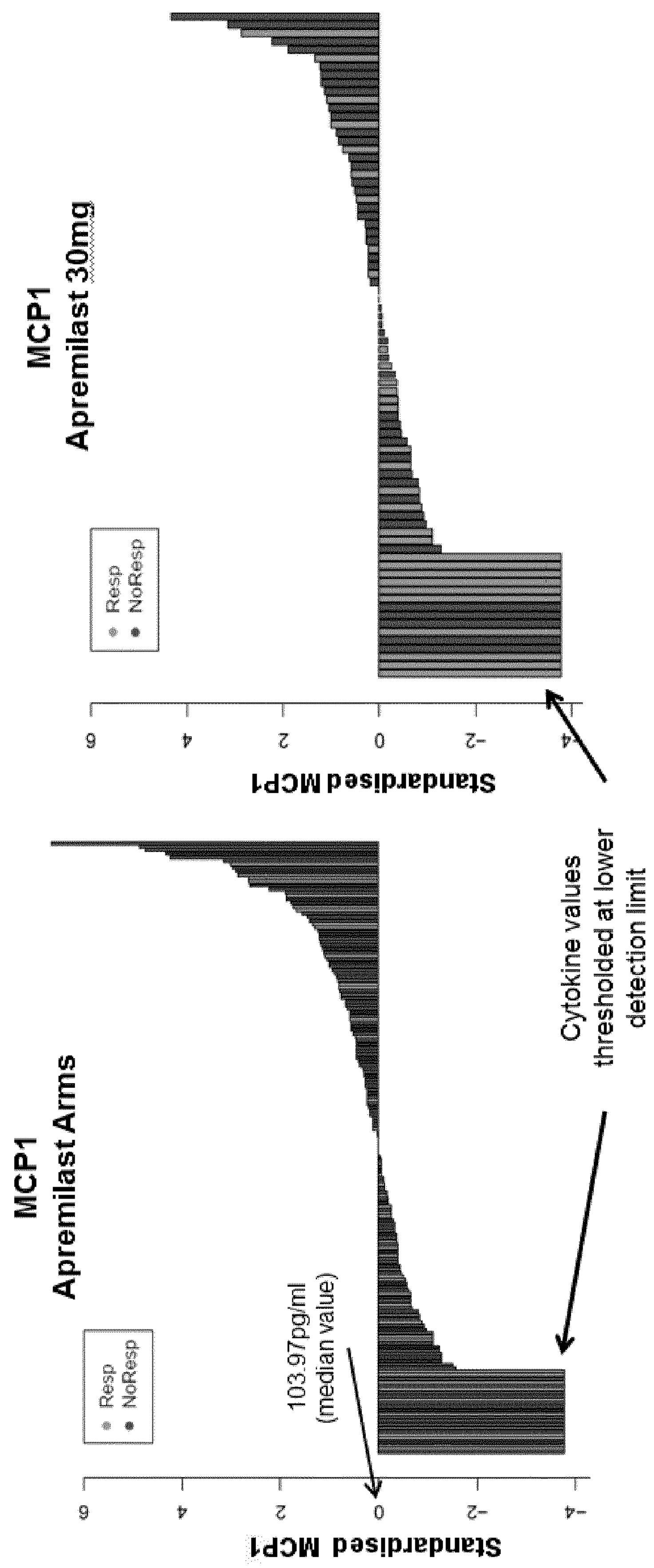
MCP1
Apremilast Arms
Resp
NoResp
103.97pg/ml
(median value)
Standardised MCP1
6
4
2
0
-2
-4
Cytokine values
thresholded at lower
detection limit
MCP1
Apremilast 30mg
Resp
NoResp
Standardised MCP1
6
4
2
0
-2
-4

FIG. 2D

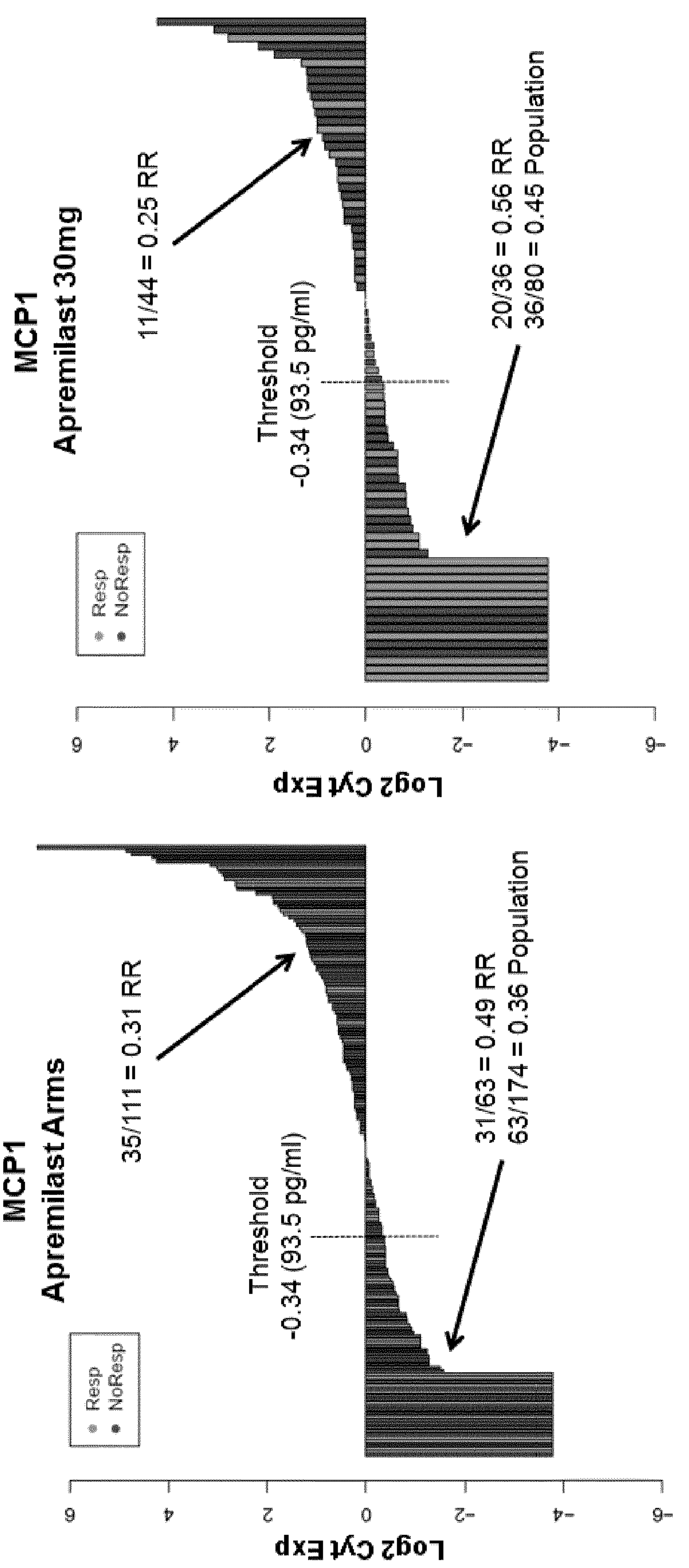
MCP1
Apremilast Arms
Resp
NoResp
35/111 = 0.31 RR
Threshold
-0.34 (93.5 pg/ml)
31/63 = 0.49 RR
63/174 = 0.36 Population
Log2 Cyt Exp
6
4
2
0
-2
-4
-6
MCP1
Apremilast 30mg
Resp
NoResp
11/44 = 0.25 RR
Threshold
-0.34 (93.5 pg/ml)
20/36 = 0.56 RR
36/80 = 0.45 Population
Log2 Cyt Exp
6
4
2
0
-2
-4
-6

FIG. 2E

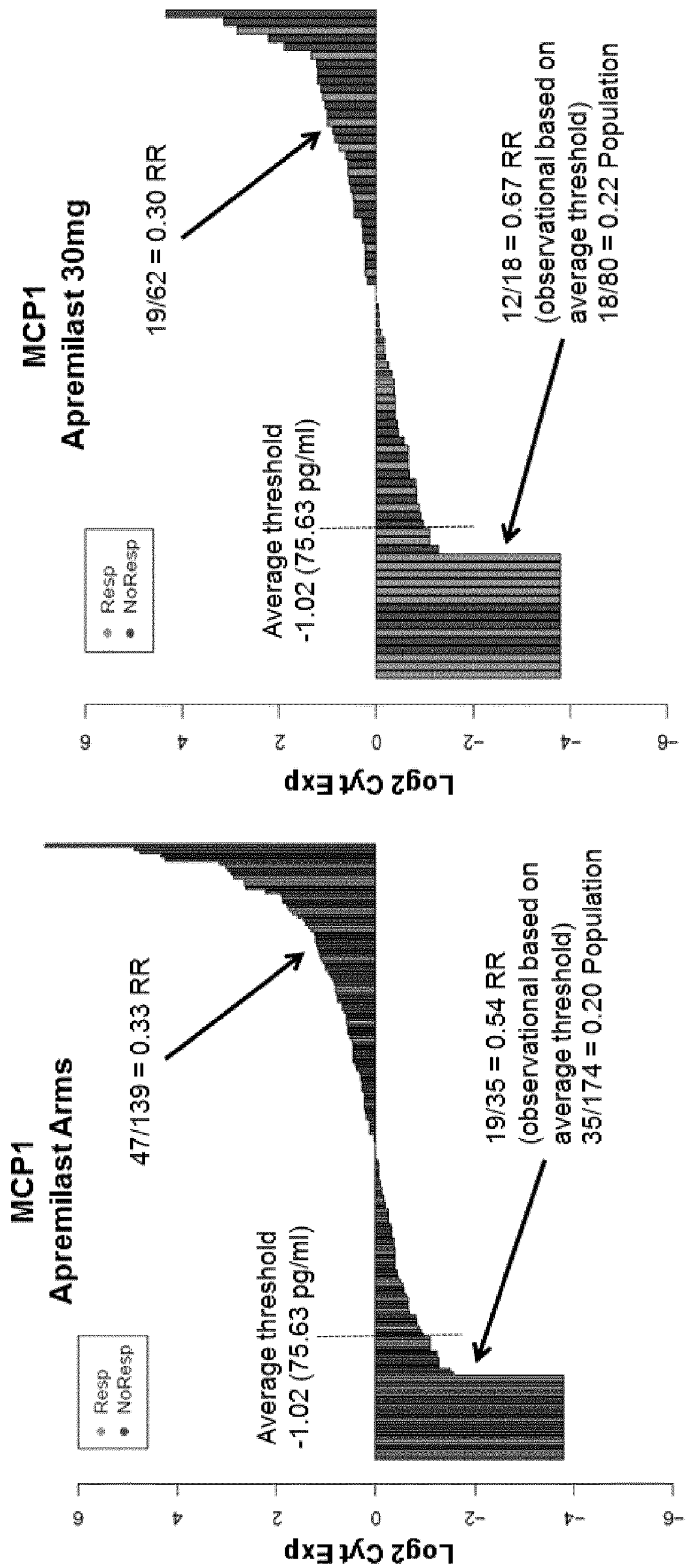
MCP1
Apremilast Arms
Resp
NoResp
Average threshold
-1.02 (75.63 pg/ml)
47/139 = 0.33 RR
19/35 = 0.54 RR
(observational based on
average threshold)
35/174 = 0.20 Population
Log2 Cyt Exp
6
4
2
0
-2
-4
-6
MCP1
Apremilast 30mg
Resp
NoResp
Average threshold
-1.02 (75.63 pg/ml)
19/62 = 0.30 RR
12/18 = 0.67 RR
(observational based on
average threshold)
18/80 = 0.22 Population
Log2 Cyt Exp
6
4
2
0
-2
-4
-6

FIG. 2F

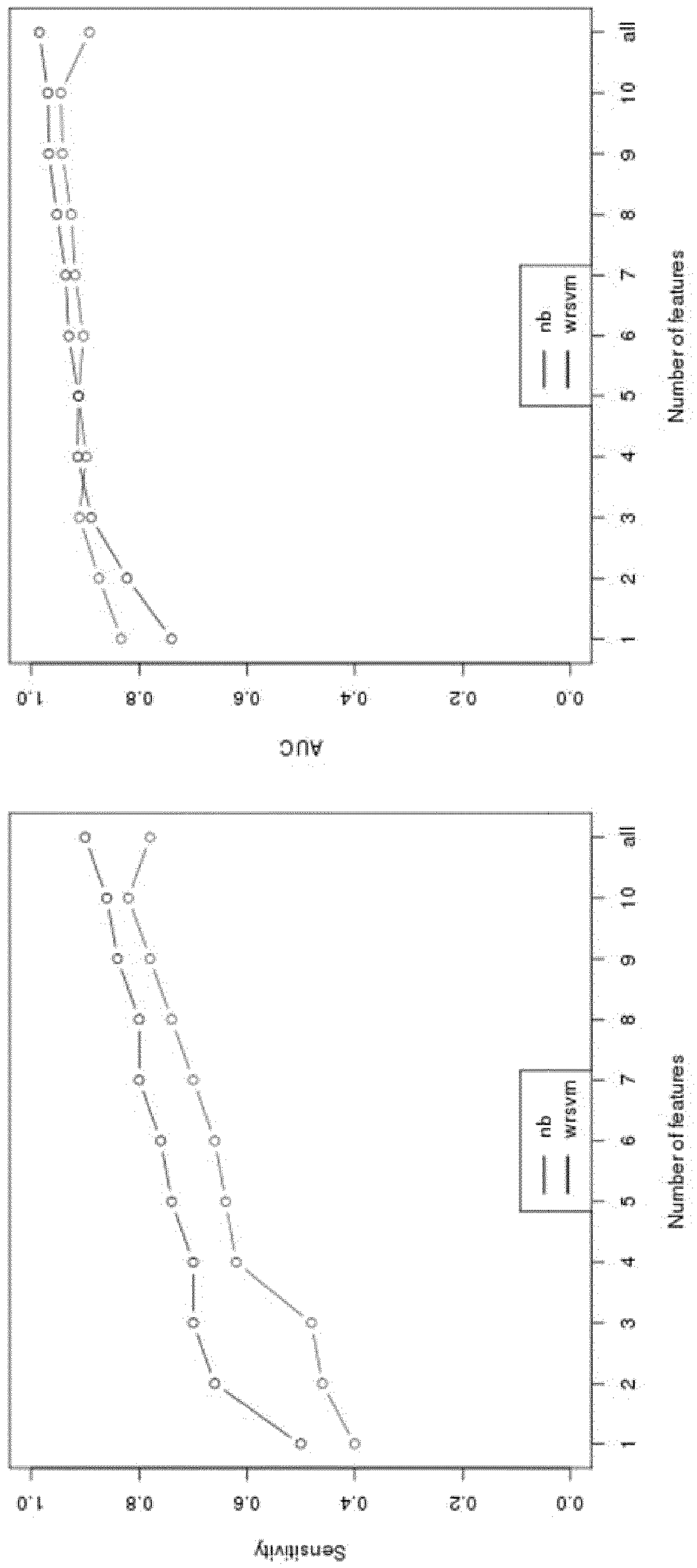
AUC
Sensitivity
Number of features
nb
wrsvm
1.0
0.8
0.6
0.4
0.2
0.0
1
2
3
4
5
6
7
8
9
10
all

FIG. 3A

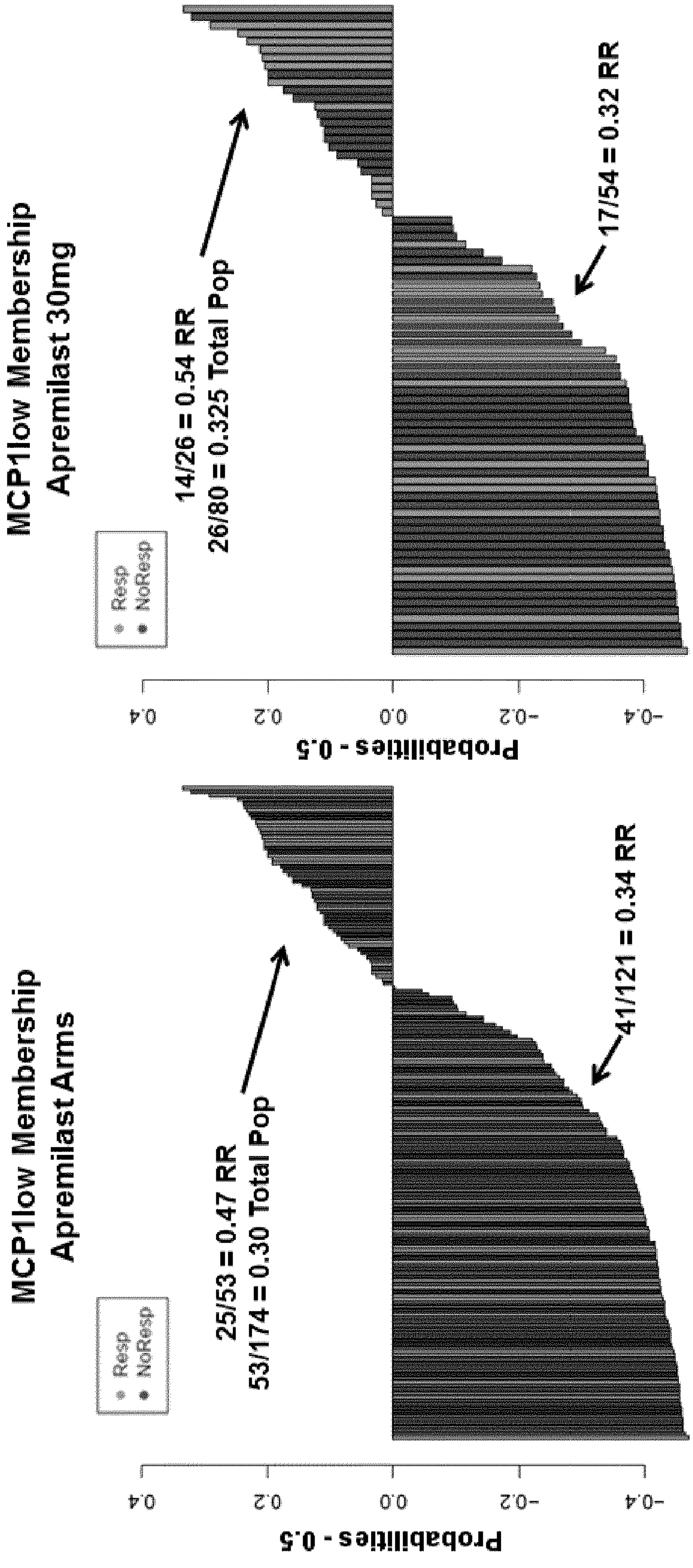
MCP1low Membership
Apremilast 30mg
Resp
NoResp
14/26 = 0.54 RR
26/80 = 0.325 Total Pop
17/54 = 0.32 RR
Probabilities - 0.5
0.4
0.2
0.0
−0.2
−0.4
MCP1low Membership
Apremilast Arms
Resp
NoResp
25/53 = 0.47 RR
53/174 = 0.30 Total Pop
41/121 = 0.34 RR
Probabilities - 0.5
0.4
0.2
0.0
−0.2
−0.4

FIG. 3B

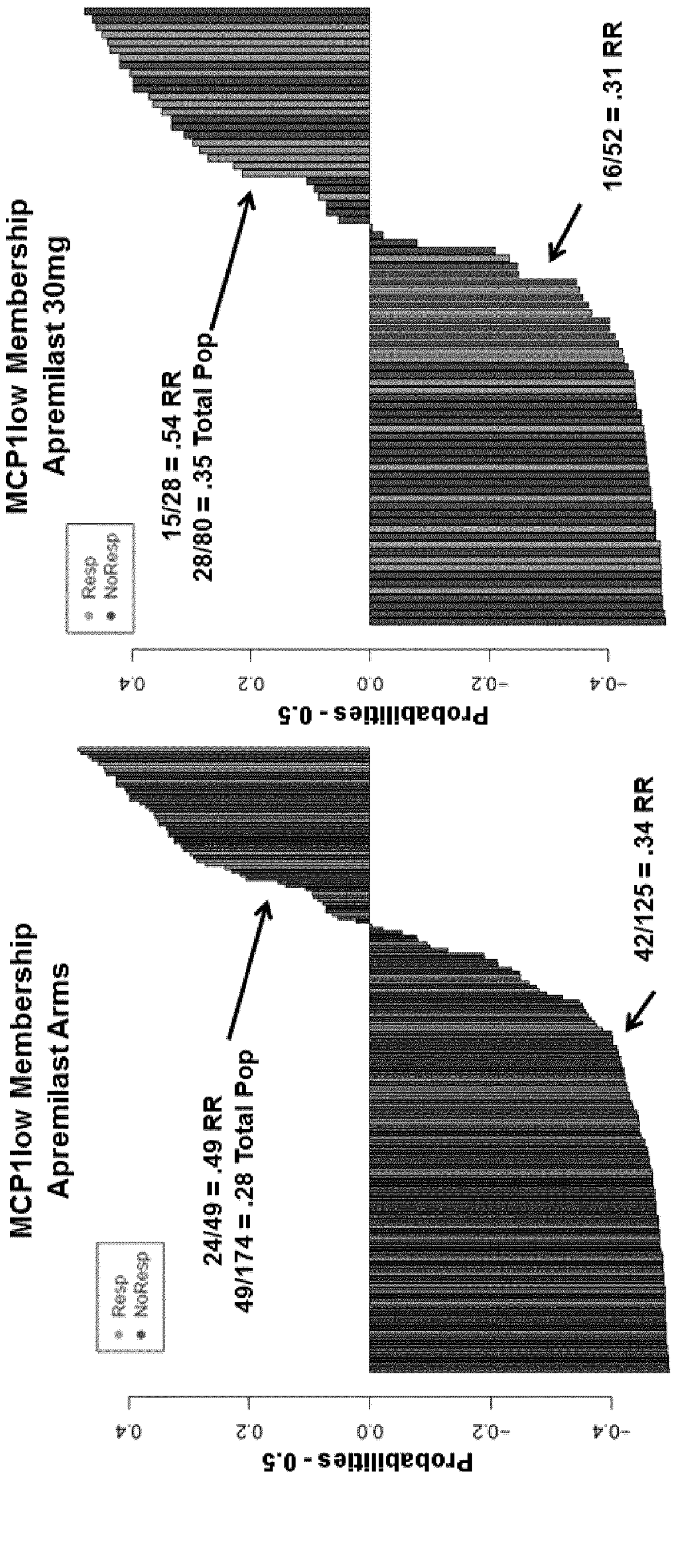
MCP1low Membership
Apremilast 30mg
Resp
NoResp
15/28 = .54 RR
28/80 = .35 Total Pop
16/52 = .31 RR
0.4
0.2
0.0
−0.2
−0.4
Probabilities - 0.5
MCP1low Membership
Apremilast Arms
Resp
NoResp
24/49 = .49 RR
49/174 = .28 Total Pop
42/125 = .34 RR
0.4
0.2
0.0
−0.2
−0.4
Probabilities - 0.5

FIG. 3C

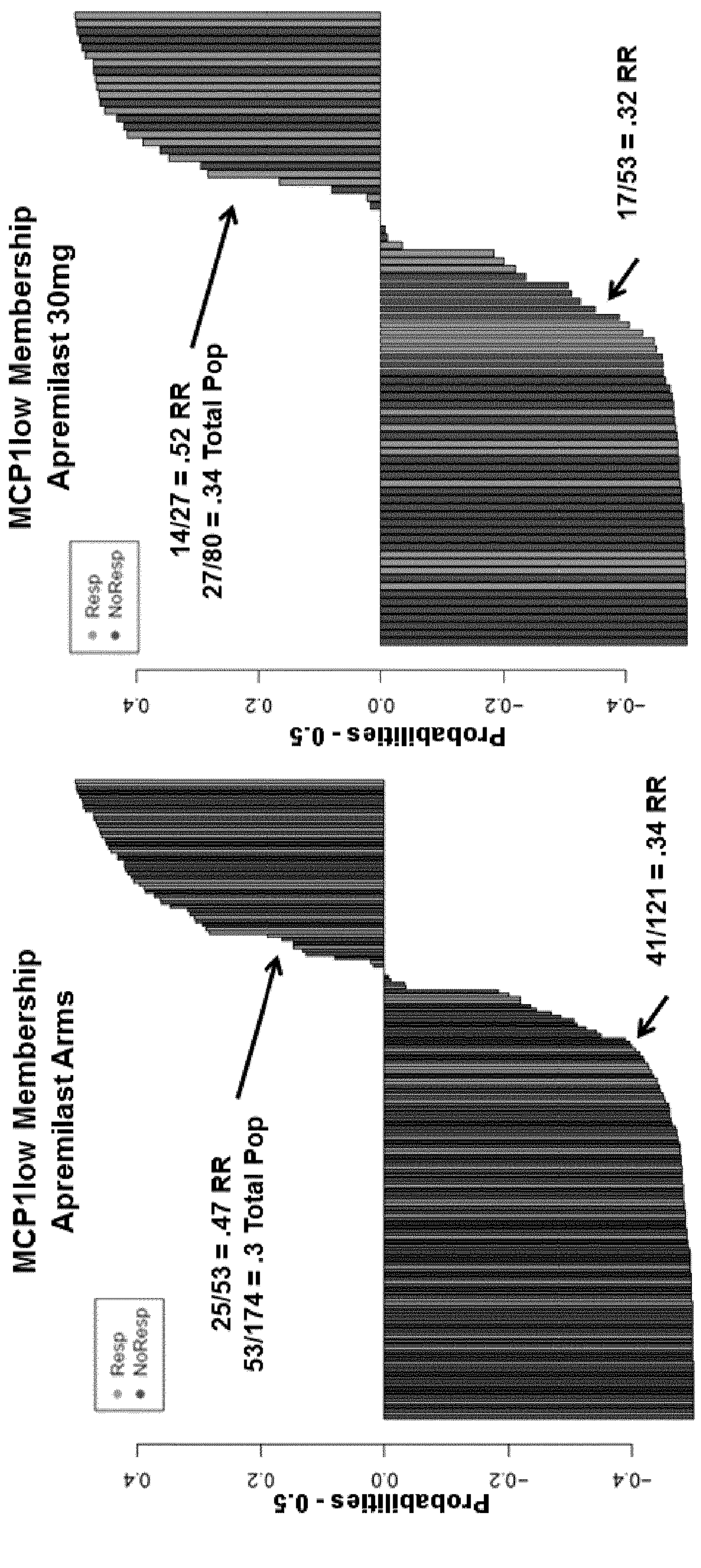
MCP1low Membership
Apremilast Arms
Resp
NoResp
25/53 = .47 RR
53/174 = .3 Total Pop
41/121 = .34 RR
Probabilities - 0.5
0.4
0.2
0.0
−0.2
−0.4
MCP1low Membership
Apremilast 30mg
Resp
NoResp
14/27 = .52 RR
27/80 = .34 Total Pop
17/53 = .32 RR
Probabilities - 0.5
0.4
0.2
0.0
−0.2
−0.4

FIG. 3D

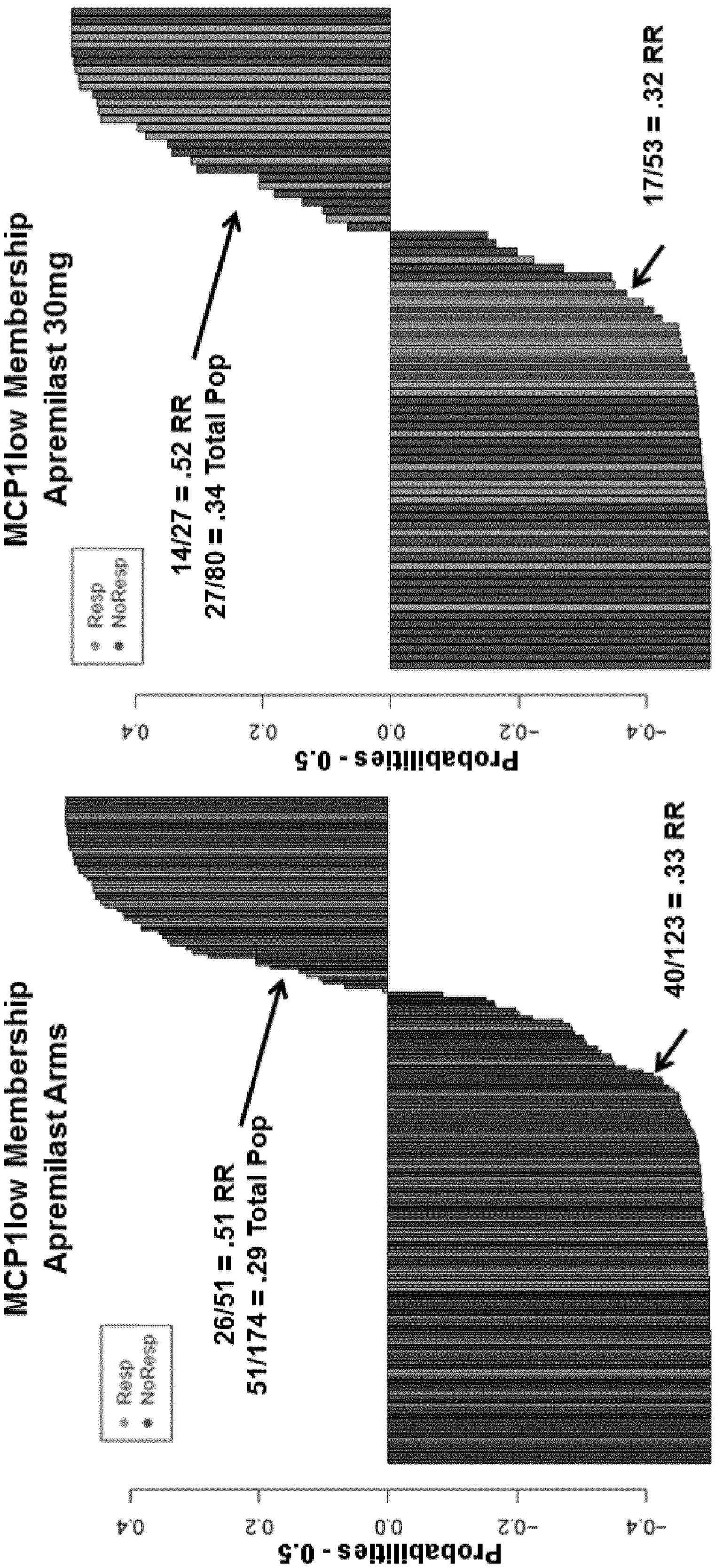
MCP1low Membership
Apremilast Arms
Resp
NoResp
26/51 = .51 RR
51/174 = .29 Total Pop
40/123 = .33 RR
Probabilities - 0.5
0.4
0.2
0.0
−0.2
−0.4
MCP1low Membership
Apremilast 30mg
Resp
NoResp
14/27 = .52 RR
27/80 = .34 Total Pop
17/53 = .32 RR
Probabilities - 0.5
0.4
0.2
0.0
−0.2
−0.4

FIG. 3E

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010077722 A1
- US 6962940 B
- US 7208516 B
- US 7427638 B
- US 7893101 B
- WO 9318186 A
- US 6927024 B
- US 7122799 B
- US 7186507 B
- US 7101663 B


### Non-patent literature cited in the description


- **MCCORMACK et al.** *BioDrugs: clinical immunotherapeutics, biopharmaceuticals and gene therapy,* 2003, vol. 18 (3), 199-205
- **AUSUBEL et al.** Short Protocols in Molecular Biology. Wiley & Sons, 1995
- **SAMBROOK et al.** Molecular Cloning: A Laboratory Manual. Cold Spring Harbor, 2001
- **KALLIONIEMI et al.** *Science,* 1992, vol. 258, 818-821
- **TIJSSEN.** Hybridization with Nucleic Acid Probes. Elsevier, 1993
- **GALL et al.** *Meth. Enzymol.,* 1981, vol. 21, 470-480
- **ANGERER et al.** Genetic Engineering: Principles and Methods. Plenum Press, 1985, vol. 7, 43-65
- **BUSTIN et al.** *Clin. Sci.,* 2005, vol. 109, 365-379